# EUROPÄISCHE PATENTSCHRIFT

(11) **EP 3 939 546 B1**
(45) Veröffentlichungstag und Bekanntmachung des Hinweises auf die Patenterteilung: **11.10.2023**
(21) Anmeldenummer: 20186551.6
(22) Anmeldetag: 17.07.2020
(51) Int. Cl.: A61F 2/28, A61F 2/30, A61F 2/36, A61F 2/38, A61F 2/40, B29C 33/12, B29C 43/02, B29C 43/36, A61B 17/56, B29C 33/30, B29C 33/00, B29C 43/04

(54) **VORRICHTUNG UND VERFAHREN ZUR HERSTELLUNG VON SPACERN**
DEVICE AND METHOD FOR PRODUCING SPACERS
DISPOSITIF ET PROCÉDÉ DE FABRICATION D'ESPACEURS

(43) Veröffentlichungstag der Anmeldung: 19.01.2022
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- EP-B1- 2 532 323
- EP-B1- 3 075 357
- EP-B1- 3 143 963
- DE-A1-102017 110 732
- US-A1- 2007 222 114
- US-A1- 2010 102 484
- US-A1- 2012 256 344
- US-A1- 2016 129 610
- US-A1- 2018 319 057
- Biomaterials Innovation Teknimed: "CEMFIX 1 & 3", , 1. Januar 2014 (2014-01-01), Seiten 1-2, XP055758318, Gefunden im Internet: URL:http://www.innovamed.uy/media/file/60 [gefunden am 2020-12-09]
- Geek Gaming Scenics: "Rock Moulds Your Questions Answered: How To Use Woodland Scenics Rock Moulds", Youtube, 17. April 2018 (2018-04-17), Seite 1 pp., XP054981183, Gefunden im Internet: URL:https://www.youtube.com/watch?v=1kxrxv wq0pk [gefunden am 2020-12-09]
- Luke Towan: "Build an Ultra-Realistic River in the Alaskan Wilderness - Realistic Scenery Vol.21", Youtube, 8. Januar 2020 (2020-01-08), Seite 1 pp., XP054981182, Gefunden im Internet: URL:https://www.youtube.com/watch?v=nCZVov oGonM [gefunden am 2020-12-09]

## Beschreibung

Die Erfindung ist in Anspruch 1 definiert und betrifft eine Vorrichtung zum Herstellen eines Spacers durch Aushärten von Knochenzementteig in einer Gießform, wobei der Spacer dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk, ein Kniegelenk oder ein Schultergelenk temporär zu ersetzen. Der Spacer ist als temporärer Platzhalter dazu vorgesehen, im medizinischen Anwendungsbereich ein Gelenk oder einen Teil eines Gelenks umfassend eine artikulierende Oberfläche des Gelenks temporär zu ersetzen. Bevorzugt ist der Spacer zum temporären Ersetzen eines Hüftgelenks, eines Kniegelenks oder eines Schultergelenks geeignet und vorgesehen. Dementsprechend ist die Vorrichtung bevorzugt zum Herstellen eines Hüftgelenkspacers, eines Kniespacers oder eines Schultergelenkspacers vorgesehen. Die Erfindung betrifft auch ein Verfahren zur Herstellung eines solchen Spacers mit einer solchen Vorrichtung. Das erfindungsgemäße Verfahren ist in Anspruch 13 definiert.

Gegenstand der Erfindung ist somit insbesondere eine Vorrichtung zur intraoperativen Herstellung von Hüft-, Schulter- und Kniespacern, die als temporäre Platzhalter (Spacer) im Rahmen von zweizeitigen Revisionen von infizierten Hüft-, Schulter- und Knietotalgelenkendoprothesen für die Interimsphase bestimmt sind. Die Vorrichtung ist für die Herstellung von Spacern mit niedrigviskosem und hochviskosem Polymethylmethacrylat-Knochenzementteig geeignet. Ein weiterer Gegenstand der Erfindung ist ein Verfahren zur Herstellung von Spacern mit der Vorrichtung.

Gelenk-Endoprothesen, wie Hüftgelenk-Endoprothesen, Kniegelenk-Endoprothesen und Schultergelenk-Endoprothesen, werden in großem Umfang weltweit implantiert. Leider kommt es in einem geringen Prozentsatz vor, dass Gelenk-Endoprothesen von mikrobiellen Keimen, besonders Gram-positiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Gelenk-Endoprothesen, die einzeitige septische Revision und die zweizeitige septische Revision. Bei der einzeitigen Revision wird innerhalb einer OP zuerst die infizierte Gelenk-Endoprothesen entfernt, anschließend radikal debridiert und dann wird eine Revisions-Gelenk-Endoprothese implantiert.

Ein Hüftgelenkspacer besteht aus einem Schaft, einem Kragen, einem Hals und einem Kugelkopf und ist den Hüftgelenk-Endoprothesen in Form und Größe nachgebildet. Analog ist ein Schultergelenkspacer einer Schultergelenk-Endoprothesen in Form und Größe nachgebildet. Ein Kniespacer hat eine Tibia-Komponente und eine Femur-Komponente und ist grundsätzlich den Kniegelenk-Endoprothesen in Form und Größe nachgebildet. Die Tibia-Komponente weist einen Schaft und ein Tibia-Plateau auf, wobei das Tibia-Plateau eine Gleitfläche beziehungsweise Abrollfläche des Kniegelenks bildet und der Schaft in der Tibia verankert werden kann. Die Femur-Komponente weist einen Schaft zur Verankerung im Femur und zwei Kondylen zum Abrollen auf dem Tibia-Plateau auf. Die Tibia-Komponente und die Femur-Komponente werden mit Knochenzement an dem jeweiligen Knochen verankert, also beispielsweise bei der Femur-Komponente am distalen Femur beziehungsweise im Femurkanal verankert und bei der Tibia-Komponente mit dem Schaft an der proximalen Tibia beziehungsweise im Tibiakanal verankert.

Der Spacer wird mit Knochenzement an dem jeweiligen Knochen verankert, also beispielsweise bei Hüftgelenkspacern am proximalen Femur beziehungsweise im Femurkanal verankert. Der Spacer verbleibt bis zu mehreren Wochen im Patienten bis die Entzündung abgeklungen ist und die klinischen Entzündungsmarker zurückgegangen sind. Dann wird in einer zweiten OP der Spacer entfernt und nach erneutem Debridement eine Revisions-Gelenk-Endoprothese implantiert.

Bei Spacern werden dem Zementpulver vor der eigentlichen Spacerherstellung Antibiotika zugesetzt. Mit diesem antibiotisch modifizierten Knochenzementpulver wird anschließend durch Beimischen von Monomerflüssigkeit ein Knochenzementteig hergestellt und aus diesem Knochenzementteig werden Spacer gegossen, die dann durch Polymerisation mit Hilfe der dem Zementpulver zugesetzten Monomerflüssigkeit aushärten. Der Knochenzementteig schließt dabei die Antibiotika im Wesentlichen ein. Die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In dem Patent US 6 245 111 B1 wird eine Hüftgelenksprothese vorgeschlagen, deren Oberflächen mit einem Antibiotikum beschichtet sind. Das Patent US 5 681 289 offenbart eine Einrichtung zum Verteilen eines flüssigen Wirkstoffs mit Hilfe einer Blase im Inneren der Einrichtung. In der EP 1 991 170 B1 und der US 2011/0015754 A1 werden ein Hüftgelenkspacer beschrieben, die Wirkstoffe enthalten. Die US 2019/0290833 A1 offenbart einen spülbaren Hüftgelenkspacer, mit dem ein Flüssigkeitskreislauf erzeugbar ist. In der WO 2016/205077 A1 und der US 8 900 322 B2 werden weitere Spacer mit einer Spülfunktion beschrieben.

Bei zweizeitigen septischen Revisionen wird in einer ersten OP zuerst die infizierte Gelenk-Endoprothese entfernt, dann wird debridiert und anschließend erfolgt die Implantation eines Spacers. Im Rahmen von zweizeitigen septischen Wechseloperationen von Knie-, Hüft- und Schultertotalgelenkendoprothesen sind Spacer als temporäre Platzhalter in der Interimsphase von wesentlicher Bedeutung. Bei der intraoperativen Herstellung dieser Spacer kann vom medizinischen Personal je nach vorliegenden Antibiogramm der für die Infektionen ursächlichen mikrobiellen Keime ein oder mehrere speziell auf die Keime abgestimmtes Antibiotikum oder mehrere Antibiotika dem Polymethylmethacrylat-Knochenzement (PMMA-Knochenzement) zugesetzt werden. Ein solcher Knochenzement ist beispielsweise aus der EP 1 985 317 B1 bekannt. Für die intraoperative Herstellung von Spacern mit Polymethylmethacrylat-Knochenzement sind Kunststoffgießformen üblich. Derartige Gießformen sind beispielsweise aus den Patenten EP 2 617 393 B1 und EP 3 075 357 B1 sowie den nicht vorveröffentlichten europäischen Patentanmeldungen EP 20 164 534 und EP 20 164 541 bekannt. Eine beheizbare Gießform ist aus dem Patent EP 2 818 138 B1 bekannt. Die US 2018/319057 A1 offenbart eine mehrteilige Gießform mit austauschbaren Einsätzen unterschiedlicher Größe sowie einem unteren und einem oberen Formelement, in das die Einsätze eingelegt werden können. Ein Austreten von überschüssigem Knochenzement ist nicht vorgesehen.

Spacer können einerseits vom OP-Personal während der OP selbst aus PMMA-Knochenzementpulver, Antibiotika und Monomerflüssigkeit hergestellt werden, zum Beispiel mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Spacer einzusetzen. Kunststoffgießformen zur intraoperativen Herstellung von einteiligen Hüftspacern werden in der US 6 361 731 B1 beschrieben. Diese Gießformen sind transparent und haben zwei separate Einfüllöffnungen. Dadurch kann auch hochviskoser Knochenzementteig mit geringem Druck in die Gießform eingebracht werden, weil die Fließwege des Knochenzementteigs relativ kurz sind. Bei der Verwendung von nicht hochviskosem Knochenzementteig besteht die Gefahr, dass Knochenzementteig nach erfolgter Füllung der Gießform vor der einsetzenden Aushärtung wieder aus den Einfüllöffnungen herausfließt.

In einer Weiterentwicklung wurden in den Patentschriften US 7 637 729 B2, US 7 789 646 B2, US 8 480 389 B2 und US 8 801 983 B2 mehrteilige Gießformen für die Herstellung modularer Hüftspacer vorgeschlagen. Diese modularen Hüftspacer bestehen aus einem Spacerkopf und einem separaten Schaft. Daher sind eine Gießform für den Spacerkopf und eine separate Gießform für den Schaft notwendig. Die Gießform für den Schaft ist einteilig und besitzt an der Einfüllöffnung ein Gewinde zur Verbindung der Gießform mit einer Zementkartusche enthaltend den Knochenzementteig. Derartige modulare Systeme müssen individuell passend zusammengesetzt werden und die Teile miteinander verbunden werden, um dem Druck beim Einpressen des oft zähflüssigen Knochenzementteigs standhalten zu können. Dementsprechend sind die mehrteiligen Gießformen kostenaufwendig und auch aufwendig in der Anwendung.

Im Patent US 7 789 646 B2 wird eine Gießform beschrieben, bei der mit einem Stöpsel die Einfüllöffnung der Gießform verschlossen werden kann, wenn der Knochenzementteig in die Gießform eingebracht wurde. Zuvor muss jedoch die Gießform von der Zementkartusche abgeschraubt werden. Bei Verwendung von niedrigviskosen Zementteig ist es daher möglich, wenn die Gießform ungünstig gehalten wird, dass Zementteig während der Trennung der Gießform von der Zementkartusche ausläuft, bevor der Stöpsel eingeschraubt ist. Bei der Anwendung von hochviskosem Knochenzementteig besteht im Grunde die gleiche Gefahr, da der zum Befüllen notwendige Druck zu einer elastischen Verformung der Gießform führt, die bei einer Rückstellung der Form der Gießform den enthaltenen Knochenzementteig aus der geöffneten Einfüllöffnung drücken kann.

In der US 2007/0222114 A1 wird eine Hüftspacerform beschrieben. Diese Spacerform besteht aus einer Vielzahl von Formsegmenten, die miteinander verbunden werden. Durch die Vielzahl der Segmente kann die Spacerform recht genau an die anatomischen Gegebenheiten des Patienten angepasst werden. Die Spacerform-Segmente werden mittels Schraubschellen aneinandergefügt. Durch Kanäle in der Spacerform wird ein PMMA-Knochenzementteig (Polymethylmethacrylat-Knochenzementteig) eingeführt. Durch den komplexen Aufbau der Gießform ist es sehr aufwändig, die Spacerform-Segmente zusammenzufügen und nach erfolgter Aushärtung des PMMA-Knochenzementteigs den Hüftspacer zu entnehmen.

In der WO 2009/073 781 A2 wird eine Spacerform für einen Hüftspacer vorgeschlagen, die aus zwei Teilen besteht, die in Bezug zueinander verschoben werden können, um die Länge des Schafts anpassen zu können. Eine weitere Gießform ist in der EP 2 522 310 A1 offenbart. Diese Vorrichtung besteht aus wenigstens zwei Teilen, wobei in einem ersten Teil eine Einschubabschnitt und im zweiten Teil eine Einschubaufnahme angeordnet sind. Beide Teile sind in einander steckbar und bilden eine Gießform für die Herstellung des Schafts des Hüftspacers. In der EP 2 787 928 A1 wird eine komplexe Gießform beschrieben. Diese ermöglicht die Herstellung von Hüftspacern mit unterschiedlichen Kugelköpfen. Die Fixierung der Elemente der Gießform erfolgt über Verbindungselemente. Im Patent US 7 789 646 B2 wird eine Gießform beschrieben, bei der mit einem Stöpsel die Einfüllöffnung der Gießform verschlossen werden kann, wenn der Knochenzementteig in die Gießform eingebracht wurde. Zuvor muss jedoch die Gießform von der Knochenzementkartusche abgeschraubt werden. Bei Verwendung von nicht hochviskosem Knochenzementteig ist es daher möglich, wenn die Gießform ungünstig gehalten wird, dass Knochenzementteig während der Trennung der Gießform von der Knochenzementkartusche ausläuft, bevor der Stöpsel eingeschraubt beziehungsweise eingesteckt ist.

Die US 2016/129610 A1 offenbart Spacerformen zur Herstellung von Kniespacern, bei denen eine Abdeckung in ein Formteil eingesteckt werden kann, wobei überschüssiger Knochenzementteig durch Löcher austreten kann. Aus der US 2012/0256344 A1 ist eine mehrteilige Spacerform mit einem Verbindungs- und Verstärkungselement zur stabilen Verbindung der Teile der Spacerform bekannt.

Die Druckschriften EP 2 931 180 B1, US 2010/0102484 A1 und EP 2 532 323 B1 offenbaren Knochenzementformen zur Herstellung einer Tibia-Komponente eines Kniespacers, der zur Abgabe von Antibiotika geeignet ist. Der Zusammenbau und das Fixieren der Gießformeile aneinander ist jedoch mit erheblichem Aufwand verbunden. Zudem sind die Gießformen durch die hierfür notwendigen Bauteile komplex, nicht kostengünstig und aufwendig in der Anwendung. Das Patent EP 3 143 963 B1 offenbart eine dreiteilige Spacerform, mit einer Tibia-Spacerform, einer Femur-Spacerform und einer dritten Form zur Herstellung einer, einen intramedullären Kanal füllenden Komponente.

Aus der US 10 071 511 B2 sind Gießformen für die Herstellung von Tibia-Komponenten von Kniespacern bekannt. Mit diesen Gießformen könne unterschiedlich hohe Tibia-Komponenten gefertigt werden. Die Gießform besteht aus einem Unterteil und einem Oberteil, welche einen Hohlraum bilden, in den ein Polymethylmethacrylat-Knochenzementteig über einen Port eingepresst werden kann. Die Höhe der zu gießenden Tibiakomponente wird durch einen Rastmechanismus mit Zähnen definiert, der den Abstand zwischen dem Unterteil und dem Oberteil der Gießform festlegt.

Ein ähnliches Konzept ist aus der US 9 433 506 B2 bekannt, die eine Form für einen Kniespacer beansprucht. Dabei enthält die Gießform einen Port, der nach der Entformung im intramedullären Raum als Schaft vorliegt. Die Gießform wird über den Port mit Hilfe einer Zementkartusche befüllt.

Die Aufgabe der vorliegenden Erfindung besteht also darin, die Nachteile des Stands der Technik zu überwinden. Insbesondere besteht die Aufgabe der Erfindung in der Entwicklung einer kostengünstigen und mit möglichst geringem Aufwand einsetzbaren Vorrichtung zum Herstellen eines Spacers durch Aushärten von Knochenzementteig in einer Gießform und in der Entwicklung eines einfach und kostengünstig durchführbaren Verfahrens zum Herstellen eines Spacers durch Aushärten von Knochenzementteig in einer Gießform, mit dem vom medizinischen Personal im OP unter Verwendung von Knochenzementteig, insbesondere von Polymethylmethacrylat-Knochenzement, einteilige Spacer, insbesondere Hüft-, Knie- und Schulterspacer, hergestellt werden können.

Aufgabe der vorliegenden Erfindung ist es somit, eine kostengünstige einfach herstellbare Vorrichtung zu entwickeln, mit dem vom medizinischen Personal im OP unter Verwendung von Polymethylmethacrylat-Knochenzement auf einfachste Weise artikulierende Knie-, Hüft- und Schulterspacer sowie die Tibia- und die Femurkomponenten von Kniespacern hergestellt werden können. Hüft- und Schulterspacer sind ähnlich aufgebaut. Sie bestehen aus einem Schaft und einem Spacerkopf. Ein Metallkern kann zur mechanischen Stabilisierung im Inneren des Hüft- und Schulterspacers angeordnet sein beziehungsweise werden. Die Kniespacer bestehen aus einer Tibia- und einer Femurkomponente. Es soll möglich sein, sowohl mit (Polymethylmethacrylat-)Knochenzementteig niedriger beziehungsweise nicht hoher Viskosität als auch mit hoher Viskosität Spacer herzustellen.

Die Vorrichtung soll vorzugsweise so beschaffen sein, dass für die Herstellung der Spacer keine auf auspressbare Kartuschen basierende Zementiersysteme notwendig sind. Es soll möglich sein, in einer Mischschüssel mit einem Mischspatel manuell gemischten Knochenzementteig zur Herstellung der Spacer zu verwenden. Die Befüllung der Vorrichtung soll vorzugsweise ohne die Anwendung von Kartuschenauspressvorrichtungen erfolgen können. Eine Kontamination des medizinischen Personals und des OP-Umfelds durch austretenden überschüssigen Knochenzementteig soll möglichst vermieden werden.

Die Aufgaben der Erfindung werden gelöst durch eine Vorrichtung zum Herstellen eines Spacers durch Aushärten von Knochenzementteig in einer Gießform, wobei der Spacer dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk, ein Kniegelenk oder ein Schultergelenk temporär zu ersetzen, die Vorrichtung aufweisend
ein Gießformunterteil, wobei das Gießformunterteil eine Kavität zur Aufnahme eines Knochenzementteigs und zum Formen eines ersten Oberflächenbereichs des Spacers aus dem Knochenzementteig aufweist;
eine Gießformwand, die sich von einem umlaufenden Rand der Kavität umlaufend von der Kavität des Gießformunterteils weg erstreckt und die auf der der Kavität gegenüberliegenden Seite offen ist, so dass die Kavität durch einen von der Gießformwand begrenzten Innenraum zugänglich ist;
ein Gießformoberteil, wobei das Gießformoberteil eine formgebende Oberfläche zum Formen eines zweiten Oberflächenbereichs des Spacers aus dem Knochenzementteig aufweist, wobei das Gießformoberteil durch die offene, auf der der Kavität gegenüberliegenden Seite der Gießformwand in den Innenraum einsteckbar und in Richtung der Kavität verschiebbar ist, so dass sich ein von der Kavität des Gießformunterteils, der formgebenden Oberfläche des Gießformoberteils und der Gießformwand begrenzter Hohlraum bildet, in dem der Spacer formbar ist;
mindestens einen Behälter zur Aufnahme von überschüssigem Knochenzementteig; und mindestens eine Durchbrechung in der formgebenden Oberfläche des Gießformoberteils und/oder in der Kavität des Gießformunterteils, wobei die mindestens eine Durchbrechung in den mindestens einen Behälter zur Aufnahme von überschüssigem Knochenzementteig mündet, wobei
die Vorrichtung einen oder zwei Deckel aufweist, mit dem das Gießformoberteil auf der der formgebenden Oberfläche des Gießformoberteils gegenüberliegenden Seite nach außen verschlossen oder verschließbar ist, so dass sich zwischen dem Gießformoberteil und dem Deckel ein nach außen geschlossener oberer Behälter als einer der mindestens einen Behälter zur Aufnahme von Knochenzementteig bildet, und/oder mit dem das Gießformunterteil auf der der Kavität des Gießformunterteils gegenüberliegenden Seite nach außen verschlossen oder verschließbar ist, so dass sich zwischen dem Gießformunterteil und dem Deckel ein nach außen geschlossener unterer Behälter als einer der mindestens einen Behälter zur Aufnahme von Knochenzementteig bildet und
der oder einer der Deckel in oder auf das Gießformoberteil oder in oder auf die Innenwand des Gießformoberteils ein- oder aufgesetzt oder ein- oder aufsetzbar ist, um die der formgebenden Oberfläche des Gießformoberteils gegenüberliegenden Seite nach außen zu verschließen und dort einen geschlossenen Behälter zur Aufnahme von überschüssigem Knochenzementteig zu bilden, und/oder der oder einer der Deckel in oder auf das Gießformunterteil ein- oder aufgesetzt oder ein- oder aufsetzbar ist, um die der Kavität des Gießformunterteils gegenüberliegenden Seite nach außen zu verschließen und dort einen geschlossenen Behälter zur Aufnahme von überschüssigem Knochenzementteig bildet.

Bevorzugt kann vorgesehen sein, dass die Gießformwand eine Höhe von mindestens 10 mm hat.

Bevorzugt ist die mindestens eine Durchbrechung nur in der formgebenden Oberfläche des Gießformoberteils angeordnet. Hierdurch können auch dünnflüssige Knochenzemente in die Kavität eingefüllt werden, ohne dass dieser durch die mindestens eine Durchbrechung in der Kavität ausfließen kann.

Die formgebende Oberfläche des Gießformoberteils kann ebenfalls als eine Kavität ausgeformt sein.

Bevorzugt schließt die Gießformwand einen Innenraum ein, der an das durch die Kavität des Gießformunterteils begrenzte Volumen anschließt.

Der Behälter kann offen sein, ist jedoch bevorzugt für Knochenzementteig dicht verschlossen oder verschließbar, um eine Kontamination der Umgebung durch den Knochenzementteig zu verhindern.

Zur Herstellung von Hüft- und Schulterspacern liegt die Trennung der Gießform in das Gießformunterteil und das Gießformoberteil vorzugsweise in der Mitte des herzustellenden Spacers. Das bedeutet, die eine Spacerhälfte wird von der Kavität des Gießformunterteils geformt und die zweite Spacerhälfte von der formgebenden Oberfläche des Gießformoberteils.

Bei den Tibia- und auch bei der Femurkomponente von Kniespacern ist es vorteilhaft und bevorzugt, wenn die Kavität des Gießformunterteils die Form der Gleitflächen abbildet und die formgebende Oberfläche des Gießformoberteils die jeweilige Rückseite der Kniespacerkomponente. Hierbei wird die Trennung vorteilhaft an den oberen Rand der Rückseiten der Kniespacerkomponenten gelegt.

Die Vorrichtung ist vorzugsweise für niedrigviskosen Knochenzementteig oder für gekühlten mittel- bis hochviskosen Knochenzementteig geeignet.

Vorzugsweise ist der mindestens eine Behälter zur Aufnahme von Knochenzementteig nach außen für den Knochenzementteig undurchlässig verschlossen.

Die Kavität ist vorzugsweise nach Art einer Halbkugelschale geformt.

Bei erfindungsgemäßen Vorrichtung kann vorgesehen sein, dass einander gegenüberliegende Teile der Gießformwand parallel zueinander ausgerichtet sind oder die Gießformwand in Richtung der Kavität leicht konisch zusammenläuft oder die Gießformwand die Form eines geraden oder schiefen allgemeinen Zylinders aufweist, dessen Grundfläche von dem umlaufenden Rand der Kavität begrenzt ist.

Hierdurch kann das Gießformoberteil in der Gießformwand linear verschoben werden und gleichzeitig den Hohlraum bis auf die mindestens eine Durchbrechung nach oben abdichten. Dadurch kann der Knochenzementteig in der Gießform in die gewünschte Form gepresst werden.

Dass die Gießform in Richtung der Kavität leicht konisch zusammenläuft, bedeutet, dass der spitze Winkel des Konus beziehungsweise der Winkel, in dem die Wandungen gegeneinander geneigt sind, nicht mehr als 15° beträgt, bevorzugt nicht mehr als 4° beträgt, besonders bevorzugt nicht mehr als 1° beträgt.

Ein allgemeiner Zylinder entsteht durch die lineare Verschiebung einer Grundfläche, hier der Grundfläche, die von dem umlaufenden Rand der Kavität begrenzt ist. Bei einem geraden Zylinder erfolgt die Verschiebung senkrecht zu dieser Grundfläche, bei einem schiefen Zylinder in einem von 90° abweichenden Winkel.

Des Weiteren kann vorgesehen sein, dass sich eine Innenwand von einem umlaufenden Rand der formgebenden Oberfläche des Gießformoberteils umlaufend von der Kavität des Gießformunterteils weg erstreckt, wobei vorzugsweise die Innenwand den mindestens einen Behälter zumindest bereichsweise begrenzt und/oder einander gegenüberliegende Teile der Innenwand parallel zueinander ausgerichtet sind oder die Innenwand die Form eines geraden oder schiefen allgemeinen Zylinders aufweist, dessen Grundfläche von dem umlaufenden Rand der formgebenden Oberfläche des Gießformoberteils begrenzt ist.

Mit der Innenwand kann eine flächige Abdichtung gegen die Gießformwand erfolgen. Gleichzeitig wird das Gießformoberteil in der Gießformwand stabil geführt.

Die Innenwand wird als solche bezeichnet, da sie innenliegend zur Gießformwand angeordnet wird. Unter Innenwand soll vorliegend also keinesfalls eine nach Innen ausgerichtete Seite einer Wandung verstanden werden sondern eine körperliche Wandung mit einem Volumen.

Die Innenwand kann bevorzugt einteilig mit dem Gießformoberteil ausgeführt sein und besonders bevorzugt ein Teil des Gießformoberteils sein.

Es kann auch vorgesehen sein, dass die Innenwand eine Höhe von mindestens 10 mm hat.

Des Weiteren kann vorgesehen sein, dass die Innenwand in ihrem Außenumfang genauso groß oder geringfügig kleiner ist als der Innenumfang der Gießformwand.

Auch kann vorgesehen sein, dass die Innenwand und die Gießformwand flächenbündig aneinander anliegen, wenn das Gießform oberteil in die Gießformwand eingeschoben ist und/oder die Innenwand gegen die Gießformwand für den Knochenzementteig abdichtet, wenn das Gießformoberteil in die Gießformwand eingeschoben ist.

Hierdurch wird im Inneren der zusammengesetzten Gießformteile der Vorrichtung ein nach außen abgeschlossener Hohlraum zum Ausformen des Spacers geformt. Zudem wird hierdurch ein Verkippen des Gießformoberteils gegen das Gießformunterteil verhindert. Damit kann vermieden werden, dass die Oberflächen des Spacers ungewollt gegeneinander geneigt werden, beispielsweise das Tibiaplateau ungewollt gegen eine restliche Tibia-Kniespacerkomponente verkippt. Des Weiteren wird hierdurch das stempelförmige Gießformoberteil stabilisiert, so dass das Gießformoberteil auch aus dünnen Kunststofffolien gefertigt werden kann.

Es ist vorgesehen, dass die Vorrichtung einen oder zwei Deckel aufweist, mit dem das Gießformoberteil auf der der formgebenden Oberfläche des Gießformoberteils gegenüberliegenden Seite nach außen verschlossen oder verschließbar ist, so dass sich zwischen dem Gießformoberteil und dem Deckel ein nach außen geschlossener oberer Behälter als einer der mindestens einen Behälter zur Aufnahme von Knochenzementteig bildet, und/oder mit dem das Gießformunterteil auf der der Kavität des Gießformunterteils gegenüberliegenden Seite nach außen verschlossen oder verschließbar ist, so dass sich zwischen dem Gießformunterteil und dem Deckel ein nach außen geschlossener unterer Behälter als einer der mindestens einen Behälter zur Aufnahme von Knochenzementteig bildet.

Dabei ist vorgesehen, dass der oder einer der Deckel in oder auf das Gießformoberteil oder in oder auf die Innenwand des Gießformoberteils ein- oder aufgesetzt oder ein- oder aufsetzbar ist, um die der formgebenden Oberfläche des Gießformoberteils gegenüberliegenden Seite nach außen zu verschließen und dort einen geschlossenen Behälter zur Aufnahme von überschüssigem Knochenzementteig zu bilden, und/oder der oder einer der Deckel in oder auf das Gießformunterteil ein- oder aufgesetzt oder ein- oder aufsetzbar ist, um die der Kavität des Gießformunterteils gegenüberliegenden Seite nach außen zu verschließen und dort einen geschlossenen Behälter zur Aufnahme von überschüssigem Knochenzementteig bildet.

Der Deckel stabilisiert oder die Deckel stabilisieren das Gießformoberteil und/oder das Gießformunterteil mechanisch, insbesondere dann, wenn das Gießformoberteil und das Gießformunterteil aus oder mit Kunststofffolien hergestellt sind. Ferner wird dadurch verhindert, dass der durch die mindestens eine Durchbrechung austretende Knochenzementteig die Umgebung kontaminiert beziehungsweise verschmutzt. Hierdurch wird der Anwender vor einem möglicherweise schädlichen Kontakt mit dem Knochenzementteig geschützt und eine Verunreinigung des Spacers durch die mindestens eine Durchbrechung hindurch verhindert oder zumindest erschwert.

Gemäß einer Weiterbildung der vorliegenden Erfindung kann vorgesehen sein, dass der oder die Deckel zumindest eine Entlüftungsöffnung aufweist oder aufweisen und/oder der oder die Deckel gasdurchlässig mit dem Gießformoberteil und/oder dem Gießformunterteil verschlossen oder verschließbar sind.

Hierdurch kann Luft aus dem mit dem Deckel begrenzten oberen oder unteren Behälter oder den mit den Deckeln begrenzten Behältern entweichen, wenn der Knochenzementteig durch die mindestens eine Durchbrechung in den oder die Behälter fließt. Damit kann ein sich möglicherweise aufbauender Gegengasdruck verhindert werden.

Des Weiteren kann vorgesehen sein, dass das Volumen der Kavität des Gießformunterteils und das von der Gießformwand begrenzte Volumen zusammen größer sind als das Volumen des zu erzeugenden Spacers.

Hiermit wird sichergestellt, dass der Knochenzementteig im Überschuss eingefüllt werden kann, um Lufteinschlüsse in dem Spacer zu vermeiden.

Auch kann vorgesehen sein, dass die Gießformwand und das Gießformunterteil einteilig ausgeführt sind, wobei vorzugsweise die Gießformwand ein Teil des Gießformunterteils ist.

Hierdurch wird die Herstellung der Vorrichtung kostengünstiger und vereinfacht.

Es kann auch vorgesehen sein, dass die Vorrichtung ein Mischsystem zum Mischen von Knochenzementteig, ein Zementpulver und eine Monomerflüssigkeit aufweist, wobei das Zementpulver und die Monomerflüssigkeit getrennt voneinander gelagert sind, wobei der Knochenzementteig mit Hilfe des Mischsystems aus dem Zementpulver und der Monomerflüssigkeit mischbar ist.

Dabei kann bevorzugt vorgesehen sein, dass das Mischsystem einen Mischbecher aufweist, der bevorzugt eine Tülle zum Einfüllen des Knochenzementteigs aus dem Mischbecher in die Kavität und den von der Gießformwand begrenzten Innenraum aufweist. Alternativ kann vorgesehen sein, dass das Mischsystem eine Knochenzementkartusche zum Lagern und Mischen des Zementpulvers und der Monomerflüssigkeit und zum Austragen von gemischtem Knochenzementteig aus der Knochenzementkartusche heraus ist, wobei besonders bevorzugt die Knochenzementkartusche die Knochenzement-Ausgangskomponenten zur Herstellung des Knochenzements in flüssigkeitsdicht voneinander getrennten Bereichen beinhaltet.

Durch das Mischsystem wird die Vorrichtung weiter komplettiert und kann unmittelbar zur Herstellung des Spacers eingesetzt werden.

Theoretisch kann die Vorrichtung auch nur das Zementpulver und die Monomerflüssigkeit aufweisen, bevorzugt einen geschlossenen ersten Behälter enthaltend das Zementpulver und einen geschlossenen zweiten Behälter enthaltend die Monomerflüssigkeit.

Ferner kann vorgesehen sein, dass das Gießformunterteil und das Gießformoberteil, und bevorzugt auch die Gießformwand und gegebenenfalls der oder die Deckel, aus einer Kunststofffolie bestehen oder im Wesentlichen aus einer Kunststofffolie bestehen oder das Gießformunterteil und das Gießformoberteil, und bevorzugt auch die Gießformwand und gegebenenfalls der oder die Deckel, jeweils aus zwei oder mehreren Kunststofffolien aufgebaut sind, die miteinander verbunden sind, besonders bevorzugt miteinander verschweißt oder verklebt sind.

Hierdurch ist der Aufbau besonders kostengünstig aber gleichzeitig überraschenderweise noch stabil genug, um darin den Spacer aus Knochenzement zu formen. Zudem kann die Vorrichtung so nach Gebrauch kostengünstig und hygienisch durch Verbrennen entsorgt werden.

Dass das Gießformunterteil und das Gießformoberteil, sowie gegebenenfalls die Gießformwand und/oder der Deckel, im Wesentlichen aus einer Kunststofffolie oder aus zwei oder mehreren miteinander verbundenen Kunststofffolien besteht, bedeutet, dass zumindest 50% des Volumens oder des Gewichts des Gießformunterteils und des Gießformoberteils, sowie gegebenenfalls der Gießformwand und/oder des Deckels, aus der Kunststofffolie oder aus den zwei oder mehreren miteinander verbundenen Kunststofffolien besteht.

Das Gießformunterteil und das Gießformoberteil, und bevorzugt auch die Gießformwand und gegebenenfalls der oder die Deckel, können vorzugsweise durch Tiefziehen von Kunststofffolien hergestellt sein.

Eine Folie im Sinne der vorliegenden Patentanmeldung sollte vorzugsweise eine Stärke von maximal 2 mm aufweisen, bevorzugt eine Stärke von maximal 1 mm.

Die Folie oder die miteinander verbundenen Folien sollen vorzugsweise freistehend sein, das heißt, nicht als eine Beschichtung vorliegen.

Bevorzugt kann auch vorgesehen sein, dass das Gießformunterteil, das Gießformoberteil und die Gießformwand und gegebenenfalls der oder die Deckel im Wesentlichen oder vollständig aus einem Kunststoff bestehen, bevorzugt aus einem Polyolefin, Polyethylen (PE) oder Polypropylen (PP), besonders bevorzugt aus einer PETG-Folie und/oder einer Polyamid-Folie und/oder einer PE-Folie gefertigt sind.

Auch hierdurch ist der Aufbau besonders kostengünstig. Zudem kann die Vorrichtung so nach Gebrauch kostengünstig und hygienisch durch Verbrennen entsorgt werden.

Es kann vorgesehen sein, dass die mindestens eine Durchbrechung eine minimale Querschnittslänge von maximal 2,5 mm hat, bevorzugt eine minimal Querschnittslänge von maximal 2 mm hat, besonders bevorzugt eine minimal Querschnittslänge von maximal 1,5 mm hat, ganz besonders bevorzugt eine minimal Querschnittslänge von maximal 1 mm hat.

Durch die maximalen Querschnittslängen wird sichergestellt, dass der in der mindestens einen Durchbrechung ausgehärtete Knochenzementteig, das heißt, der in der mindestens einen Durchbrechung gebildete Steg, noch ohne Werkzeug abgeschert oder abgebrochen werden kann, um den Spacer zu entformen. Nach der Aushärtung des Zementteigs können aufgrund des geringen Durchmessers der mindestens einen Durchbrechung die durchgreifenden Knochenzement-Stege, welche den ausgehärteten Spacer mit dem überschüssigen Knochenzementteig auf der der formgebenden Oberfläche des Gießformoberteils gegenüberliegenden Seite verbinden, leicht durch Scherung oder durch Brechen abgetrennt werden.

Unter der minimalen Querschnittslänge einer Durchbrechung ist die die engste Abmessung der zum freien Fließen geeigneten Querschnittsfläche (senkrecht zur Strömungsrichtung) zu verstehen. Bei einer spaltförmigen Durchbrechung ist dies beispielsweise die Spaltbreite und nicht etwa die Spaltlänge.

Des Weiteren kann vorgesehen sein, dass die mindestens eine Durchbrechung eine minimale Querschnittslänge von mindestens 0,2 mm hat, bevorzugt von mindestens 0,5 mm hat, besonders bevorzugt von mindestens 1 mm hat.

Durch die minimalen Querschnittslängen wird erreicht, dass der Knochenzementteig ohne zu großen mechanischen Druck durch die mindestens eine Durchbrechung gedrückt werden kann, auch wenn der Knochenzementteig eine erhöhte Viskosität aufweist. Der überschüssige Knochenzementteig kann durch derartige bemessene Durchbrechungen problemlos fließen, ohne dass ein zu großer Druck auf den Knochenzementteig ausgeübt werden muss, der entweder nicht manuell aufbringbar ist oder der zu einer Zerstörung oder Beeinträchtigung der Gießform führen könnte.

Bevorzug ist die mindestens eine Durchbrechung nicht spaltförmig und besonders bevorzugt höchstens doppelt so lang wie breit.

Gemäß einer Weiterbildung kann vorgesehen sein, dass an dem der Kavität des Gießformunterteils gegenüberliegenden Ende der Gießformwand ein Anschlag zur Begrenzung der Bewegung des Gießformoberteils innerhalb der Gießformwand in Richtung der Kavität angeordnet ist, wobei bevorzugt eine Anlagefläche als Anschlag an dem der Kavität des Gießformunterteils gegenüberliegenden Ende der Gießformwand angeordnet ist, die besonders bevorzugt im rechten Winkel von der Gießformwand absteht.

Hierdurch kann das Gießformoberteil bis zum Anschlag in die Gießformwand eingeschoben werden, um den Spacer aus dem Knochenzementteig zu formen.

Auch kann vorgesehen sein, dass die Vorrichtung einen Metallkern aufweist, der in der Kavität anzuordnen oder angeordnet ist, wobei bevorzugt die Vorrichtung mehrere Abstandhalter aufweist, die den Metallkern in der Kavität von der Innenseite der Kavität und von der Innenseite der Gießformwand beabstandet halten, wobei besonders bevorzugt die Abstandhalter aus ausgehärtetem Knochenzement, insbesondere aus Polymethylmethacrylat (PMMA) bestehen.

Hierdurch kann in dem herzustellenden Spacer eine mechanisch stabilisierende Armierung in Form des Metallkerns angeordnet werden.

Bei Vorrichtungen mit Metallkern kann vorgesehen sein, dass der Metallkern Bohrungen zur Aufnahme von stiftförmigen Abstandhaltern aufweist, wobei bevorzugt die Bohrungen nicht in einem Bereich der Kavität zum Formen einer Gleitfläche des Spacers angeordnet sind, besonders bevorzugt die Bohrungen in einem Bereich der Kavität zum Formen eines Schafts des Spacers angeordnet sind.

Zur Herstellung von Hüft- und Schulterspacern ist es vorteilhaft, dass ein Metallkern im Gießformunterteil angeordnet ist, der durch Abstandhalter von der Wand des Gießformunterteils beabstandet ist. Als Abstandhalter eignen sich besonders Stifte aus Polymethylmethacrylat. Diese Stifte verbinden sich mit dem Knochenzementteig und können nach Aushärtung der Kontur des Spacers folgend abgeschnitten werden.

Des Weiteren kann vorgesehen sein, dass das Gießform unterteil oder das Gießform unterteil und die Gießformwand oder das Gießformunterteil, die Gießformwand und die Gießformoberteil transparent oder transluzent ist oder sind.

Hierdurch kann beim Befüllen der Kavität und auch beim Ausformen des Spacers optisch nachvollzogen werden, ob und wie sich die Spacerform mit dem Knochenzementteig füllt und wie er darin aushärtet. Damit kann optisch nachvollzogen werden, ob der Spacer die gewünschte Form erhält.

Ferner kann vorgesehen sein, dass die mindestens eine Durchbrechung an den Bereichen angeordnet ist, die bei normaler Aufstellung der Gießform die höchsten Punkte des Hohlraums sind.

Hierdurch kann verhindert werden, dass sich Lufteinschlüsse in dem Hohlraum bilden, die nicht aus dem Hohlraum entweichen.

Auch kann vorgesehen sein, dass die formgebende Oberfläche des Gießformoberteils mit einem äußeren Rand innen an der Gießformwand anliegt und der Rand beim Einschieben des Gießformoberteils innen an der Gießformwand gleitet, wobei vorzugsweise der Rand eine Abstreifkante oder eine Abstreiflippe umfasst.

Hierdurch wird erreicht, dass der Knochenzementteig beim Einschieben des Gießformoberteils zum Sammeln und Verdichten des Knochenzementteigs im Inneren der Gießform verwendet werden kann.

Es kann des Weiteren vorgesehen sein, dass die Vorrichtung einen Standfuß aufweist, wobei das Gießformunterteil in den Standfuß einsetzbar ist und der Standfuß dazu geeignet ist, mit dem Gießformunterteil darin auf eine ebene Unterlage gestellt zu werden, wobei bevorzugt der Standfuß im Wesentlichen oder vollständig aus einer Kunststofffolie oder aus zwei oder mehreren miteinander verbundenen Kunststofffolien besteht, besonders bevorzugt miteinander verschweißt oder verklebt sind.

Dies hat den Vorteil, dass die Vorrichtung beim Formen des Spacers auf einer ebenen Unterlage, wie einem Tisch aufgestellt werden kann. Gleichzeitig kann der Standfuß zum Stabilisieren der Gießform, insbesondere des Gießformunterteils verwendet werden.

Der Standfuß kann im Wesentlichen oder vollständig aus PE, PP oder Polyolefin bestehen Dass der Standfuß im Wesentlichen aus einer Kunststofffolie oder aus zwei oder mehreren miteinander verbundenen Kunststofffolien besteht, bedeutet, dass zumindest 50% des Volumens oder des Gewichts des Standfußes aus der Kunststofffolie oder aus den zwei oder mehreren miteinander verbundenen Kunststofffolien besteht.

Die der vorliegenden Erfindung zugrundeliegenden Aufgaben werden auch gelöst durch ein Verfahren zur Herstellung eines Spacers zum temporären Ersetzen eines Gelenks oder eines Teils eines Gelenks, insbesondere eines Hüftgelenks, eines Kniegelenks oder eines Schultergelenks, umfassend eine artikulierende Oberfläche des Gelenks, wobei das Verfahren mit einer oben genannten Vorrichtung durchgeführt wird, das Verfahren aufweisend die folgenden chronologischen Schritte:
A) Einfüllen von fließfähigem Knochenzementteig in die Kavität und den von der Gießformwand begrenzten Innenraum, wobei der fließfähige Knochenzementteig mit einem größeren Volumen eingefüllt wird, als es der Spacer erfordert;
B) Einsetzen des Gießformoberteils in die Gießformwand, wobei die formgebende Oberfläche in Richtung der Kavität weist;
C) Eindrücken des Gießformoberteils in Richtung der Kavität innerhalb der Gießformwand;
D) Austreten von überschüssigem Knochenzementteig durch die mindestens eine Durchbrechung unter fortwährendem Eindrücken des Gießformoberteils, wobei der überschüssige Knochenzementteig in einen durch einen Deckel verschlossenen unteren Behälter zur Aufnahme des überschüssigen Knochenzementteigs fließt und dort eingeschlossen wird und/oder in einen durch einen Deckel verschlossenen oberen Behälter zur Aufnahme des überschüssigen Knochenzementteigs an dem Gießformoberteil fließt und dort eingeschlossen wird;
E) Beenden des Eindrückens bei Erreichen eines Anschlags oder bei Erreichen einer gewünschten Höhe des Spacers;
F) Aushärten des Knochenzementteigs in dem durch die Kavität, die formgebende Oberfläche des Gießformoberteils und die Gießformwand gebildeten Hohlraum; und
G) Entnehmen des so geformten und ausgehärteten Spacers aus dem Hohlraum, wobei in der mindestens einen Durchbrechung gebildete Stege des ausgehärteten Knochenzementteigs abgetrennt werden.

Das Verfahren wird vorzugsweise mit PMMA-Knochenzementteig durchgeführt.

Das Verfahren wird bevorzugt mit niedrigviskosen Knochenzementteig oder mit gekühlten mittel- bis hochviskosen Knochenzementteig angewendet.

Es kann vorgesehen sein, dass in Schritt D) der überschüssige Knochenzementteig in einen durch einen Deckel verschlossenen unteren Behälter zur Aufnahme des überschüssigen Knochenzementteigs an dem Gießformunterteil fließt und dort eingeschlossen wird und/oder in einen durch einen Deckel verschlossenen oberen Behälter zur Aufnahme des überschüssigen Knochenzementteigs an dem Gießformoberteil fließt und dort eingeschlossen wird, wobei beim Entformen des ausgehärteten Spacers nach Schritt F) oder in Schritt G) der ausgehärtete überschüssige Knochenzementteig in dem unteren Behälter und/oder dem oberen Behälter verbleibt.

Hiermit wird verhindert, dass der Knochenzementteig nach außen gelangt und die Umgebung kontaminiert.

Des Weiteren kann vorgesehen sein, dass vor Schritt A) der Knochenzementteig aus einer Monomerflüssigkeit und aus einem Zementpulver gemischt wird, insbesondere bis ein homogener Knochenzementteig entstanden ist, und bevorzugt vor Schritt A) ein Metallkern in der Kavität angeordnet wird, wobei besonders bevorzugt der Metallkern mit Hilfe von stiftförmigen Abstandhaltern von der Innenseite der Kavität und der Gießformwand beabstandet wird.

Hiermit wird das Verfahren komplettiert.

Auch kann vorgesehen sein, dass während Schritt C) die Luft aus dem von der Kavität des Gießformunterteils, der formgebenden Oberfläche des Gießformoberteils und der Gießformwand begrenzten Hohlraum durch die mindestens eine Durchbrechung herausgedrückt wird.

Hierdurch wird verhindert, dass sich in dem Spacer ungewollt Fehlstellen durch Lufteinschlüsse bilden.

Schließlich kann vorgesehen sein, dass nach Schritt F) und vor Schritt G) das Gießformoberteil aus dem offenen Ende der Gießformwand herausgezogen wird.

Dadurch wird die Entformung des Spacers vereinfacht.

Der Erfindung liegt die überraschende Erkenntnis zugrunde, dass durch ein Ineinanderschieben zweier Teile einer Gießform enthaltend einen Überschuss an Knochenzementteig gelingt, mit einer sehr stark vereinfachten und kostengünstigen Gießform einen Spacer herzustellen, indem der überschüssige Knochenzementteig durch mindestens eine geeignete Durchbrechungen in den formgebenden Oberflächen aus der Gießform austreten kann. Bevorzugt wird der austretende Knochenzementteig in geschlossenen Bereichen an der Gießform aufgefangen. Die mindestens eine Durchbrechung ist vorzugsweise an einer Stelle der Gießform angeordnet, die im Gebrauch der Gießform oben angeordnet ist, um ein Austreten von Lufteinschlüssen durch die mindestens eine Durchbrechung zu ermöglichen und damit Fehlstellen im Spacer zu vermeiden. Durch den mindestens einen Behälter zur Aufnahme von überschüssigem Knochenzementteig wird verhindert, dass der Anwender und die Umgebung bei der Anwendung der Vorrichtung nicht mit aus der Gießform austretendem Knochenzementteig verunreinigt wird. Gleichzeitig wird dadurch die Anwendung der Vorrichtung vereinfacht.

Eine beispielhafte erfindungsgemäße Vorrichtung kann zusammengesetzt sein aus
a) einem Gießformunterteil, das an seiner Oberseite mindestens eine Kavität zur Formgebung des Spacers enthält,
b) einer sich an die Kavität nach oben anschließenden, senkrecht angeordneten Gießformwand, wobei einander gegenüberliegende Wandteile der Gießformwand parallel zueinander sind, wobei die umlaufende Gießformwand einen Innenraum umschließt,
c) einem Gießformoberteil, das an seiner Unterseite mindestens eine formgebende Oberfläche zur Formgebung des Spacers enthält,
d) einer sich an die formgebende Oberfläche nach oben anschließenden, senkrecht angeordneten Innenwand, wobei einander gegenüberliegende Wandteile der Innenwand parallel zueinander sind,
e) einem Deckel der in das Gießformoberteil eingesetzt ist und den von der Oberseite des Gießformoberteils und der Innenwand gebildeten Raum als Behälter zur Aufnahme von überschüssigem Knochenzementteig abschließt,
f) mindestens einer Durchbrechung in der Wand des Gießformoberteils, die die formgebende Oberfläche des Gießformoberteils mit dem von der Oberseite des Gießformoberteils und der Innenwand gebildeten Raum verbindet,
g) wobei das Gießformoberteil vertikal gleitfähig in dem Gießformunterteil angeordnet ist,
h) wobei die Summe der Volumina der Kavität und des durch die umlaufende Gießformwand begrenzten Innenraums größer ist als das Volumen des herzustellenden Spacers, und
i) wobei nach Befüllen der der Kavität und des durch die umlaufende Gießformwand begrenzten Innenraums mit Knochenzementteig und nachfolgendem Schieben des Gießformoberteils in Richtung der Kavität der überschüssige Knochenzementteig durch die mindestens eine Durchbrechung in den durch den Deckel nach oben verschlossenen Behälter zur Aufnahme von überschüssigem Knochenzementteig tritt und dort verbleibt.

Die erfindungsgemäße Vorrichtung wird in der Weise verwendet, dass zuerst Knochenzementteig in die Kavität des Gießformunterteils und den durch die Gießformwand begrenzten Innenraum eingebracht werden. Das Volumen des Knochenzementteigs sollte etwas größer sein als das Volumen des herzustellenden Spacers. Dann wird das Gießformoberteil in das Gießformunterteil eingesetzt. Die Außenseite der Innenwand des Gießformoberteils liegt innen an der Gießformwand des Gießformunterteils an. Dann wird auf das Gießformoberteil oder den Deckel des Gießformoberteil gedrückt und das Gießformoberteil nach unten in Richtung der Kavität des Gießformunterteils bewegt. Die über dem Knochenzementteig stehende Luft in dem durch die Gießformwand begrenzten Innenraum und der formgebenden Oberfläche des Gießformoberteils entweicht durch die mindestens eine Durchbrechung in den von der Oberseite des Gießformoberteils und der Innenwand gebildeten Raum, das heißt in den Behälter zur Aufnahme von überschüssigem Knochenzementteig. Der Knochenzementteig berührt und bedeckt dann die formgebende Oberfläche des Gießformoberteils. Bei weiterer Bewegung fließt der Knochenzementteig entlang der Kontur der Wandung der Kavität (der Innenseite der Kavität) des Gießformunterteils bis der Knochenzementteig die mindestens eine Durchbrechung erreicht hat. Damit sind die Kavität des Gießformunterteils und die formgebende Oberfläche des Gießformoberteils mit Knochenzementteig gefüllt. Der Spacer wird dadurch geformt. Anschließend erfolgt die Aushärtung des Knochenzementteigs durch radikalische Polymerisation. Nach Beendigung der Aushärtung wird das Gießformoberteil aus dem Gießformunterteil gezogen. Der gebildete Spacer bleibt meistens am Gießformoberteil hängen, da er durch Hinterschneidungen im Bereich der mindestens einen Durchbrechung mit dem Gießformoberteil verbunden ist. Dann wird das Gießformoberteil vom Spacer abgetrennt. Dabei werden Knochenzementstege, welche durch die mindestens eine Durchbrechung führen, abgeschert und verbleiben in dem Behälter zur Aufnahme von überschüssigem Knochenzementteig.

Ein beispielhaftes erfindungsgemäßes Verfahren zur Herstellung von Spacern mit der erfindungsgemäßen Vorrichtung kann die folgenden nacheinander ablaufenden Schritte umfassen:
a) Vermischen eines Zementpulvers mit einer Monomerflüssigkeit und Homogenisieren der Zementkomponenten bis ein homogener Knochenzementteig entstanden ist,
b) Einfüllen des Knochenzementteigs in die Kavität des Gießformunterteils und den durch die Gießformwand begrenzten Innenraum,
c) Einsetzen des Gießformoberteils in den durch die Gießformwand begrenzten Innenraum des Gießformunterteils,
d) Herunterdrücken des Gießformoberteils in Richtung der Kavität des Gießformunterteils,
e) Herauspressen der Luft durch die mindestens eine Durchbrechung aus dem Inneren der Gießform zwischen dem Knochenzementteig und der formgebenden Oberfläche des Gießformoberteils in den von der Oberseite des Gießformoberteils und der Innenwand gebildeten Raum,
f) Herauspressen des überschüssigen Knochenzementteigs aus dem Inneren der Gießform durch die mindestens eine Durchbrechung in den von der Oberseite des Gießformoberteils und der Innenwand gebildeten Raum als Behälter zur Aufnahme von überschüssigem Knochenzementteig,
g) Aushärten des Knochenzementteigs,
h) Herausziehen des Gießformoberteils und
i) Entformen des ausgehärteten Spacers, wobei der ausgehärtete überschüssige Knochenzementteig in dem von der Oberseite des Gießformoberteils und der Innenwand gebildeten Raum als Behälter zur Aufnahme von überschüssigem Knochenzementteig verbleibt.

Im Folgenden werden weitere Ausführungsbeispiele der Erfindung anhand von fünfunddreißig schematisch dargestellten Figuren erläutert, ohne jedoch dabei die Erfindung zu beschränken. Dabei zeigt:
Figur 1: eine schematische perspektivische Außenansicht eines ersten Ausführungsbeispiels der vorliegenden Erfindung als Vorrichtung zum Herstellen eines Spacers für ein Hüftgelenk mit separat dargestellten Einzelteilen;
Figur 2: eine schematische Aufsicht auf das offene Gießformoberteil;
Figur 3: eine schematische perspektivische Außenansicht des ersten Ausführungsbeispiels nach den Figuren 1 und 2 vor dem Anordnen des Metallkerns im Gießformunterteil;
Figur 4: eine schematische perspektivische Außenansicht der Gießform des ersten Ausführungsbeispiels beim Einfüllen von Knochenzementteig;
Figur 5: eine schematische perspektivische Außenansicht des ersten Ausführungsbeispiels vor dem Einsetzen des Gießformoberteils in das Gießformunterteil;
Figur 6: eine schematische Aufsicht auf die geschlossene Gießform des ersten Ausführungsbeispiels mit eingezeichneter Schnittebene B;
Figur 7: eine schematische Querschnittansicht des ersten Ausführungsbeispiels im geschlossenen Zustand auf den Schnitt B nach Figur 6;
Figur 8: eine schematische perspektivische gewinkelte Querschnittansicht auf die mit Knochenzementteig gefüllte Gießform des ersten Ausführungsbeispiels im geschlossenen Zustand;
Figur 9: eine schematische perspektivische Ansicht auf die Vorrichtung nach Figur 8 ohne Deckel;
Figur 10: eine schematische perspektivische Seitenansicht auf einen Spacer, der mit der Vorrichtung des ersten Ausführungsbeispiels nach den Figuren 1 bis 9 hergestellt worden ist;
Figur 11: eine schematische Querschnittansicht auf den Spacer nach Figur 10 mit abgetrennten Abstandhaltern;
Figur 12: eine schematische perspektivische Ansicht auf die Einzelteile eines zweiten Ausführungsbeispiels der vorliegenden Erfindung als Vorrichtung zur Herstellung eines Spacers für ein Schultergelenk;
Figur 13: eine schematische perspektivische Außenansicht des zweiten Ausführungsbeispiels nach Figur 12 vor dem Anordnen des Metallkerns in dem Gießformunterteil;
Figur 14: eine schematische perspektivische Außenansicht der Gießform des zweiten Ausführungsbeispiels beim Einfüllen von Knochenzementteig;
Figur 15: eine schematische perspektivische Außenansicht des zweiten Ausführungsbeispiels vor dem Einsetzen des Gießformoberteils in das Gießformunterteil;
Figur 16: eine schematische perspektivische Ansicht auf die geschlossene Gießform des zweiten Ausführungsbeispiels;
Figur 17: eine schematische perspektivische Ansicht des ersten Ausführungsbeispiels beim Entnehmen des ausgehärteten Spacers;
Figur 18: eine schematische perspektivische Seitenansicht auf den Spacer, der mit der Vorrichtung des zweiten Ausführungsbeispiels nach den Figuren 12 bis 17 hergestellt worden ist;
Figur 19: eine schematische Aufsicht auf die geschlossene Gießform des zweiten Ausführungsbeispiels mit eingezeichneten Schnittebenen A und B;
Figur 20: eine schematische Querschnittansicht des zweiten Ausführungsbeispiels im geschlossenen Zustand auf den Schnitt B nach Figur 19;
Figur 21: eine schematische Querschnittansicht des zweiten Ausführungsbeispiels im geschlossenen Zustand auf den Schnitt A nach Figur 19;
Figur 22: eine schematische perspektivische Querschnittansicht auf die mit Knochenzementteig gefüllte Gießform des zweiten Ausführungsbeispiels im geschlossenen Zustand;
Figur 23: eine schematische perspektivische Ansicht auf die Einzelteile eines dritten Ausführungsbeispiels der vorliegenden Erfindung als Vorrichtung zur Herstellung zweier Komponenten eines artikulierenden Spacers für ein Kniegelenk;
Figur 24: eine schematische perspektivische Außenansicht des dritten Ausführungsbeispiels nach Figur 23 mit geöffneten Gießformunterteilen;
Figur 25: eine schematische perspektivische Außenansicht der Gießformen des dritten Ausführungsbeispiels beim Einfüllen von Knochenzementteig;
Figur 26: eine schematische perspektivische Außenansicht des dritten Ausführungsbeispiels vor dem Einsetzen der Gießformoberteile in die Gießformunterteile;
Figur 27: eine schematische perspektivische Außenansicht des dritten Ausführungsbeispiels während dem Einsetzen der Gießformoberteile in die Gießformunterteile;
Figur 28: eine schematische perspektivische Ansicht auf die geschlossenen Gießformen des dritten Ausführungsbeispiels;
Figur 29: eine schematische perspektivische Querschnittansicht der mit Knochenzementteig gefüllten Gießformen des dritten Ausführungsbeispiels im geschlossenen Zustand;
Figur 30: eine schematische perspektivische Außenansicht des dritten Ausführungsbeispiels bei dem Herausziehen der Gießformoberteile aus den Gießformunterteilen;
Figur 31: eine schematische perspektivische Ansicht auf die Einzelteile des dritten Ausführungsbeispiels mit den beiden Spacern für das Kniegelenk;
Figur 32: eine schematische perspektivische Ansicht des dritten Ausführungsbeispiels mit geschlossenen Gießformen und Standfüßen für die Gießformen;
Figur 33: eine schematische Querschnittansicht der Gießformen des dritten Ausführungsbeispiels im geschlossenen Zustand;
Figur 34: eine schematische perspektivische Ansicht auf die beiden Teile des Spacers, der mit der Vorrichtung nach dem dritten Ausführungsbeispiel hergestellt wurden; und
Figur 35: eine schematische Aufsicht auf die Gießformen mit abgenommenem Deckel des dritten Ausführungsbeispiels.

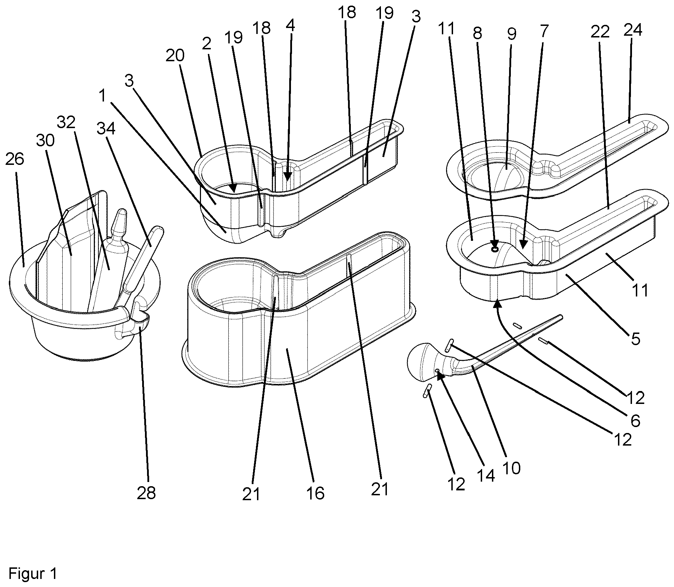

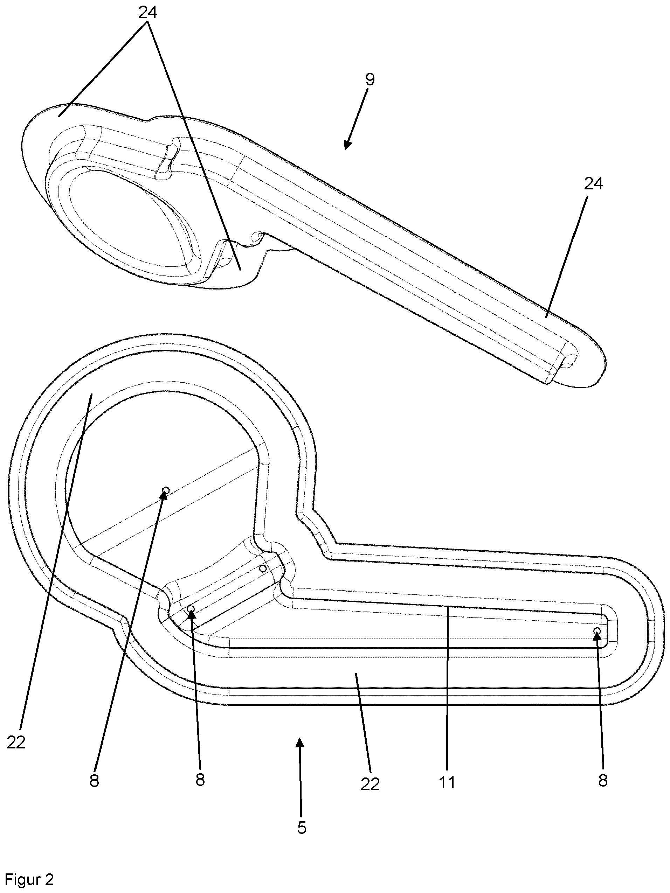

In den Figuren 1 bis 9 sind Abbildungen eines ersten Ausführungsbeispiels einer erfindungsgemäßen Vorrichtung zur Herstellung eines Spacers für ein Hüftgelenk und Teile der Vorrichtung in verschiedenen Ansichten gezeigt. Die Figuren 1 bis 11 zeigen dabei den Ablauf eines ersten Ausführungsbeispiels eines erfindungsgemäßen Verfahrens, das mit der Vorrichtung nach dem ersten Ausführungsbeispiel durchgeführt wird.

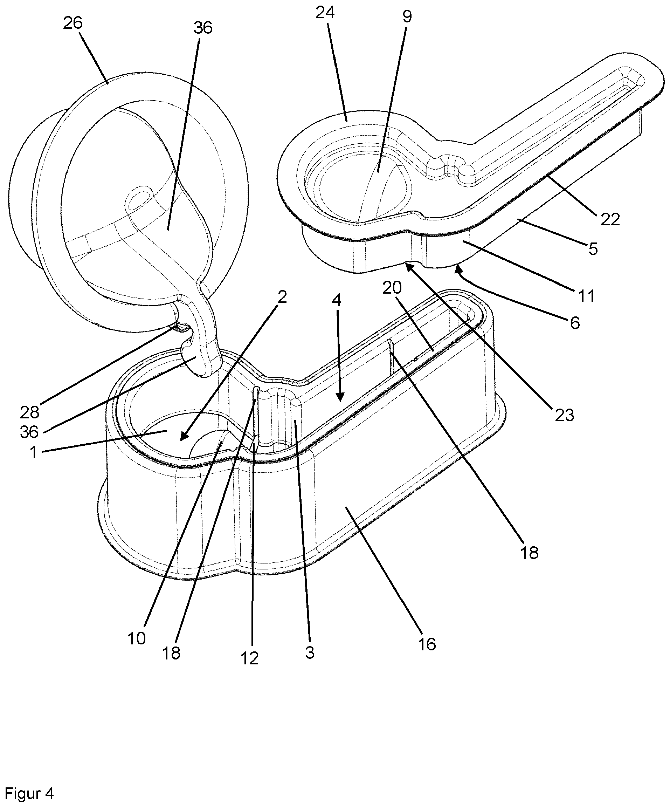

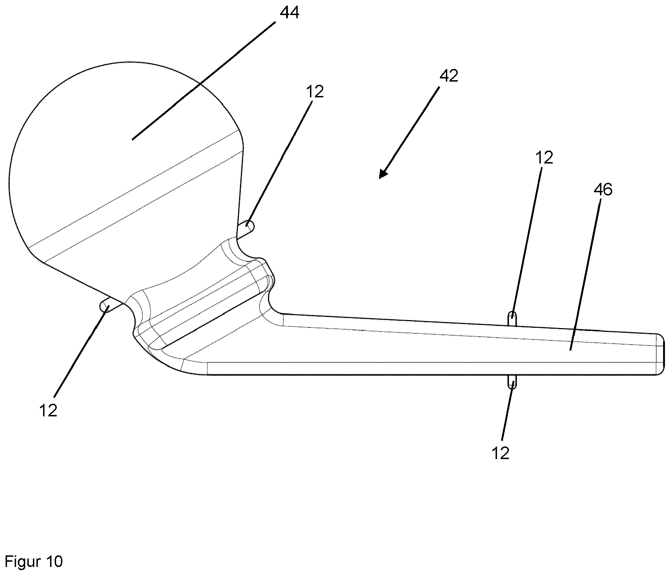

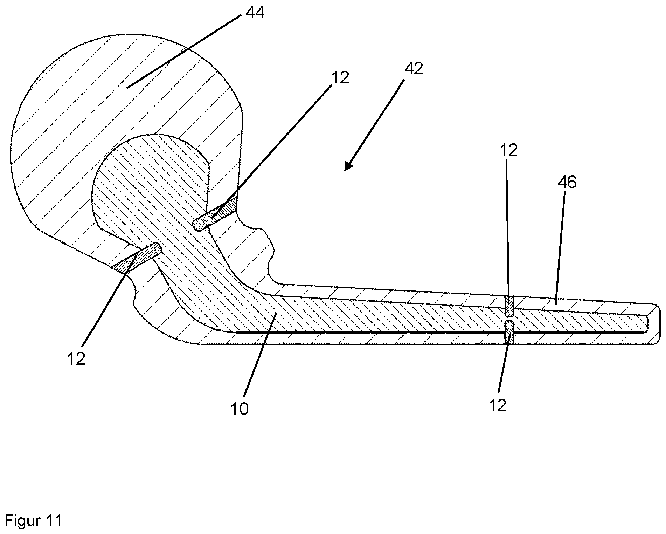

Die erste erfindungsgemäße Vorrichtung ist zur Herstellung eines Spacers 42 (siehe Figuren 10 und 11) für ein Hüftgelenk geeignet und vorgesehen. Die Vorrichtung weist eine mehrteilige Gießform auf. Die Gießform hat ein Gießformunterteil 1 und ein Gießformoberteil 5. In dem Gießformunterteil 1 ist eine Kavität 2 zur Aufnahme von Knochenzementteig 36 (siehe Figuren 4, 5 und 8) und zum Ausformen eines Teils der Oberfläche des zu erzeugenden Spacers 42 angeordnet. Die Kavität 2 kann eine Hälfte des Spacers 42 formen. An dem Rand der Kavität 2 kann eine Gießformwand 3 angeordnet sein, die den durch die Kavität 2 gebildeten Hohlraum mit einem Innenraum 4 verlängert. Hierzu kann die Gießformwand 3 umlaufend den Rand der Kavität 2 umschließen. Die Wandungen der Gießformwand 3 können parallel zueinander ausgerichtet sein, so dass die Gießformwand 3 eine allgemein zylindrische Geometrie aufweist, wobei die Grundfläche des allgemeinen Zylinders durch die Fläche gegeben ist, die den Rand der Kavität 2 begrenzt. Innerhalb der Wandungen der Gießformwand 3 ist der Innenraum 4 zum Einfüllen von Knochenzementteig 36 angeordnet, der mit der Kavität 2 verbunden ist und vorzugsweise auch fluchtet.

Das Gießformoberteil 5 kann vorzugsweise in das Gießformunterteil 1 eingesteckt beziehungsweise eingeschoben werden. Das Gießformoberteil 5 weist an seiner Unterseite eine formgebende Oberfläche 6 auf, mit der ein weiterer Teil der Oberfläche des zu erzeugenden Spacers 42 ausformbar ist, bevorzugt der restliche Teil der der Oberfläche des zu erzeugenden Spacers 42 ausformbar ist. Die formgebende Oberfläche 6 kann zusammen mit der Kavität 2 die gesamte Oberfläche des Spacers 42 definieren oder zumindest 90% der gesamten Oberfläche des Spacers 42 definieren. Die formgebende Oberfläche 6 kann eine Vertiefung in dem Gießformoberteil 5 bilden. Es ist auch möglich, dass ein Teil der Gießformwand 3 einen Teil der Oberfläche des Spacers 42 formt oder ein oder mehrere Einsätze (nicht gezeigt) zusätzlich in die Kavität 2 eingesetzt und/oder an die formgebende Oberfläche 6 angelegt werden, die einen Teil der Oberfläche des Spacers 42 formen.

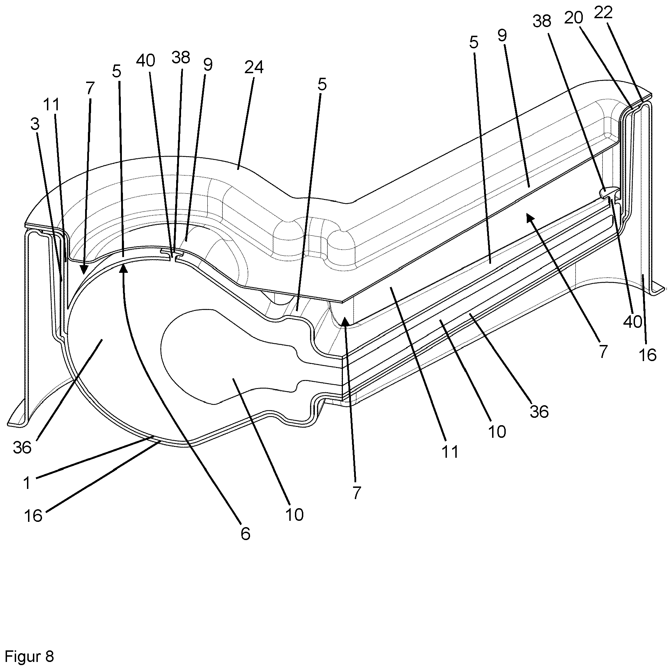

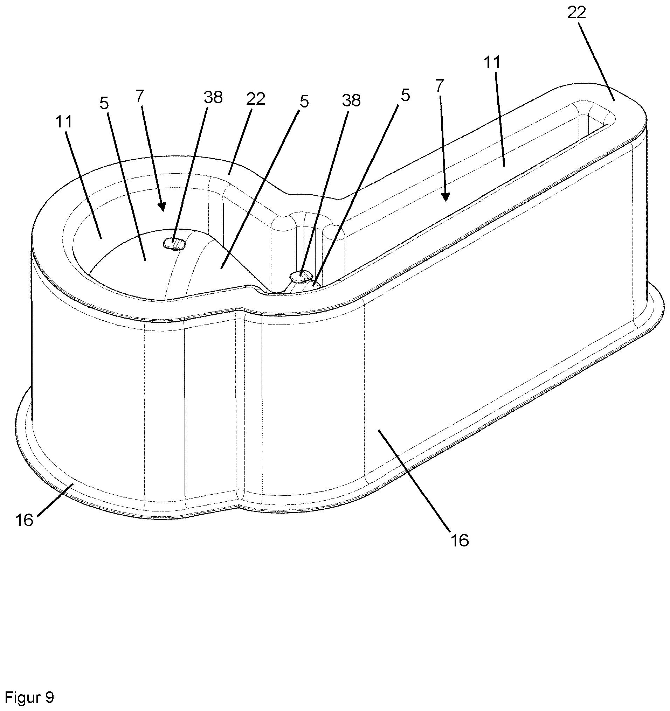

An der der formgebenden Oberfläche 6 gegenüberliegenden Seite des Gießformoberteils 5 kann ein Behälter 7 zur Aufnahme von überschüssigem Knochenzementteig 38 (siehe Figuren 8 und 9) angeordnet sein. In der formgebenden Oberfläche 6 können Durchbrechungen 8 vorhanden sein, die die Seite der formgebenden Oberfläche 6 mit dem Behälter 7 für den überschüssigen Knochenzementteig 38 durchgehend verbinden. Die Durchbrechungen 8 sind vorzugsweise an den Stellen der formgebenden Oberfläche 6 angeordnet, die bei ordnungsgemäßer Aufstellung der Vorrichtung die höchsten formgebenden Punkte der Gießform sind. Hierdurch können in der Gießform eingeschlossene Gase durch die Durchbrechungen 8 entweichen und so Lufteinschlüsse in dem herzustellenden Spacer 42 vermieden werden.

Der Behälter 7 kann mit einem Deckel 9 für den Knochenzementteig 36 undurchlässig nach außen verschlossen sein oder verschlossen werden. Ausgehend von der Rückseite der formgebenden Oberfläche 6 kann sich eine Innenwand 11 weg erstrecken, die den Behälter 7 begrenzt. Die Innenwand 11 kann außen passgenau mit der Gießformwand 3 fluchten. Hierdurch kann das Gießformoberteil 5 passgenau in die Gießformwand 3 des Gießformunterteils 1 eingesteckt werden und so der Knochenzementteig 36 innen von der Gießformwand 3 gesammelt und in die Kavität 2 und die formgebende Oberfläche 6 gedrückt werden. Hierzu kann es auch ausreichen, wenn sich die Gießformwand 3 in Richtung der Kavität 2 verjüngt, wobei die Verjüngung hierzu einen spitzen Winkel aufweisen muss.

Der Behälter 7 kann gasdurchlässig mit der Umgebung verbunden sein. Hierzu kann vorgesehen sein, dass der Deckel 9 nicht druckdicht schließt und/oder kleine Entlüftungsöffnungen (in den Figuren nicht zu sehen) in dem Deckel und/oder der Innenwand 11 angeordnet sind.

Ein Metallkern 10 kann als Armierung für den Spacer 42 vorgesehen sein. Der Metallkern 10 kann hierzu mit Hilfe von stiftförmigen Abstandhaltern 12 aus ausgehärtetem PMMA von der Innenseite der Kavität 2 und von der Gießformwand 3 beabstandet gehalten werden, so dass der Knochenzementteig 36 den Metallkern 10 zwischen dem Metallkern 10 einerseits und der Innenseite der Kavität 2 und der formgebenden Oberfläche 6 andererseits vollständig umfließen kann. Zur Positionierung der Abstandhalter 12 können in dem Metallkern 10 Bohrungen 14 zur Aufnahme eines Endes der stiftförmigen Abstandhalter 12 vorgesehen sein. In der Innenseite der Kavität 2 können ebenfalls passende Vertiefungen zur Aufnahme des gegenüberliegenden Endes der stiftförmigen Abstandhalter 12 angeordnet sein.

Zur korrekten Positionierung der Gießform mit dem Gießformunterteil 1 unten und der Gießformwand 3 senkrecht nach oben kann ein Standfuß 16 vorgesehen sein, in den das Gießformunterteil 1 eingelegt oder eingesteckt werden kann. Der Standfuß 16 kann zur Auflage auf einer ebenen Oberfläche wie einem Tisch vorgesehen sein.

Das Gießformunterteil 1, das Gießformoberteil 5, der Deckel 9 und der Standfuß 16 können kostengünstig aus einer Kunststofffolie oder aus mehreren miteinander verbundenen Kunststofffolien hergestellt sein, insbesondere durch Spritzguss oder durch Ziehen hergestellt sein. Im zusammengesetzten Zustand stabilisieren sich dabei die aus den Kunststofffolien hergestellten Teile der Vorrichtung gegenseitig mechanisch. Die Kunststofffolie(n) bestehen vorzugsweise aus einem Polyolefin, einem Polyethylen (PET) oder einem mit Glycol modifiziertem PET (PETG). Bei der Verwendung von mehreren Folien können diese mit einem Klebstoff oder durch erhöhte Temperatur miteinander laminiert sein.

In der Gießformwand 3 können innen liegende Nuten 18 und außen liegende Stege 19 angeordnet sein, die von einem Anschlag 20 an der der Kavität 2 gegenüberliegenden Seite der Gießformwand 3 bis hinab zum Rand der Kavität 2 reichen. Die Stege 19 können das Volumen zum Ausformen der Nuten 18 beinhalten. Die von dem Metallkern 10 abstehenden Enden der stiftförmigen Abstandhalter 12, die in die Bohrungen 14 des Metallkerns eingesteckt sind, können entlang dieser Nuten 18 bis zur Kavität 2 geführt werden.

In dem Standfuß 16 können innen zu den Stegen 19 passende Nuten 21 vorgesehen sein. Dadurch kann das Gießformunterteil 1 geführt in den Standfuß 16 eingeschoben werden und das Gießformunterteil 1 löst sich nicht so leicht von dem Standfuß 16 und bewegt sich nicht so leicht gegen den Standfuß 16.

Zur Fixierung der Abstandhalter 12 und damit des Metallkerns 10 können am Rand der formgebenden Oberfläche 6 des Gießformoberteils 5 passende Ausnehmungen 23 angeordnet sein. Die Ausnehmungen 23 können derart an dem Rand der formgebenden Oberfläche 6 angeordnet sein, dass sie entlang der Nuten 18 der Gießformwand 3 bewegt werden, wenn das Gießformoberteil 5 in die Gießformwand 3 des Gießformunterteils 1 eingeschoben wird. Die vom Metallkern 10 abstehenden Enden der Abstandhalter 12 werden so in der Gießform zwischen dem Gießformoberteil 5 und dem Gießformunterteil 1 eingeklemmt.

Der Anschlag 20 kann als ein senkrecht zur Gießformwand 3 abstehender umlaufender bandförmiger Rand an der von der Kavität 2 abgewandten Seite der Gießformwand 3 angeordnet sein. Der Anschlag 20 kann begrenzen, wie weit das Gießformoberteil 5 in das Gießformunterteil 1 eingeschoben werden kann. Hierzu kann das Gießformoberteil 5 einen Gegenanschlag 22 in Form eines umlaufenden Randes aufweisen, der senkrecht von der Innenwand 11 absteht und der an der formgebenden Oberfläche 6 gegenüberliegenden Seite der Innenwand 11 angeordnet ist. Bei vollständig in das Gießformunterteil 1 eingeschobenem Gießform oberteil 5 liegt der Gegenanschlag 22 an dem Anschlag 20 an. In gleicher Weise kann an dem Deckel 9 ein Deckelrand 24 angeordnet sein, der an der dem Anschlag 20 gegenüberliegenden Seite des Gegenanschlags 22 anlegbar ist. Dadurch, dass der Anschlag 20, der Gegenanschlag 22 und der Deckelrand 24 von den angrenzenden Teilen in einem Winkel abstehen, wird die Form des Gießformunterteils 1, des Gießformoberteils 5 und des Deckels 9 im zusammengesetzten Zustand mechanisch stabilisiert. Das ist insbesondere dann hilfreich, wenn diese Teile durch Ziehen aus einer Kunststofffolie oder aus mehreren Kunststofffolien gefertigt sind.

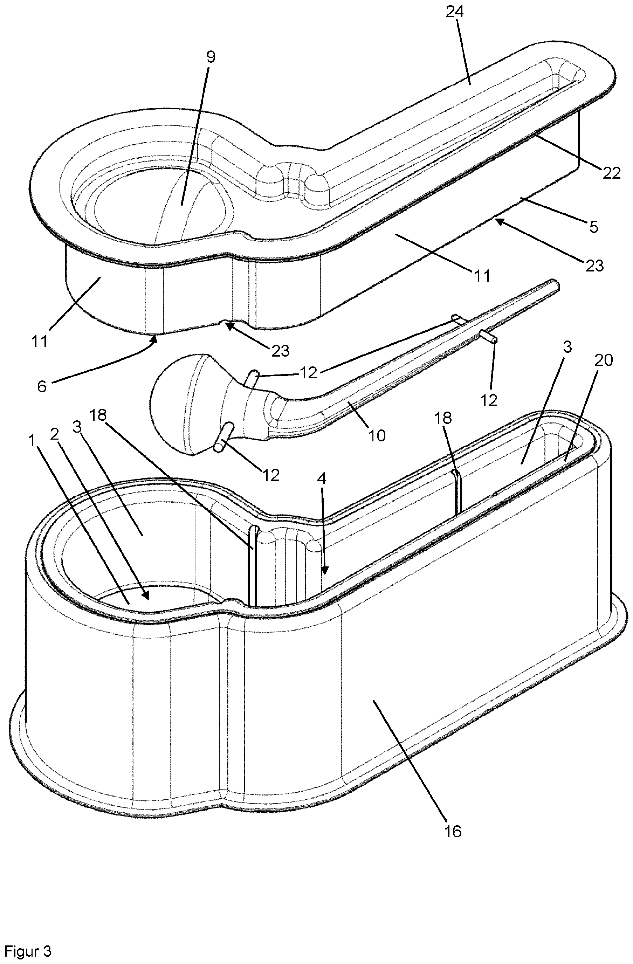

Die Vorrichtung kann des Weiteren einen Mischbecher 26 mit einer Tülle 28 zum Ausgießen von Knochenzementteig 36 aus dem Mischbescher 26 (siehe Figur 3) sowie einen Folienbeutel 30 enthaltend Zementpulver, eine Ampulle 32 enthaltend Monomerflüssigkeit und einen Spatel 34 zum Mischen des Zementpulvers mit der Monomerflüssigkeit in dem Mischbecher 26 aufweisen. Der Knochenzementteig 36 kann dann in dem Mischbecher 26 gemischt werden, bevor er in die Gießform beziehungsweise in das Gießformunterteil 1 gefüllt wird. Der Knochenzementteig 36 kann aber auch auf andere Weise hergestellt werden, bevor er in die Gießform gefüllt wird. Die Vorrichtung benötigt also nicht zwingend den Mischbecher 26 und auch nicht den Knochenzementteig 36 oder dessen Ausgangskomponenten. Die Vorrichtung kann grundsätzlich mit irgendeinem anderen bekannten System zum Herstellen eines Knochenzementteigs angewendet und verwendet werden, wie beispielsweise mit einem geeigneten Kartuschensystem zum Lagern und Mischen von Knochenzementteig.

Im Folgenden wird der Ablauf eines ersten Ausführungsbeispiels eines erfindungsgemäßen Verfahrens anhand der Figuren 1 bis 11 beschrieben.

Die Vorrichtung kann zunächst steril aus einer Verpackung (nicht gezeigt) entnommen werden und kann dann so wie in Figur 1 gezeigt oder wie in Figur 3 oder 4 gezeigt vorliegen. Sofern das Gießformunterteil 1 nicht bereits in den Standfuß 16 eingesetzt ist, kann das Gießformunterteil 1 über die Nuten 21 in den Standfuß 16 eingeschoben werden. Sofern noch nicht darin enthalten, kann der Metallkern 10 in die Kavität 2 eingesetzt werden (siehe Figur 3). Dabei gleiten die abstehenden Enden der Abstandhalter 12 in den Nuten 18 der Gießformwand 3.

Der Knochenzementteig 36 kann durch Mischen des Zementpulvers aus der Folienbeutel 30 und der Monomerflüssigkeit aus der Ampulle 32 in dem Mischbecher 26 mit Hilfe des Spatels 34 gemischt werden. Alternativ kann der Knochenzementteig 36 auch auf irgendeine andere Weise hergestellt werden.

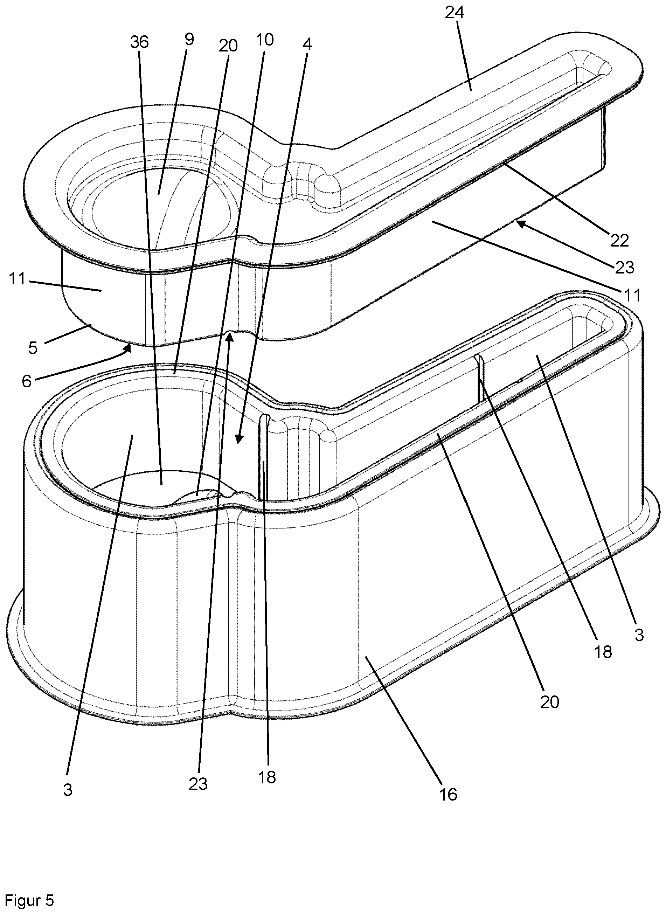

Wenn der Metallkern 10 in die Kavität 2 beziehungsweise in den Innenraum 4 eingesetzt ist, kann der Knochenzementteig 36 im Überschuss in den Innenraum 4 und die Kavität 2 eingefüllt werden (siehe Figur 4). Anschließend kann das Gießformoberteil 5 in das Gießformunterteil 1 beziehungsweise in die Gießformwand 3 eingesetzt und eingeschoben werden. Dazu kann der Behälter 7 mit dem Deckel 9 geschlossen sein (siehe Figur 5).

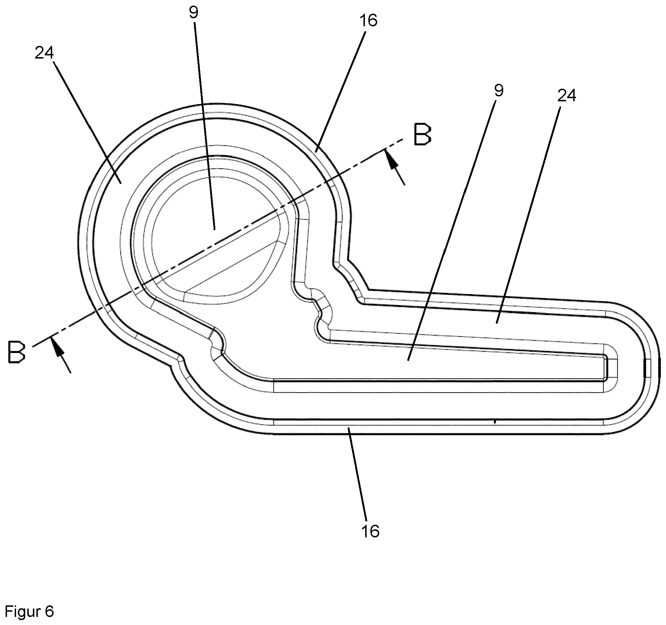

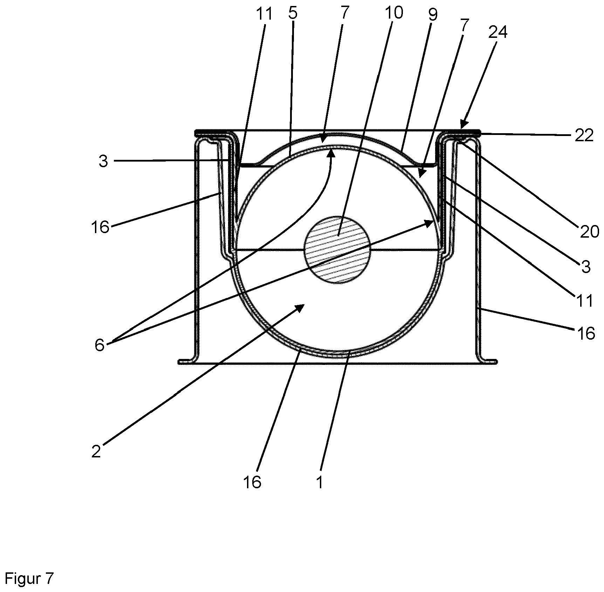

Das Gießformoberteil 5 kann in den Innenraum 4 des Gießformunterteils 1 eingeschoben werden, bis der Gegenanschlag 22 an dem Anschlag 20 anliegt (siehe hierzu die Figuren 6 und 7, wobei dort der in der Gießform eingeschlossene Knochenzementteig nicht dargestellt ist). Beim Einschieben des Gießformoberteils 5 kann der enthaltene Knochenzementteig 36 in die Kavität 2 gepresst werden und es verringert sich fortwährend das Volumen innerhalb der Gießform. Überschüssiger Knochenzementteig 38 wird durch die Durchbrechungen 8 hindurch in den Behälter 7 gedrückt (siehe Figuren 8 und 9). Mit Hilfe des Deckels 9 kann der überschüssige Knochenzementteig 38 in dem Behälter 7 eingeschlossen werden.

Anschließend kann der Knochenzementteig 36 in der Gießform aushärten, wobei seine Oberfläche durch die Oberfläche der Kavität 2 des Gießformunterteils 1 und die formgebende Oberfläche 6 des Gießformoberteils 5 geformt wird. Dabei entsteht der Spacer 42, wie er beispielsweise in den Figuren 10 und 11 gezeigt ist. Beim Aushärten des Knochenzementteigs 36 können in den Durchbrechungen 8 Stege 40 gebildet werden, die den Spacer 42 mit dem ausgehärteten überschüssigen Knochenzementteig 38 in dem Behälter 7 verbinden. Der Spacer 42 wird entformt, indem er von dem Gießformunterteil 1 und dem Gießformoberteil 5 gelöst wird. Die Stege 40 können dabei abgeschert oder abgebrochen werden. Um ein einfaches Entformen sicherzustellen, müssen die Stege 40 einen derart geringen Durchmesser haben, dass sie manuell abgebrochen oder abgeschert werden können, wenn der Spacer 42 vom Gießformoberteil 5 getrennt wird. Hierzu hat es sich bewährt, wenn die Stege 40 einen Durchmesser von maximal 2,5 cm bevorzugt von maximal 2 cm haben. Dementsprechend sollten die Durchbrechungen 8 einen Innendurchmesser von maximal 2,5 cm, bevorzugt von maximal 2 cm haben. Um das Trennen der Stege 40 weiter zu vereinfachen, können auch kleinere Durchmesser gewählt werden. Allerdings sollten die Durchbrechungen 8 nicht einen Durchmesser von weniger als 0,2 mm haben, damit der Knochenzementteig 36 noch ohne zu großen Widerstand durch die Durchbrechungen 8 hindurch gepresst werden kann. Ansonsten kann abhängig von der Viskosität des Knochenzementteigs 36 der Widerstand beim Einschieben des Gießformoberteils 5 in das Gießformunterteil 1 zu groß werden, um noch manuell durchgeführt werden zu können oder um eine Beschädigung oder Zerstörung der Gießform zu vermeiden. Der optimale Durchmesser der Durchbrechungen 8 hängt dabei davon ab, welche Viskosität der verwendete Knochenzementteig 36 hat. Gut geeignete Durchmesser für typische Knochenzemente liegen zwischen 1 mm und 20 mm. Dabei gilt: Je höher die Viskosität des Knochenzementteigs 36 desto größer sollte der Innendurchmesser der Durchbrechungen 8 gewählt werden.

Nach dem Entformen können die vorstehenden Abstandshalter 12, möglicherweise entstandene Grate, die an der Verbindung des Gießformunterteils 1 zum Gießformoberteil 5 entstanden sind, und vorspringende Reste der Stege 40 entfernt werden, beispielsweise durch Abschneiden mit einem Messer oder Skalpell oder durch Abschleifen mit einem Schleifkopf. Als Endergebnis erhält man den Spacer 42, wie er in Figur 11 gezeigt ist.

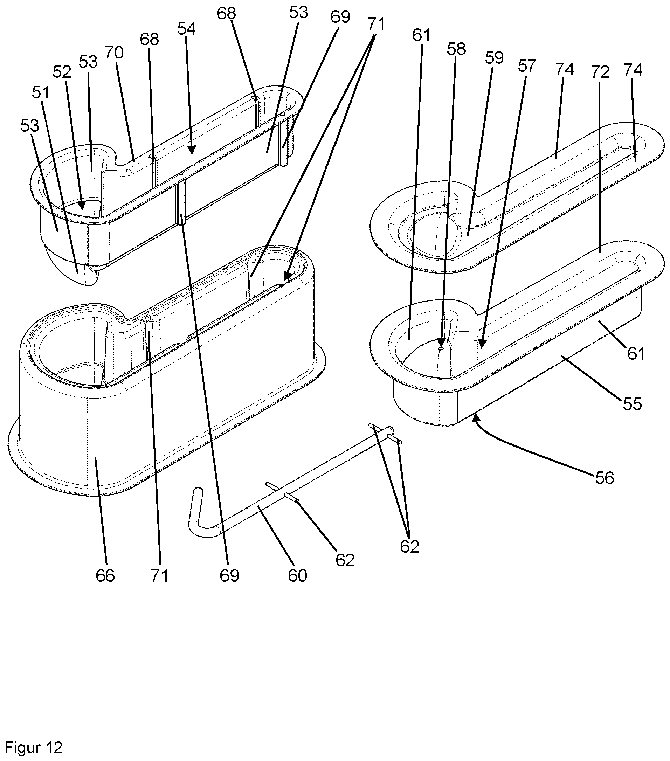

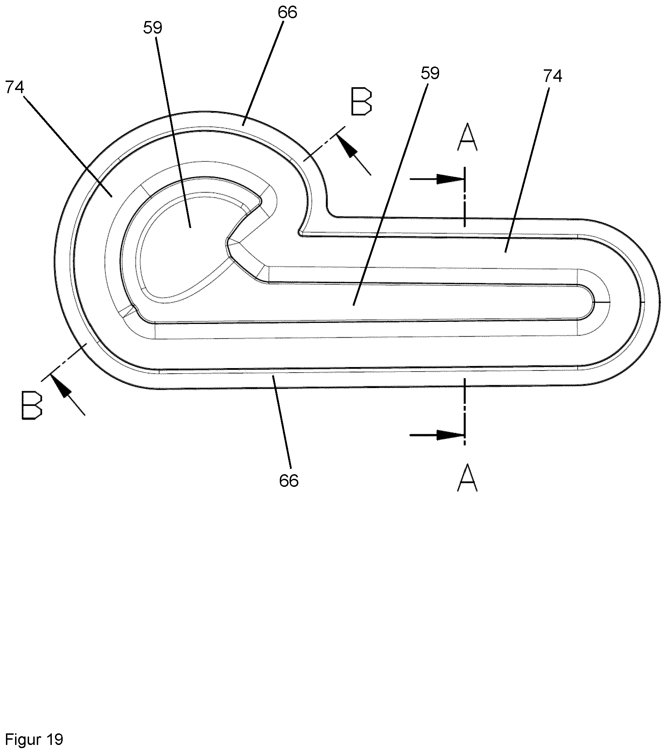

In den Figuren 12 bis 22 sind Abbildungen eines zweiten Ausführungsbeispiels einer erfindungsgemäßen Vorrichtung zur Herstellung eines Spacers für ein Schultergelenk, Teile der Vorrichtung und einen mit der Vorrichtung erzeugten Spacer in verschiedenen Ansichten gezeigt. Die Figuren 12 bis 18 und 22 zeigen dabei den Ablauf eines zweiten Ausführungsbeispiels eines erfindungsgemäßen Verfahrens, das mit der Vorrichtung nach dem zweiten Ausführungsbeispiel durchgeführt wird.

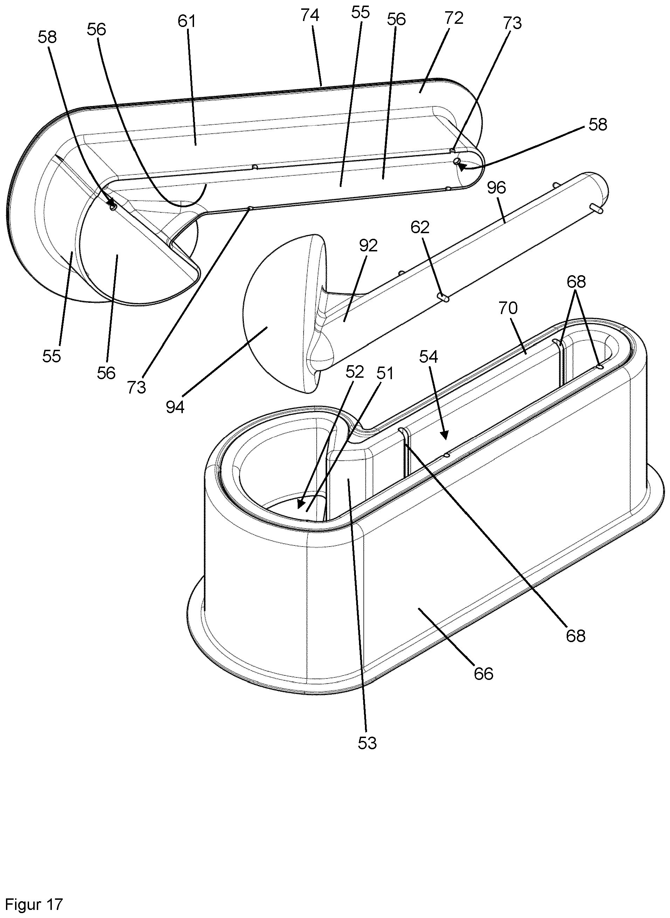

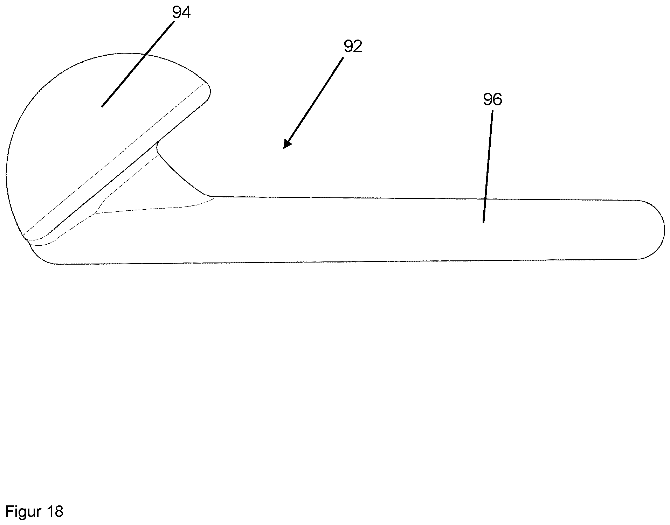

Die zweite erfindungsgemäße Vorrichtung ist zur Herstellung eines Spacers 92 (siehe Figuren 17 und 18) für ein Schultergelenk geeignet und vorgesehen. Die Vorrichtung weist eine mehrteilige Gießform auf. Die Gießform hat ein Gießformunterteil 51 und ein Gießformoberteil 55. In dem Gießformunterteil 51 ist eine Kavität 52 zur Aufnahme von Knochenzementteig 36 (siehe Figuren 12 bis 15) und zum Ausformen eines Teils der Oberfläche des zu erzeugenden Spacers 92 angeordnet. Die Kavität 52 kann eine Hälfte des Spacers 92 formen. An dem Rand der Kavität 52 kann eine Gießformwand 53 angeordnet sein, die den durch die Kavität 52 gebildeten Hohlraum mit einem Innenraum 54 verlängert. Hierzu kann die Gießformwand 53 umlaufend den Rand der Kavität 52 umschließen. Die Wandungen der Gießformwand 53 können parallel zueinander ausgerichtet sein, so dass die Gießformwand 53 eine allgemein zylindrische Geometrie aufweist, wobei die Grundfläche des allgemeinen Zylinders durch die Fläche gegeben ist, die den Rand der Kavität 52 begrenzt. Innerhalb der Wandungen der Gießformwand 53 ist der Innenraum 54 zum Einfüllen von Knochenzementteig 36 angeordnet, der mit der Kavität 52 verbunden ist und vorzugsweise auch fluchtet.

Das Gießformoberteil 55 kann vorzugsweise in das Gießformunterteil 51 eingesteckt beziehungsweise eingeschoben werden. Das Gießformoberteil 55 weist an seiner Unterseite eine formgebende Oberfläche 56 auf, mit der ein weiterer Teil der Oberfläche des zu erzeugenden Spacers 92 ausformbar ist, bevorzugt der restliche Teil der der Oberfläche des zu erzeugenden Spacers 92 ausformbar ist. Die formgebende Oberfläche 56 kann zusammen mit der Kavität 52 die gesamte Oberfläche des Spacers 92 definieren oder zumindest 90% der gesamten Oberfläche des Spacers 92 definieren. Die formgebende Oberfläche 56 kann eine Vertiefung in dem Gießformoberteil 55 bilden. Es ist auch möglich, dass ein Teil der Gießformwand 53 einen Teil der Oberfläche des Spacers 92 formt oder ein oder mehrere Einsätze (nicht gezeigt) zusätzlich in die Kavität 52 eingesetzt und/oder an die formgebende Oberfläche 56 angelegt werden, die einen Teil der Oberfläche des Spacers 92 formen.

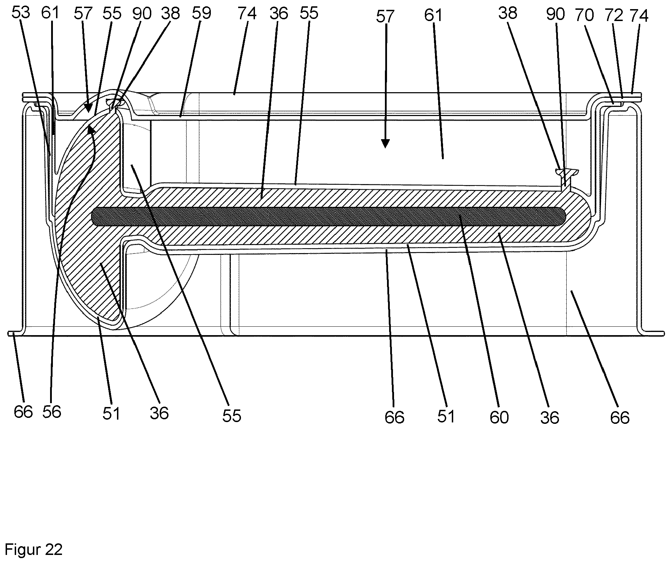

An der der formgebenden Oberfläche 56 gegenüberliegenden Seite des Gießformoberteils 55 kann ein Behälter 57 zur Aufnahme von überschüssigem Knochenzementteig 38 (siehe Figur 22) angeordnet sein. In der formgebenden Oberfläche 56 können Durchbrechungen 58 vorhanden sein, die die Seite der formgebenden Oberfläche 56 mit dem Behälter 57 für den überschüssigen Knochenzementteig 38 durchgehend verbinden. Die Durchbrechungen 58 sind vorzugsweise an den Stellen der formgebenden Oberfläche 56 angeordnet, die bei ordnungsgemäßer Aufstellung der Vorrichtung die höchsten formgebenden Punkte der Gießform sind. Hierdurch können in der Gießform eingeschlossene Gase durch die Durchbrechungen 58 entweichen und so Lufteinschlüsse in dem herzustellenden Spacer 92 vermieden werden.

Der Behälter 57 kann mit einem Deckel 59 für den Knochenzementteig 36 undurchlässig nach außen verschlossen sein oder verschlossen werden. Ausgehend von der Rückseite der formgebenden Oberfläche 56 kann sich eine Innenwand 61 weg erstrecken, die den Behälter 57 begrenzt. Die Innenwand 61 kann außen passgenau mit der Gießformwand 53 fluchten. Hierdurch kann das Gießformoberteil 55 passgenau in die Gießformwand 53 des Gießformunterteils 51 eingesteckt werden und so der Knochenzementteig 36 innen von der Gießformwand 53 gesammelt und in die Kavität 52 und die formgebende Oberfläche 56 gedrückt werden. Hierzu kann es auch ausreichen, wenn sich die Gießformwand 53 in Richtung der Kavität 52 verjüngt, wobei die Verjüngung hierzu einen spitzen Winkel aufweisen muss.

Der Behälter 57 kann gasdurchlässig mit der Umgebung verbunden sein. Hierzu kann vorgesehen sein, dass der Deckel 59 nicht druckdicht schließt und/oder kleine Entlüftungsöffnungen (in den Figuren nicht zu sehen) in dem Deckel und/oder der Innenwand 61 angeordnet sind.

Ein Metallkern 60 kann als Armierung für den Spacer 92 vorgesehen sein. Der Metallkern 60 kann hierzu mit Hilfe von stiftförmigen Abstandhaltern 62 aus ausgehärtetem PMMA von der Innenseite der Kavität 52 und von der Gießformwand 53 beabstandet gehalten werden, so dass der Knochenzementteig 36 den Metallkern 60 zwischen dem Metallkern 60 einerseits und der Innenseite der Kavität 52 und der formgebenden Oberfläche 56 andererseits vollständig umfließen kann. Zur Positionierung der Abstandhalter 62 können in dem Metallkern 60 Bohrungen (nicht zu sehen) zur Aufnahme eines Endes der stiftförmigen Abstandhalter 62 vorgesehen sein. In der Innenseite der Kavität 52 können ebenfalls passende Vertiefungen zur Aufnahme des gegenüberliegenden Endes der stiftförmigen Abstandhalter 62 angeordnet sein.

Zur korrekten Positionierung der Gießform mit dem Gießform unterteil 51 unten und der Gießformwand 53 senkrecht nach oben kann ein Standfuß 66 vorgesehen sein, in den das Gießformunterteil 51 eingelegt oder eingesteckt werden kann. Der Standfuß 66 kann zur Auflage auf einer ebenen Oberfläche wie einem Tisch vorgesehen sein.

Das Gießformunterteil 51, das Gießformoberteil 55, der Deckel 59 und der Standfuß 66 können kostengünstig aus einer Kunststofffolie oder aus mehreren miteinander verbundenen Kunststofffolien hergestellt sein, insbesondere durch Spritzguss oder durch Ziehen hergestellt sein. Im zusammengesetzten Zustand stabilisieren sich dabei die aus den Kunststofffolien hergestellten Teile der Vorrichtung gegenseitig mechanisch. Die Kunststofffolie(n) bestehen vorzugsweise aus einem Polyolefin, einem Polyethylen (PET) oder einem mit Glycol modifiziertem PET (PETG). Bei der Verwendung von mehreren Folien können diese mit einem Klebstoff oder durch erhöhte Temperatur miteinander laminiert sein.

In der Gießformwand 53 können innen liegende Nuten 68 und außen liegende Stege 69 angeordnet sein, die von einem Anschlag 70 an der der Kavität 52 gegenüberliegenden Seite der Gießformwand 53 bis hinab zum Rand der Kavität 52 reichen. Die Stege 69 können das Volumen zum Ausformen der Nuten 68 beinhalten. Die von dem Metallkern 60 abstehenden Enden der stiftförmigen Abstandhalter 62, können entlang dieser Nuten 68 bis zur Kavität 52 geführt werden.

In dem Standfuß 66 können innen zu den Stegen 69 passende Nuten 71 vorgesehen sein. Dadurch kann das Gießformunterteil 51 geführt in den Standfuß 66 eingeschoben werden und das Gießformunterteil 51 löst sich nicht so leicht von dem Standfuß 66 und bewegt sich nicht so leicht gegen den Standfuß 66.

Zur Fixierung der Abstandhalter 62 und damit des Metallkerns 60 können am Rand der formgebenden Oberfläche 56 des Gießformoberteils 55 passende Ausnehmungen 73 angeordnet sein. Die Ausnehmungen 73 können derart an dem Rand der formgebenden Oberfläche 56 angeordnet sein, dass sie entlang der Nuten 68 der Gießformwand 53 bewegt werden, wenn das Gießformoberteil 55 in die Gießformwand 53 des Gießformunterteils 51 eingeschoben wird. Die vom Metallkern 60 abstehenden Enden der Abstandhalter 62 werden so in der Gießform zwischen dem Gießformoberteil 55 und dem Gießformunterteil 51 eingeklemmt.

Der Anschlag 70 kann als ein senkrecht zur Gießformwand 53 abstehender umlaufender bandförmiger Rand an der von der Kavität 52 abgewandten Seite der Gießformwand 53 angeordnet sein. Der Anschlag 70 kann begrenzen, wie weit das Gießformoberteil 55 in das Gießformunterteil 51 eingeschoben werden kann. Hierzu kann das Gießformoberteil 55 einen Gegenanschlag 72 in Form eines umlaufenden Randes aufweisen, der senkrecht von der Innenwand 61 absteht und der an der formgebenden Oberfläche 56 gegenüberliegenden Seite der Innenwand 61 angeordnet ist. Bei vollständig in das Gießformunterteil 51 eingeschobenem Gießformoberteil 55 liegt der Gegenanschlag 72 an dem Anschlag 70 an. In gleicher Weise kann an dem Deckel 59 ein Deckelrand 74 angeordnet sein, der an der dem Anschlag 70 gegenüberliegenden Seite des Gegenanschlags 72 anlegbar ist. Dadurch, dass der Anschlag 70, der Gegenanschlag 72 und der Deckelrand 74 von den angrenzenden Teilen in einem Winkel abstehen, wird die Form des Gießformunterteils 51, des Gießformoberteils 55 und des Deckels 59 im zusammengesetzten Zustand mechanisch stabilisiert. Das ist insbesondere dann hilfreich, wenn diese Teile durch Ziehen aus einer Kunststofffolie oder aus mehreren Kunststofffolien gefertigt sind.

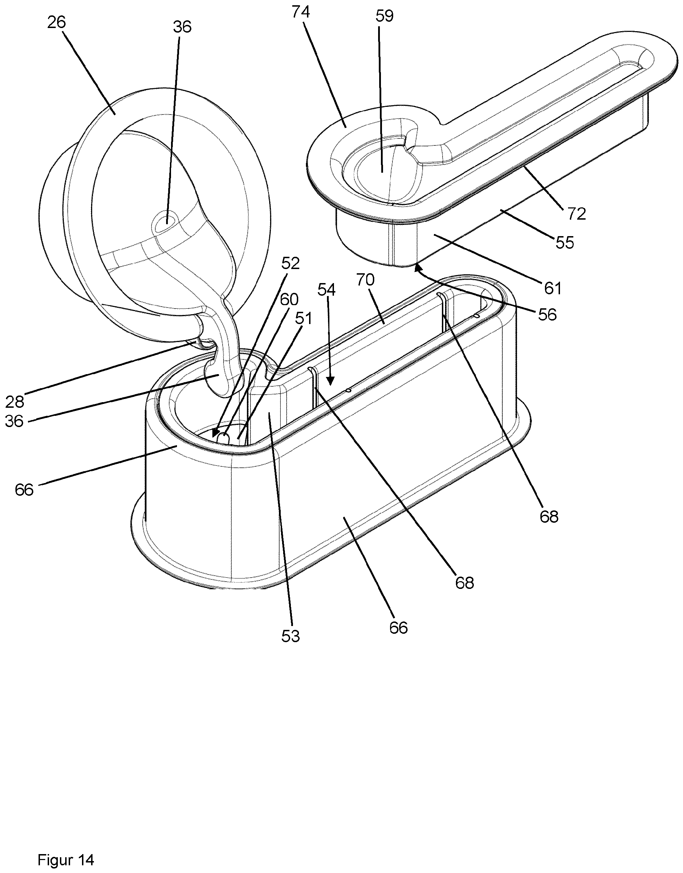

Die Vorrichtung kann des Weiteren einen Mischbecher 26 mit einer Tülle 28 zum Ausgießen von Knochenzementteig 36 aus dem Mischbescher 26 (siehe Figur 14) aufweisen. Die Vorrichtung kann auch die Ausgangskomponenten (nicht gezeigt) des Knochenzementteigs 36 in separaten Behältern aufweisen. Der Knochenzementteig 36 kann dann in dem Mischbecher 26 gemischt werden, bevor er in die Gießform beziehungsweise in das Gießformunterteil 51 gefüllt wird. Der Knochenzementteig 36 kann aber auch auf andere Weise hergestellt werden, bevor er in die Gießform gefüllt wird. Die Vorrichtung benötigt also nicht zwingend den Mischbecher 26 und auch nicht den Knochenzementteig 36 oder dessen Ausgangskomponenten. Die Vorrichtung kann grundsätzlich mit irgendeinem anderen bekannten System zum Herstellen eines Knochenzementteigs angewendet und verwendet werden, wie beispielsweise mit einem geeigneten Kartuschensystem zum Lagern und Mischen von Knochenzementteig.

Im Folgenden wird der Ablauf eines ersten Ausführungsbeispiels eines erfindungsgemäßen Verfahrens anhand der Figuren 12 bis 22 beschrieben.

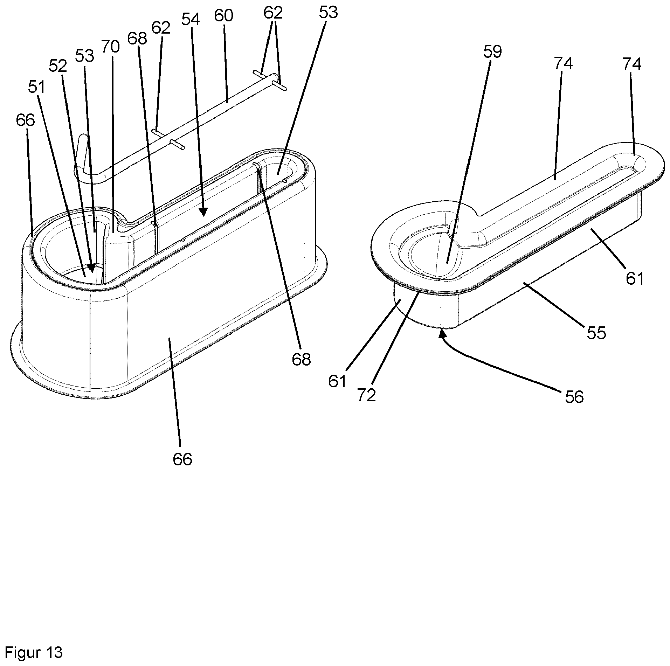

Die Vorrichtung kann zunächst steril aus einer Verpackung (nicht gezeigt) entnommen werden und kann dann so wie in Figur 12 gezeigt oder wie in Figur 13 gezeigt vorliegen. Sofern das Gießformunterteil 51 nicht bereits in den Standfuß 66 eingesetzt ist, kann das Gießformunterteil 51 über die Nuten 71 in den Standfuß 66 eingeschoben werden. Sofern noch nicht darin enthalten, kann der Metallkern 60 in die Kavität 52 eingesetzt werden (siehe Figur 13). Dabei gleiten die abstehenden Enden der Abstandhalter 62 in den Nuten 68 der Gießformwand 53.

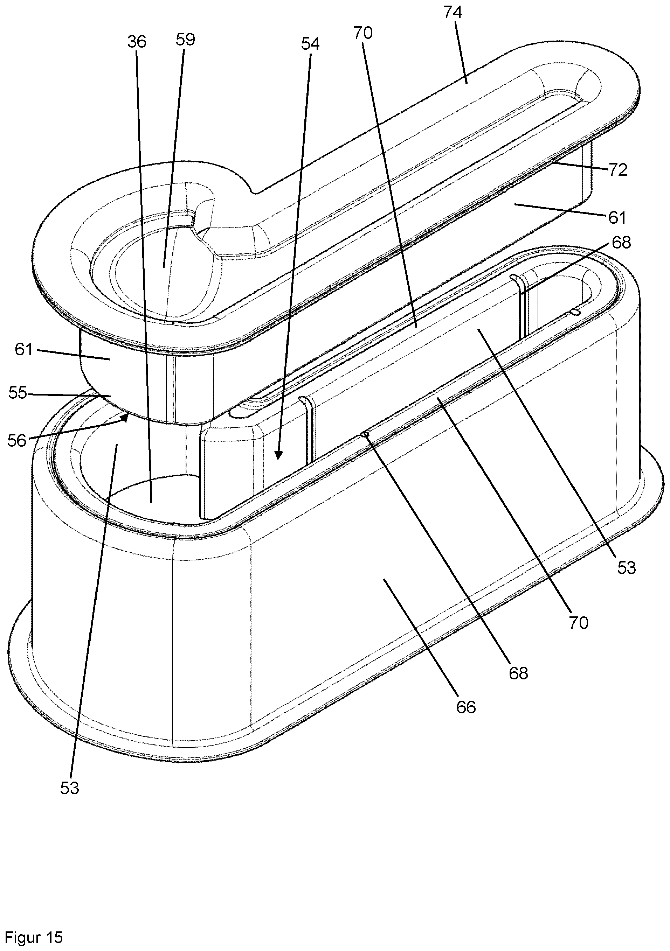

Wenn der Metallkern 60 in die Kavität 52 beziehungsweise in den Innenraum 54 eingesetzt ist, kann der Knochenzementteig 36 im Überschuss in den Innenraum 54 und die Kavität 52 eingefüllt werden (siehe Figur 14). Anschließend kann das Gießformoberteil 55 in das Gießformunterteil 51 beziehungsweise in die Gießformwand 53 eingesetzt und eingeschoben werden. Dazu kann der Behälter 57 mit dem Deckel 59 geschlossen sein (siehe Figur 15).

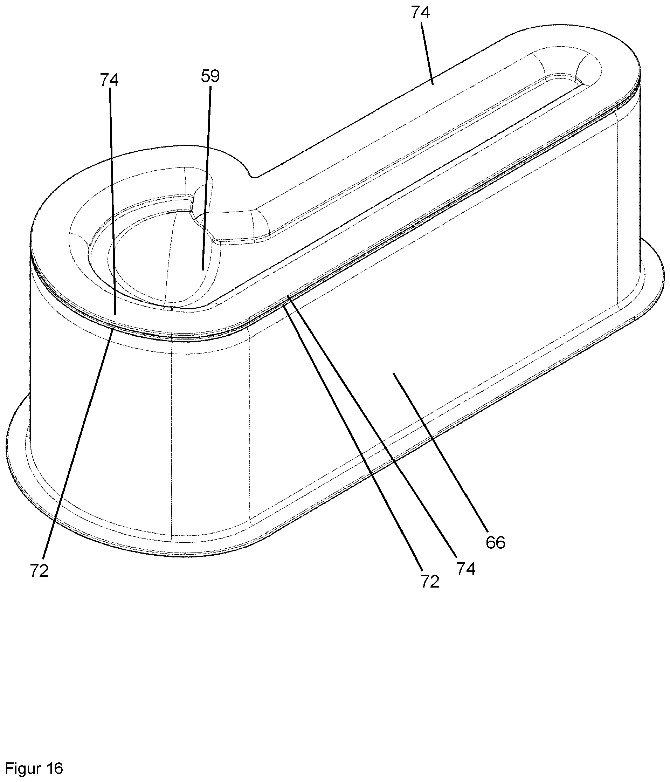

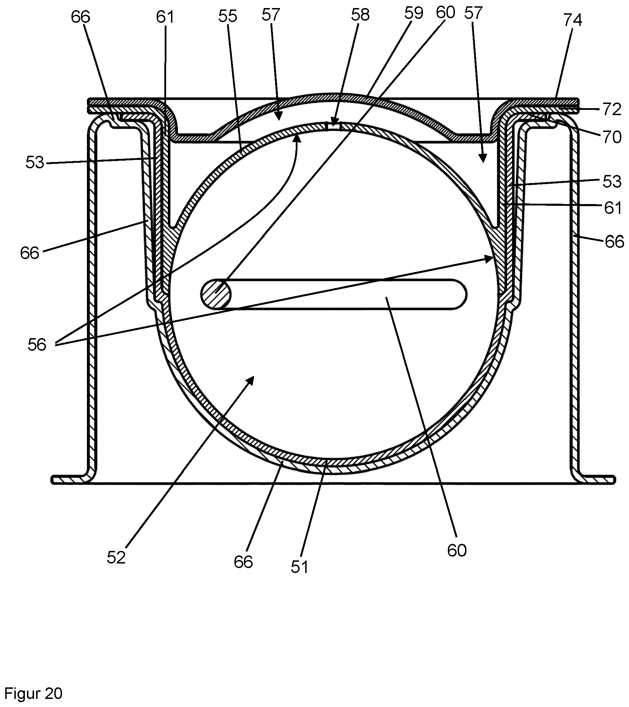

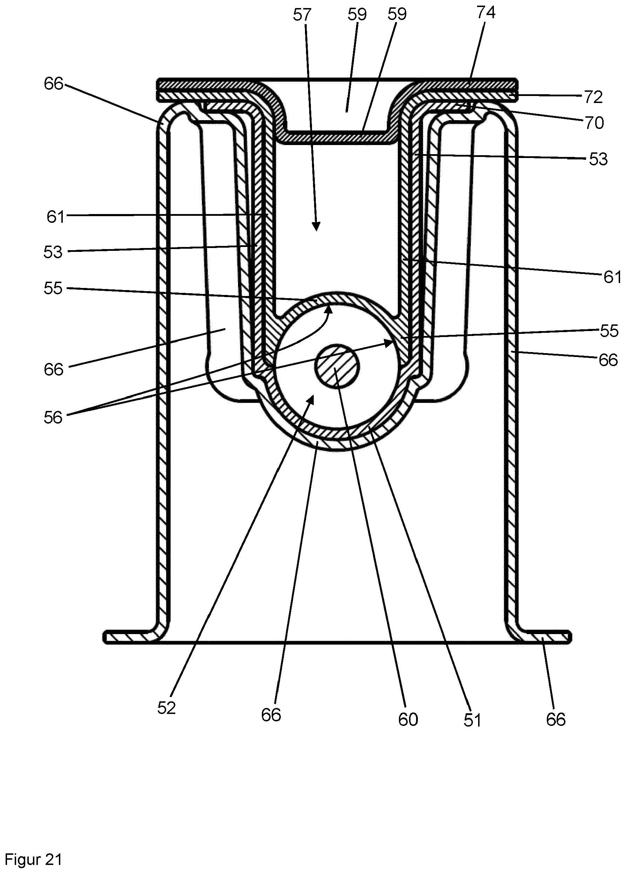

Das Gießformoberteil 55 kann in den Innenraum 54 des Gießformunterteils 51 eingeschoben werden, bis der Gegenanschlag 72 an dem Anschlag 70 anliegt (siehe hierzu die Figuren 16, 20 und 21, wobei dort der in der Gießform eingeschlossene Knochenzementteig nicht dargestellt (Figuren 20 und 21) beziehungsweise nicht zu sehen ist (Figur 16)). Beim Einschieben des Gießformoberteils 55 kann der enthaltene Knochenzementteig 36 in die Kavität 52 gepresst werden und es verringert sich fortwährend das Volumen innerhalb der Gießform. Überschüssiger Knochenzementteig 38 wird durch die Durchbrechungen 58 hindurch in den Behälter 57 gedrückt (siehe Figur 22). Mit Hilfe des Deckels 59 kann der überschüssige Knochenzementteig 38 in dem Behälter 57 eingeschlossen werden.

Anschließend kann der Knochenzementteig 36 in der Gießform aushärten, wobei seine Oberfläche durch die Oberfläche der Kavität 52 des Gießformunterteils 51 und die formgebende Oberfläche 56 des Gießformoberteils 55 geformt wird. Dabei entsteht der Spacer 92, wie er beispielsweise in den Figuren 17 und 18 gezeigt ist. Beim Aushärten des Knochenzementteigs 36 können in den Durchbrechungen 58 Stege 90 gebildet werden, die den Spacer 92 mit dem ausgehärteten überschüssigen Knochenzementteig 38 in dem Behälter 57 verbinden. Der Spacer 92 wird entformt, indem er von dem Gießform unterteil 51 und dem Gießformoberteil 55 gelöst wird. Die Stege 90 können dabei abgeschert oder abgebrochen werden. Um ein einfaches Entformen sicherzustellen, müssen die Stege 90 einen derart geringen Durchmesser haben, dass sie manuell abgebrochen oder abgeschert werden können, wenn der Spacer 92 vom Gießformoberteil 55 getrennt wird. Hierzu hat es sich bewährt, wenn die Stege 90 einen Durchmesser von maximal 2,5 cm bevorzugt von maximal 2 cm haben. Dementsprechend sollten die Durchbrechungen 58 einen Innendurchmesser von maximal 2,5 cm, bevorzugt von maximal 2 cm haben. Um das Trennen der Stege 90 weiter zu vereinfachen, können auch kleinere Durchmesser gewählt werden. Allerdings sollten die Durchbrechungen 58 nicht einen Durchmesser von weniger als 0,2 mm haben, damit der Knochenzementteig 36 noch ohne zu großen Widerstand durch die Durchbrechungen 58 hindurch gepresst werden kann. Ansonsten kann abhängig von der Viskosität des Knochenzementteigs 36 der Widerstand beim Einschieben des Gießformoberteils 55 in das Gießformunterteil 51 zu groß werden, um noch manuell durchgeführt werden zu können oder um eine Beschädigung oder Zerstörung der Gießform zu vermeiden. Der optimale Durchmesser der Durchbrechungen 58 hängt dabei davon ab, welche Viskosität der verwendete Knochenzementteig 36 hat. Gut geeignete Durchmesser für typische Knochenzemente liegen zwischen 1 mm und 20 mm. Dabei gilt: Je höher die Viskosität des Knochenzementteigs 36 desto größer sollte der Innendurchmesser der Durchbrechungen 58 gewählt werden.

Nach dem Entformen können die vorstehenden Abstandshalter 62, möglicherweise entstandene Grate, die an der Verbindung des Gießformunterteils 51 zum Gießformoberteil 55 entstanden sind, und vorspringende Reste der Stege 90 entfernt werden, beispielsweise durch Abschneiden mit einem Messer oder Skalpell oder durch Abschleifen mit einem Schleifkopf. Als Endergebnis erhält man den Spacer 92, wie er in Figur 18 gezeigt ist.

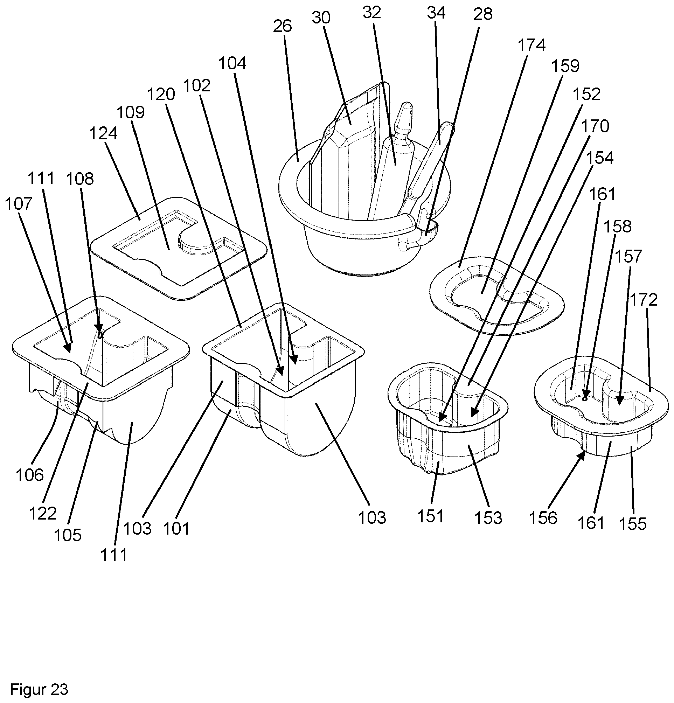

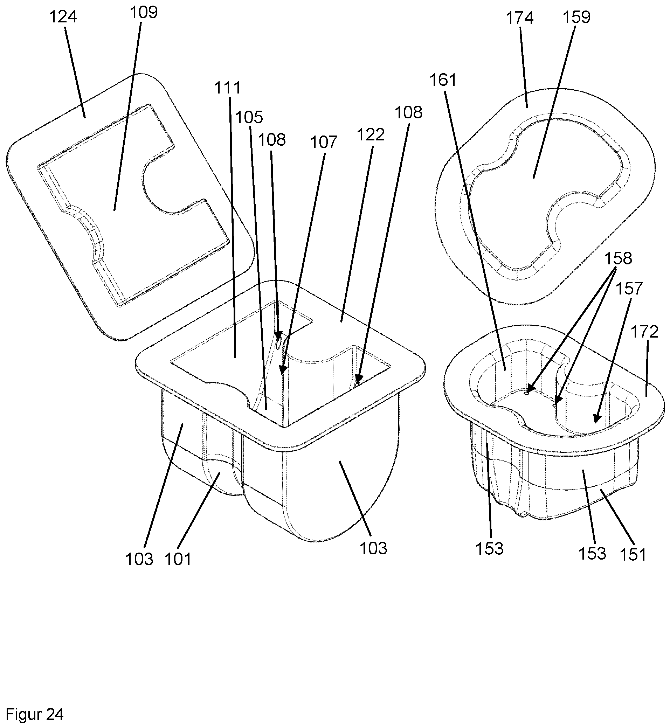

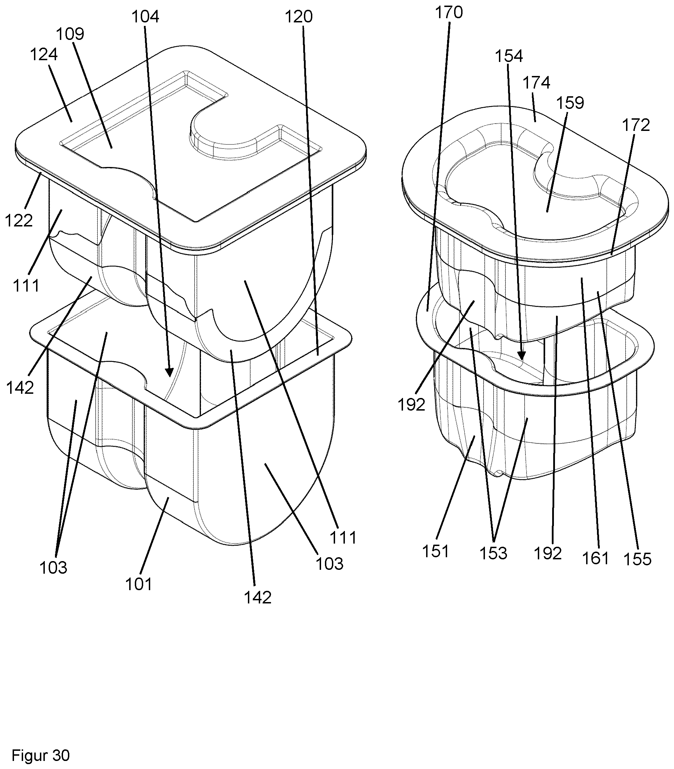

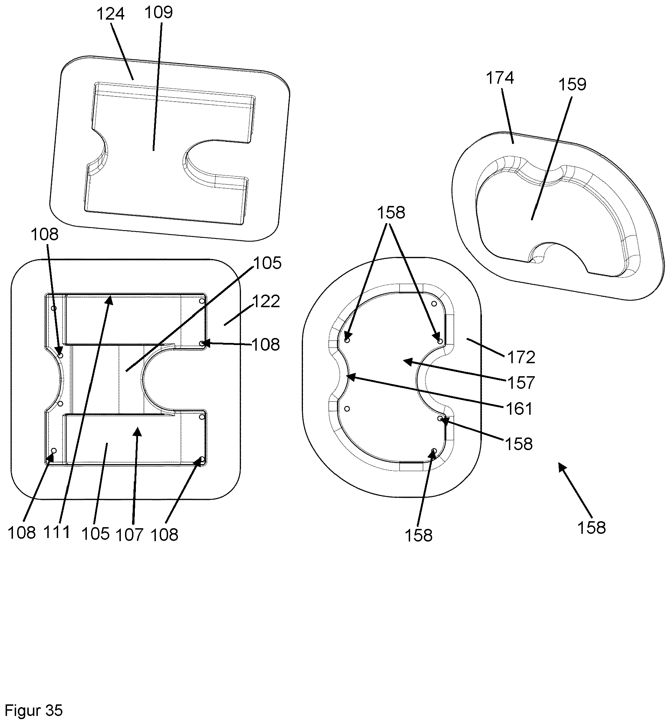

In den Figuren 23 bis 35 sind Abbildungen eines dritten Ausführungsbeispiels einer erfindungsgemäßen Vorrichtung zur Herstellung eines Spacers für ein Kniegelenk, die Teile des Spacers und Teile der Vorrichtung in verschiedenen Ansichten gezeigt. Die Figuren 23 bis 31 zeigen dabei den Ablauf eines dritten Ausführungsbeispiels eines erfindungsgemäßen Verfahrens, das mit der Vorrichtung nach dem dritten Ausführungsbeispiel durchgeführt wird.

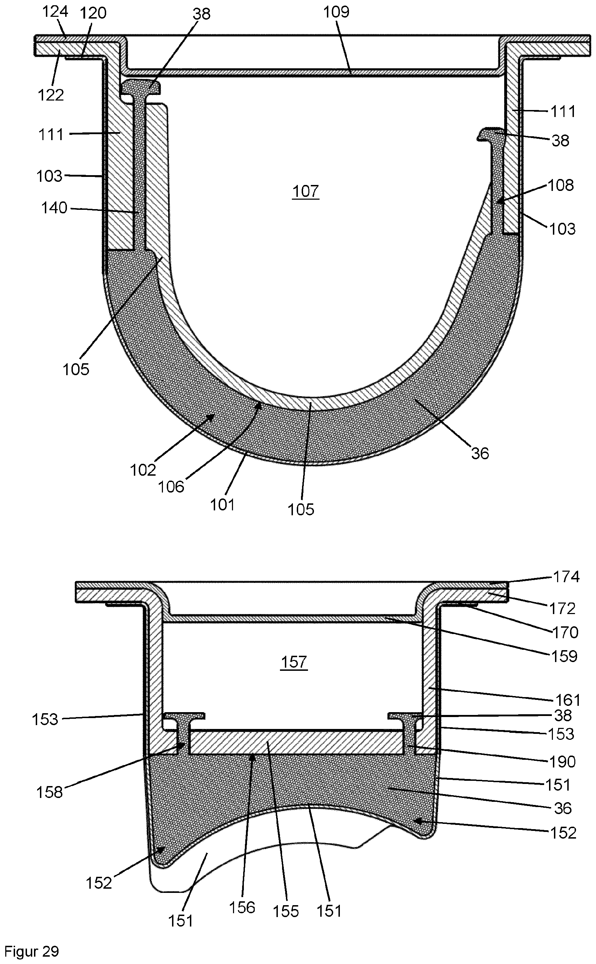

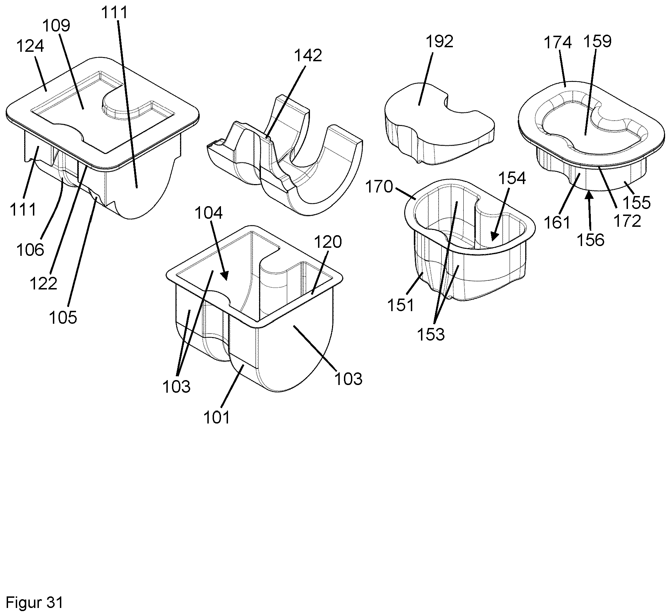

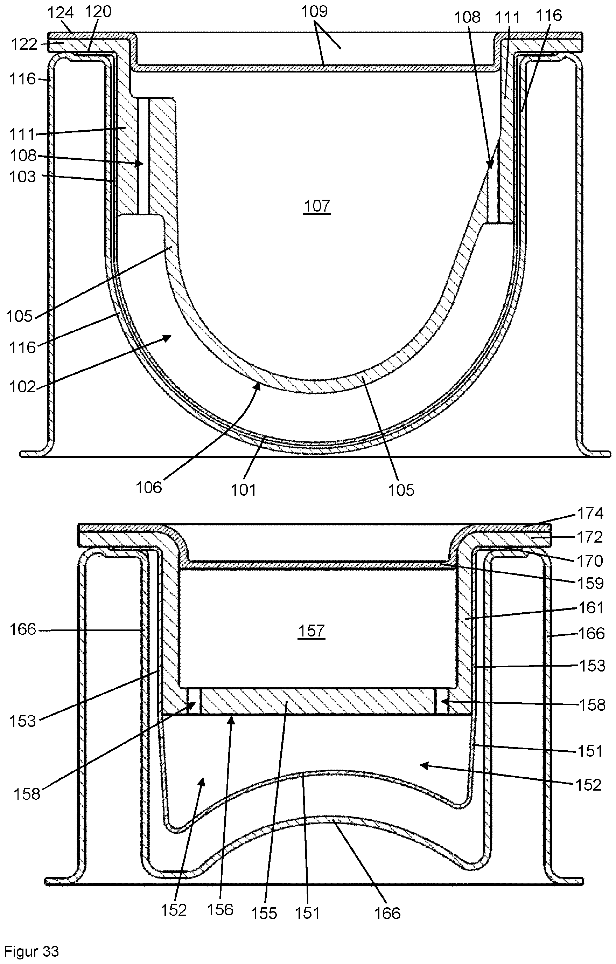

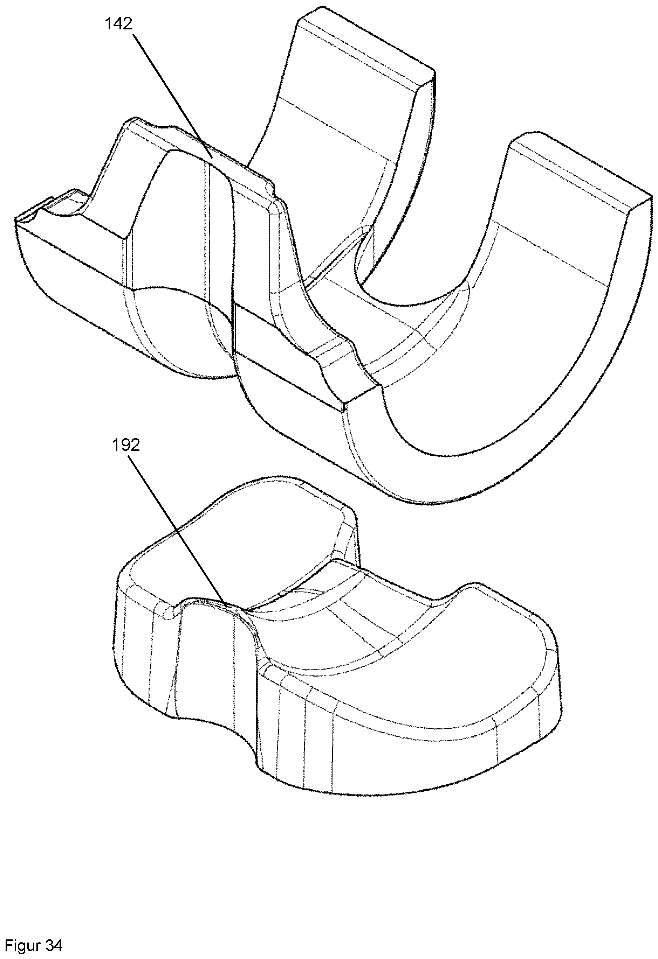

Die dritte erfindungsgemäße Vorrichtung ist zur Herstellung eines zweiteiligen artikulierenden Spacers mit einem Spacer 142 für den Femur und einem Spacer 192 für die Tibia (siehe Figuren 31 und 34) zum Ersetzen eines Kniegelenks geeignet und vorgesehen. Die Vorrichtung weist zwei mehrteilige Gießformen auf, eine Femur-Komponenten-Gießform zum Herstellen des Spacers 142 für den Femur (in den Figuren 23 bis 28 und 30 bis 32 und 35 links sowie in den Figuren 29 und 33 oben) und eine Tibia-Komponenten-Gießform zum Herstellen des Spacers 192 für die Tibia (in den Figuren 23 bis 28 und 30 bis 32 und 35 rechts sowie in den Figuren 29 und 33 unten). Das dritte Ausführungsbeispiel kann im Sinne der vorliegenden Erfindung auch als zwei unterschiedliche erfindungsgemäße Vorrichtungen aufgefasst werden, nämlich als eine vierte Vorrichtung zur Herstellung eines Spacers 142 für den Femur und eine fünfte Vorrichtung zur Herstellung eines Spacers 192 für die Tibia.

Die Femur-Komponenten-Gießform hat ein Gießformunterteil 101 und ein Gießformoberteil 105. In dem Gießformunterteil 101 ist eine Kavität 102 zur Aufnahme von Knochenzementteig 36 (siehe Figuren 23 bis 25, 29 und 33) und zum Ausformen eines Teils der Oberfläche des zu erzeugenden Spacers 142 angeordnet. Die Kavität 102 kann eine Hälfte des Spacers 142 formen. An dem Rand der Kavität 102 kann eine Gießformwand 103 angeordnet sein, die den durch die Kavität 102 gebildeten Hohlraum mit einem Innenraum 104 verlängert. Hierzu kann die Gießformwand 103 umlaufend den Rand der Kavität 102 umschließen. Die Wandungen der Gießformwand 103 können parallel zueinander ausgerichtet sein, so dass die Gießformwand 103 eine allgemein zylindrische Geometrie aufweist, wobei die Grundfläche des allgemeinen Zylinders durch die Fläche gegeben ist, die den Rand der Kavität 102 begrenzt. Innerhalb der Wandungen der Gießformwand 103 ist der Innenraum 104 zum Einfüllen von Knochenzementteig 36 angeordnet, der mit der Kavität 102 verbunden ist und vorzugsweise auch fluchtet.

Das Gießformoberteil 105 kann vorzugsweise in das Gießformunterteil 101 eingesteckt beziehungsweise eingeschoben werden. Das Gießformoberteil 105 weist an seiner Unterseite eine formgebende Oberfläche 106 auf, mit der ein weiterer Teil der Oberfläche des zu erzeugenden Spacers 142 ausformbar ist, bevorzugt der restliche Teil der der Oberfläche des zu erzeugenden Spacers 142 ausformbar ist. Die formgebende Oberfläche 106 kann zusammen mit der Kavität 102 die gesamte Oberfläche des Spacers 142 definieren oder zumindest 90% der gesamten Oberfläche des Spacers 142 definieren. Die formgebende Oberfläche 106 kann eine Vertiefung in dem Gießformoberteil 105 bilden. Es ist auch möglich, dass ein Teil der Gießformwand 103 einen Teil der Oberfläche des Spacers 142 formt oder ein oder mehrere Einsätze (nicht gezeigt) zusätzlich in die Kavität 102 eingesetzt und/oder an die formgebende Oberfläche 106 angelegt werden, die einen Teil der Oberfläche des Spacers 142 formen.

An der der formgebenden Oberfläche 106 gegenüberliegenden Seite des Gießformoberteils 105 kann ein Behälter 107 zur Aufnahme von überschüssigem Knochenzementteig 38 (siehe Figur 29) angeordnet sein. In der formgebenden Oberfläche 106 können Durchbrechungen 108 in Form von Kanälen in einer Innenwand 111 des Gießformoberteils 105 vorhanden sein, die die Seite der formgebenden Oberfläche 106 mit dem Behälter 107 für den überschüssigen Knochenzementteig 38 durchgehend verbinden. Die Durchbrechungen 108 sind vorzugsweise an den Stellen der formgebenden Oberfläche 106 angeordnet, die bei ordnungsgemäßer Aufstellung der Vorrichtung die höchsten formgebenden Punkte der Femur-Komponenten-Gießform sind. Hierdurch können in der Femur-Komponenten-Gießform eingeschlossene Gase durch die Durchbrechungen 108 entweichen und so Lufteinschlüsse in dem herzustellenden Spacer 142 vermieden werden.

Der Behälter 107 kann mit einem Deckel 109 für den Knochenzementteig 36 undurchlässig nach außen verschlossen sein oder verschlossen werden. Ausgehend von der Rückseite der formgebenden Oberfläche 106 kann sich die Innenwand 111 weg erstrecken, die den Behälter 107 begrenzt. Die Innenwand 111 kann außen passgenau mit der Gießformwand 103 fluchten. Hierdurch kann das Gießformoberteil 105 passgenau in die Gießformwand 103 des Gießformunterteils 101 eingesteckt werden und so der Knochenzementteig 36 innen von der Gießformwand 103 gesammelt und in die Kavität 102 und die formgebende Oberfläche 106 gedrückt werden. Hierzu kann es auch ausreichen, wenn sich die Gießformwand 103 in Richtung der Kavität 102 verjüngt, wobei die Verjüngung hierzu einen spitzen Winkel aufweisen muss.

Der Behälter 107 kann gasdurchlässig mit der Umgebung verbunden sein. Hierzu kann vorgesehen sein, dass der Deckel 109 nicht druckdicht schließt und/oder kleine Entlüftungsöffnungen (in den Figuren nicht zu sehen) in dem Deckel und/oder der Innenwand 111 angeordnet sind.

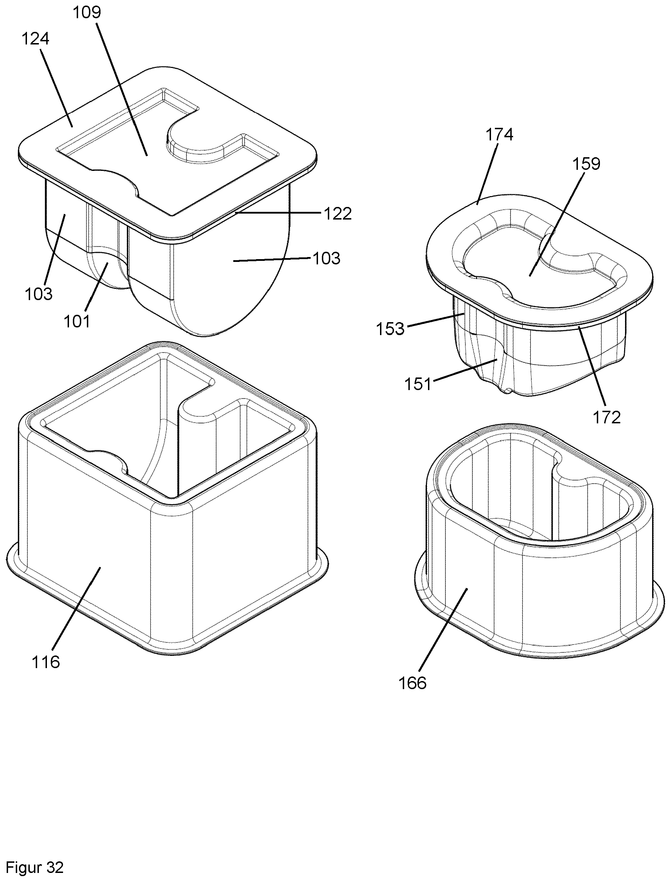

Gemäß einer bevorzugten Variante des dritten Ausführungsbeispiels kann zur korrekten Positionierung der Femur-Komponenten-Gießform mit dem Gießformunterteil 101 unten und der Gießformwand 103 senkrecht nach oben ein Standfuß 116 vorgesehen sein, in den das Gießformunterteil 101 eingelegt oder eingesteckt werden kann (siehe die Figuren 32 und 33). Der Standfuß 116 kann zur Auflage auf einer ebenen Oberfläche wie einem Tisch vorgesehen sein.

Das Gießformunterteil 101, das Gießformoberteil 105, der Deckel 109 und der Standfuß 116 können kostengünstig aus einer Kunststofffolie oder aus mehreren miteinander verbundenen Kunststofffolien hergestellt sein, insbesondere durch Spritzguss oder durch Ziehen hergestellt sein. Im zusammengesetzten Zustand stabilisieren sich dabei die aus den Kunststofffolien hergestellten Teile der Vorrichtung gegenseitig mechanisch. Die Kunststofffolie(n) bestehen vorzugsweise aus einem Polyolefin, einem Polyethylen (PET) oder einem mit Glycol modifiziertem PET (PETG). Bei der Verwendung von mehreren Folien können diese mit einem Klebstoff oder durch erhöhte Temperatur miteinander laminiert sein.

Ein Anschlag 120 kann als ein senkrecht zur Gießformwand 103 abstehender umlaufender bandförmiger Rand an der von der Kavität 102 abgewandten Seite der Gießformwand 103 angeordnet sein. Der Anschlag 120 kann begrenzen, wie weit das Gießformoberteil 105 in das Gießformunterteil 101 eingeschoben werden kann. Hierzu kann das Gießformoberteil 105 einen Gegenanschlag 122 in Form eines umlaufenden Randes aufweisen, der senkrecht von der Innenwand 111 absteht und der an der formgebenden Oberfläche 106 gegenüberliegenden Seite der Innenwand 111 angeordnet ist. Bei vollständig in das Gießform unterteil 101 eingeschobenem Gießformoberteil 105 liegt der Gegenanschlag 122 an dem Anschlag 120 an. In gleicher Weise kann an dem Deckel 109 ein Deckelrand 124 angeordnet sein, der an der dem Anschlag 120 gegenüberliegenden Seite des Gegenanschlags 122 anlegbar ist. Dadurch, dass der Anschlag 120, der Gegenanschlag 122 und der Deckelrand 124 von den angrenzenden Teilen in einem Winkel abstehen, wird die Form des Gießformunterteils 101, des Gießformoberteils 105 und des Deckels 109 im zusammengesetzten Zustand mechanisch stabilisiert. Das ist insbesondere dann hilfreich, wenn diese Teile durch Ziehen aus einer Kunststofffolie oder aus mehreren Kunststofffolien gefertigt sind.

Die Tibia-Komponenten-Gießform ist analog der Femur-Komponenten-Gießform aufgebaut und hat ein Gießformunterteil 151 und ein Gießformoberteil 155. In dem Gießform unterteil 151 ist eine Kavität 152 zur Aufnahme von Knochenzementteig 36 (siehe Figuren 23 bis 25, 29 und 33) und zum Ausformen eines Teils der Oberfläche des zu erzeugenden Spacers 192 angeordnet. Die Kavität 152 kann eine Hälfte des Spacers 192 formen. An dem Rand der Kavität 152 kann eine Gießformwand 153 angeordnet sein, die den durch die Kavität 152 gebildeten Hohlraum mit einem Innenraum 154 verlängert. Hierzu kann die Gießformwand 153 umlaufend den Rand der Kavität 152 umschließen. Die Wandungen der Gießformwand 153 können parallel zueinander ausgerichtet sein, so dass die Gießformwand 153 eine allgemein zylindrische Geometrie aufweist, wobei die Grundfläche des allgemeinen Zylinders durch die Fläche gegeben ist, die den Rand der Kavität 152 begrenzt. Innerhalb der Wandungen der Gießformwand 153 ist der Innenraum 154 zum Einfüllen von Knochenzementteig 36 angeordnet, der mit der Kavität 152 verbunden ist und vorzugsweise auch fluchtet.

Das Gießformoberteil 155 kann vorzugsweise in das Gießformunterteil 151 eingesteckt beziehungsweise eingeschoben werden. Das Gießformoberteil 155 weist an seiner Unterseite eine formgebende Oberfläche 156 auf, mit der ein weiterer Teil der Oberfläche des zu erzeugenden Spacers 192 ausformbar ist, bevorzugt der restliche Teil der der Oberfläche des zu erzeugenden Spacers 192 ausformbar ist. Die formgebende Oberfläche 156 kann zusammen mit der Kavität 152 die gesamte Oberfläche des Spacers 192 definieren oder zumindest 90% der gesamten Oberfläche des Spacers 192 definieren. Die formgebende Oberfläche 156 kann eine Vertiefung in dem Gießformoberteil 155 bilden. Es ist auch möglich, dass ein Teil der Gießformwand 153 einen Teil der Oberfläche des Spacers 192 formt oder ein oder mehrere Einsätze (nicht gezeigt) zusätzlich in die Kavität 152 eingesetzt und/oder an die formgebende Oberfläche 156 angelegt werden, die einen Teil der Oberfläche des Spacers 192 formen.

An der der formgebenden Oberfläche 156 gegenüberliegenden Seite des Gießformoberteils 155 kann ein Behälter 157 zur Aufnahme von überschüssigem Knochenzementteig 38 (siehe Figur 29) angeordnet sein. In der formgebenden Oberfläche 156 können Durchbrechungen 158 vorhanden sein, die die Seite der formgebenden Oberfläche 156 mit dem Behälter 157 für den überschüssigen Knochenzementteig 38 durchgehend verbinden. Die Durchbrechungen 158 sind vorzugsweise an den Stellen der formgebenden Oberfläche 156 angeordnet, die bei ordnungsgemäßer Aufstellung der Vorrichtung die höchsten formgebenden Punkte der Tibia-Komponenten-Gießform sind. Hierdurch können in der Tibia-Komponenten-Gießform eingeschlossene Gase durch die Durchbrechungen 158 entweichen und so Lufteinschlüsse in dem herzustellenden Spacer 192 vermieden werden.

Der Behälter 157 kann mit einem Deckel 159 für den Knochenzementteig 36 undurchlässig nach außen verschlossen sein oder verschlossen werden. Ausgehend von der Rückseite der formgebenden Oberfläche 156 kann sich eine Innenwand 161 weg erstrecken, die den Behälter 157 begrenzt. Die Innenwand 161 kann außen passgenau mit der Gießformwand 153 fluchten. Hierdurch kann das Gießformoberteil 155 passgenau in die Gießformwand 153 des Gießformunterteils 151 eingesteckt werden und so der Knochenzementteig 36 innen von der Gießformwand 153 gesammelt und in die Kavität 152 und die formgebende Oberfläche 156 gedrückt werden. Hierzu kann es auch ausreichen, wenn sich die Gießformwand 153 in Richtung der Kavität 152 verjüngt, wobei die Verjüngung hierzu einen spitzen Winkel aufweisen muss.

Der Behälter 157 kann gasdurchlässig mit der Umgebung verbunden sein. Hierzu kann vorgesehen sein, dass der Deckel 159 nicht druckdicht schließt und/oder kleine Entlüftungsöffnungen (in den Figuren nicht zu sehen) in dem Deckel und/oder der Innenwand 161 angeordnet sind.

Gemäß einer bevorzugten Variante des dritten Ausführungsbeispiels kann zur korrekten Positionierung der Tibia-Komponenten-Gießform mit dem Gießformunterteil 151 unten und der Gießformwand 153 senkrecht nach oben ein Standfuß 166 vorgesehen sein, in den das Gießformunterteil 151 eingelegt oder eingesteckt werden kann (siehe die Figuren 32 und 33). Der Standfuß 166 kann zur Auflage auf einer ebenen Oberfläche wie einem Tisch vorgesehen sein.

Das Gießformunterteil 151, das Gießformoberteil 155, der Deckel 159 und der Standfuß 166 können kostengünstig aus einer Kunststofffolie oder aus mehreren miteinander verbundenen Kunststofffolien hergestellt sein, insbesondere durch Spritzguss oder durch Ziehen hergestellt sein. Im zusammengesetzten Zustand stabilisieren sich dabei die aus den Kunststofffolien hergestellten Teile der Vorrichtung gegenseitig mechanisch. Die Kunststofffolie(n) bestehen vorzugsweise aus einem Polyolefin, einem Polyethylen (PET) oder einem mit Glycol modifiziertem PET (PETG). Bei der Verwendung von mehreren Folien können diese mit einem Klebstoff oder durch erhöhte Temperatur miteinander laminiert sein.

Ein Anschlag 170 kann als ein senkrecht zur Gießformwand 153 abstehender umlaufender bandförmiger Rand an der von der Kavität 152 abgewandten Seite der Gießformwand 153 angeordnet sein. Der Anschlag 170 kann begrenzen, wie weit das Gießformoberteil 155 in das Gießformunterteil 151 eingeschoben werden kann. Hierzu kann das Gießformoberteil 155 einen Gegenanschlag 172 in Form eines umlaufenden Randes aufweisen, der senkrecht von der Innenwand 161 absteht und der an der formgebenden Oberfläche 156 gegenüberliegenden Seite der Innenwand 161 angeordnet ist. Bei vollständig in das Gießformunterteil 151 eingeschobenem Gießformoberteil 155 liegt der Gegenanschlag 172 an dem Anschlag 170 an. In gleicher Weise kann an dem Deckel 159 ein Deckelrand 174 angeordnet sein, der an der dem Anschlag 170 gegenüberliegenden Seite des Gegenanschlags 172 anlegbar ist. Dadurch, dass der Anschlag 170, der Gegenanschlag 172 und der Deckelrand 174 von den angrenzenden Teilen in einem Winkel abstehen, wird die Form des Gießformunterteils 151, des Gießformoberteils 155 und des Deckels 159 im zusammengesetzten Zustand mechanisch stabilisiert. Das ist insbesondere dann hilfreich, wenn diese Teile durch Ziehen aus einer Kunststofffolie oder aus mehreren Kunststofffolien gefertigt sind.

Die Vorrichtung kann des Weiteren einen Mischbecher 26 mit einer Tülle 28 zum Ausgießen von Knochenzementteig 36 aus dem Mischbescher 26 (siehe Figur 23) sowie einen Folienbeutel 30 enthaltend Zementpulver, eine Ampulle 32 enthaltend Monomerflüssigkeit und einen Spatel 34 zum Mischen des Zementpulvers mit der Monomerflüssigkeit in dem Mischbecher 26 aufweisen. Der Knochenzementteig 36 kann dann in dem Mischbecher 26 gemischt werden, bevor er in die Gießform beziehungsweise in das Gießformunterteil 1 gefüllt wird. Der Knochenzementteig 36 kann aber auch auf andere Weise hergestellt werden, bevor er in die Gießform gefüllt wird. Die Vorrichtung benötigt also nicht zwingend den Mischbecher 26 und auch nicht den Knochenzementteig 36 oder dessen Ausgangskomponenten. Die Vorrichtung kann grundsätzlich mit irgendeinem anderen bekannten System zum Herstellen eines Knochenzementteigs angewendet und verwendet werden, wie beispielsweise mit einem geeigneten Kartuschensystem zum Lagern und Mischen von Knochenzementteig.

Im Folgenden wird der Ablauf eines dritten Ausführungsbeispiels eines erfindungsgemäßen Verfahrens anhand der Figuren 23 bis 35 beschrieben.

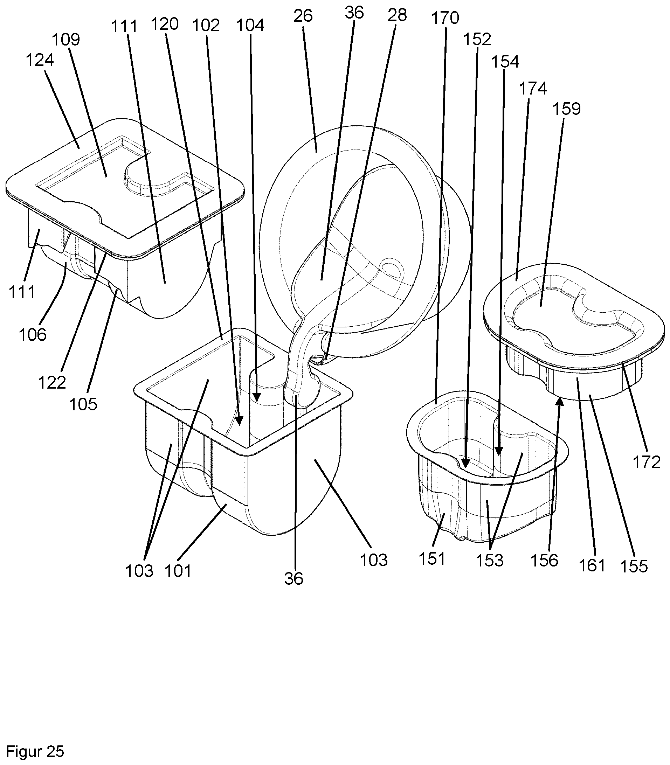

Die Vorrichtung kann zunächst steril aus einer Verpackung (nicht gezeigt) entnommen werden und kann dann so wie in Figur 23 gezeigt oder wie in Figur 25 gezeigt vorliegen. Die Gießformunterteile 101, 105 können optional in die Standfüße 116, 166 eingesetzt werden, wenn Standfüße 116, 166 vorhanden sind.

Der Knochenzementteig 36 kann durch Mischen des Zementpulvers aus der Folienbeutel 30 und der Monomerflüssigkeit aus der Ampulle 32 in dem Mischbecher 26 mit Hilfe des Spatels 34 gemischt werden. Alternativ kann der Knochenzementteig 36 auch auf irgendeine andere Weise hergestellt werden.

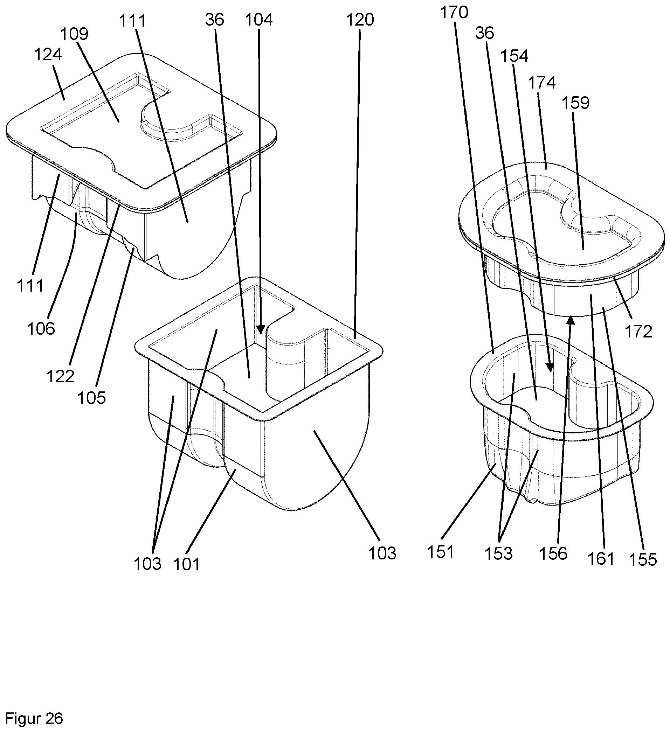

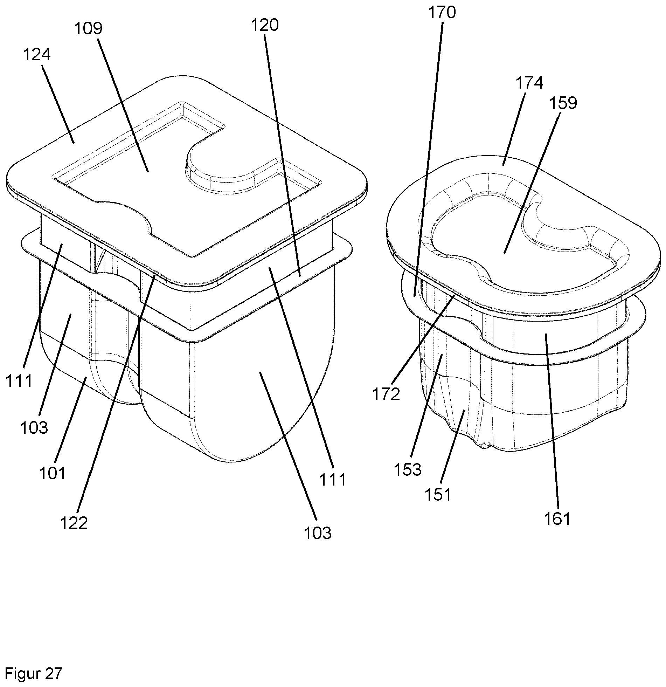

Anschließend kann der Knochenzementteig 36 jeweils im Überschuss in den Innenraum 104 und die Kavität 102 sowie in den Innenraum 154 und die Kavität 152 eingefüllt werden (siehe Figur 25). Anschließend können das Gießformoberteil 105 in das Gießformunterteil 101 sowie das Gießformoberteil 155 in das Gießformunterteil 151 eingesetzt und eingeschoben werden. Dazu können der Behälter 107 mit dem Deckel 109 und der Behälter 157 mit dem Deckel 159 geschlossen sein (siehe Figuren 26 und 27).

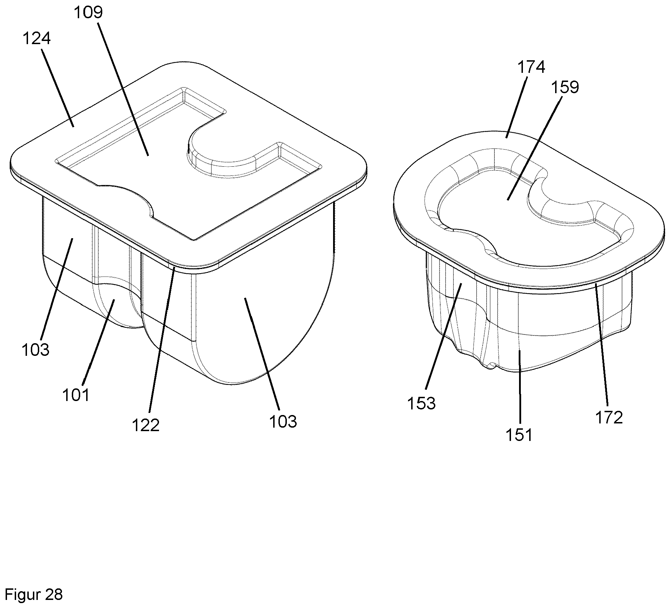

Das Gießformoberteil 105 kann in den Innenraum 104 des Gießformunterteils 101 eingeschoben werden, bis der Gegenanschlag 122 an dem Anschlag 120 anliegt, und das Gießformoberteil 155 kann in den Innenraum 154 des Gießformunterteils 151 eingeschoben werden, bis der Gegenanschlag 172 an dem Anschlag 170 anliegt (siehe hierzu die Figuren 28 und 29). Beim Einschieben der Gießformoberteile 105, 155 kann der enthaltene Knochenzementteig 36 in die Kavitäten 102, 152 gepresst werden und es verringert sich fortwährend das Volumen innerhalb der Femur-Komponenten-Gießform und der Tibia-Komponenten-Gießform. Überschüssiger Knochenzementteig 38 wird durch die Durchbrechungen 108, 158 hindurch in die Behälter 107, 157 gedrückt (siehe Figur 29). Mit Hilfe der Deckel 109, 159 kann der überschüssige Knochenzementteig 38 in den Behältern 107, 157 eingeschlossen werden.

Anschließend kann der Knochenzementteig 36 in der Femur-Komponenten-Gießform und der Tibia-Komponenten-Gießform aushärten, wobei seine Oberfläche durch die Oberfläche der Kavität 102 des Gießformunterteils 101 und die formgebende Oberfläche 106 des Gießformoberteils 105 beziehungsweise durch die Oberfläche der Kavität 152 des Gießformunterteils 151 und die formgebende Oberfläche 156 des Gießformoberteils 155 geformt wird. Dabei entstehen der Spacer 142 für den Femur und der Spacer 192 für die Tibia, wie sie beispielsweise in den Figuren 31 und 34 gezeigt sind. Beim Aushärten des Knochenzementteigs 36 können in den Durchbrechungen 108 Stege 140 gebildet werden, die den Spacer 142 für den Femur mit dem ausgehärteten überschüssigen Knochenzementteig 38 in dem Behälter 107 verbinden. Ebenso können beim Aushärten des Knochenzementteigs 36 in den Durchbrechungen 158 Stege 190 gebildet werden, die den Spacer 192 für die Tibia mit dem ausgehärteten überschüssigen Knochenzementteig 38 in dem Behälter 157 verbinden. Die Spacer 142, 192 werden entformt, indem sie von den Gießformunterteilen 101, 151 und den Gießformoberteilen 105, 155 gelöst werden. Die Stege 140, 190 können dabei abgeschert oder abgebrochen werden. Um ein einfaches Entformen sicherzustellen, müssen die Stege 140, 190 einen derart geringen Durchmesser haben, dass sie manuell abgebrochen oder abgeschert werden können, wenn die Spacer 142, 192 von den Gießformoberteilen 105, 155 getrennt werden. Hierzu hat es sich bewährt, wenn die Stege 140, 190 einen Durchmesser von maximal 2,5 cm bevorzugt von maximal 2 cm haben. Dementsprechend sollten die Durchbrechungen 8 einen Innendurchmesser von maximal 2,5 cm, bevorzugt von maximal 2 cm haben. Um das Trennen der Stege 140, 190 weiter zu vereinfachen, können auch kleinere Durchmesser gewählt werden. Allerdings sollten die Durchbrechungen 108, 158 nicht einen Durchmesser von weniger als 0,2 mm haben, damit der Knochenzementteig 36 noch ohne zu großen Widerstand durch die Durchbrechungen 108, 158 hindurch gepresst werden kann. Ansonsten kann abhängig von der Viskosität des Knochenzementteigs 36 der Widerstand beim Einschieben des Gießformoberteils 105 in das Gießformunterteil 101 beziehungsweise beim Einschieben des Gießformoberteils 155 in das Gießformunterteil 151 zu groß werden, um noch manuell durchgeführt werden zu können oder um eine Beschädigung oder Zerstörung der Femur-Komponenten-Gießform und der Tibia-Komponenten-Gießform zu vermeiden. Der optimale Durchmesser der Durchbrechungen 108, 158 hängt dabei davon ab, welche Viskosität der verwendete Knochenzementteig 36 hat. Gut geeignete Durchmesser für typische Knochenzemente liegen zwischen 1 mm und 20 mm. Dabei gilt: Je höher die Viskosität des Knochenzementteigs 36 desto größer sollte der Innendurchmesser der Durchbrechungen 108, 158 gewählt werden.

Nach dem Entformen können möglicherweise entstandene Grate, die an den Verbindungen der Gießformunterteile 101, 151 zu den Gießformoberteilen 105, 155 entstanden sind, und vorspringende Reste der Stege 140, 190 entfernt werden, beispielsweise durch Abschneiden mit einem Messer oder Skalpell oder durch Abschleifen mit einem Schleifkopf. Als Endergebnis erhält man den Spacer 142, 192, wie er in Figur 34 gezeigt ist.

### Bezugszeichenliste

- 1, 51, 101, 151: Gießformunterteil
- 2, 52, 102, 152: Kavität
- 3, 53, 103, 153: Gießformwand
- 4, 54, 104, 154: Innenraum
- 5, 55, 105, 155: Gießformoberteil
- 6, 56, 106, 156: Formgebende Oberfläche
- 7, 57, 107, 157: Behälter
- 8, 58, 108, 158: Durchbrechung
- 9, 59, 109, 159: Deckel
- 10, 60: Metallkern
- 11, 61, 111, 161: Innenwand
- 12, 62: Abstandhalter
- 14: Bohrung
- 16, 66, 116, 166: Standfuß
- 18,68: Nut
- 19,69: Steg
- 20, 70, 120, 170: Anschlag / Anlagefläche
- 21, 71: Nut
- 22, 72, 122, 172: Gegenanschlag / Anlagefläche
- 23, 73: Ausnehmung
- 24, 74, 124, 174: Deckelrand
- 26: Mischbecher
- 28: Tülle
- 30: Folienbeutel enthaltend Knochenzementpulver
- 32: Ampulle enthaltend Monomerflüssigkeit
- 34: Spatel
- 36: Knochenzementteig
- 38: Überschüssiger Knochenzementteig
- 40, 90, 140, 190: Steg
- 42, 92, 142, 192: Spacer
- 44, 94: Spacerkopf
- 46, 96: Schaft

## Patentansprüche

1. Vorrichtung zum Herstellen eines Spacers (42, 92, 142, 192) durch Aushärten von Knochenzementteig (36) in einer Gießform, wobei der Spacer (42, 92, 142, 192) dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk, ein Kniegelenk oder ein Schultergelenk temporär zu ersetzen, die Vorrichtung aufweisend
ein Gießformunterteil (1, 51, 101, 151), wobei das Gießformunterteil (1, 51, 101, 151) eine Kavität (2, 52, 102, 152) zur Aufnahme eines Knochenzementteigs (36) und zum Formen eines ersten Oberflächenbereichs des Spacers (42, 92, 142, 192) aus dem Knochenzementteig (36) aufweist;
eine Gießformwand (3, 53, 103, 153), die sich von einem umlaufenden Rand der Kavität (2, 52, 102, 152) umlaufend von der Kavität (2, 52, 102, 152) des Gießformunterteils (1, 51, 101, 151) weg erstreckt und die auf der der Kavität (2, 52, 102, 152) gegenüberliegenden Seite offen ist, so dass die Kavität (2, 52, 102, 152) durch einen von der Gießformwand (3, 53, 103, 153) begrenzten Innenraum (4, 54, 104, 154) zugänglich ist;
ein Gießformoberteil (5, 55, 105, 155), wobei das Gießformoberteil (5, 55, 105, 155) eine formgebende Oberfläche (6, 56, 106, 156) zum Formen eines zweiten Oberflächenbereichs des Spacers (42, 92, 142, 192) aus dem Knochenzementteig (36) aufweist, wobei das Gießformoberteil (5, 55, 105, 155) durch die offene, auf der der Kavität (2, 52, 102, 152) gegenüberliegenden Seite der Gießformwand (3, 53, 103, 153) in den Innenraum (4, 54, 104, 154) einsteckbar und in Richtung der Kavität (2, 52, 102, 152) verschiebbar ist, so dass sich ein von der Kavität (2, 52, 102, 152) des Gießformunterteils (1, 51, 101, 151), der formgebenden Oberfläche (6, 56, 106, 156) des Gießformoberteils (5, 55, 105, 155) und der Gießformwand (3, 53, 103, 153) begrenzter Hohlraum bildet, in dem der Spacer (42, 92, 142, 192) formbar ist;
mindestens einen Behälter (7, 57, 107, 157) zur Aufnahme von überschüssigem Knochenzementteig (38); und
mindestens eine Durchbrechung (8, 58, 108, 158) in der formgebenden Oberfläche (6, 56, 106, 156) des Gießformoberteils (5, 55, 105, 155) und/oder in der Kavität (2, 52, 102, 152) des Gießformunterteils (1, 51, 101, 151), wobei die mindestens eine Durchbrechung (8, 58, 108, 158) in den mindestens einen Behälter (7, 57, 107, 157) zur Aufnahme von überschüssigem Knochenzementteig (38) mündet, wobei
die Vorrichtung einen oder zwei Deckel (9, 59, 109, 159) aufweist,
mit dem oder mit einem von denen das Gießformoberteil (5, 55, 105, 155) auf der der formgebenden Oberfläche (6, 56, 106, 156) des Gießformoberteils (5, 55, 105, 155) gegenüberliegenden Seite nach außen verschlossen oder verschließbar ist, so dass sich zwischen dem Gießformoberteil (5, 55, 105, 155) und dem Deckel (9, 59, 109, 159) ein nach außen geschlossener oberer Behälter als einer der mindestens einen Behälter (7, 57, 107, 157) zur Aufnahme von Knochenzementteig (36) bildet, und/oder mit dem oder mit einem von denen das Gießformunterteil (1, 51, 101, 151) auf der der Kavität (2, 52, 102, 152) des Gießformunterteils (1, 51, 101, 151) gegenüberliegenden Seite nach außen verschlossen oder verschließbar ist, so dass sich zwischen dem Gießformunterteil (1, 51, 101, 151) und dem Deckel (9, 59, 109, 159) ein nach außen geschlossener unterer Behälter als einer der mindestens einen Behälter (7, 57, 107, 157) zur Aufnahme von Knochenzementteig (36) bildet, wobei
der oder einer der Deckel (9, 59, 109, 159) in oder auf das Gießformoberteil (5, 55, 105, 155) oder in oder auf eine Innenwand (11, 61, 111, 161) des Gießformoberteils (5, 55, 105, 155) ein- oder aufgesetzt oder ein- oder aufsetzbar ist, um die der formgebenden Oberfläche (6, 56, 106, 156) des Gießformoberteils (5, 55, 105, 155) gegenüberliegenden Seite nach außen zu verschließen und dort einen geschlossenen Behälter (7, 57, 107, 157) zur Aufnahme von überschüssigem Knochenzementteig (38) zu bilden, und/oder
der oder einer der Deckel (9, 59, 109, 159) in oder auf das Gießformunterteil (1, 51, 101, 151) ein- oder aufgesetzt oder ein- oder aufsetzbar ist, um die der Kavität (2, 52, 102, 152) des Gießformunterteils (1, 51, 101, 151) gegenüberliegenden Seite nach außen zu verschließen und dort einen geschlossenen Behälter (7, 57, 107, 157) zur Aufnahme von überschüssigem Knochenzementteig (38) bildet.

2. Vorrichtung nach Anspruch 1 **dadurch gekennzeichnet, dass**
einander gegenüberliegende Teile der Gießformwand (3, 53, 103, 153) parallel zueinander ausgerichtet sind oder die Gießformwand (3, 53, 103, 153) in Richtung der Kavität (2, 52, 102, 152) leicht konisch zusammenläuft oder die Gießformwand (3, 53, 103, 153) die Form eines geraden oder schiefen allgemeinen Zylinders aufweist, dessen Grundfläche von dem umlaufenden Rand der Kavität (2, 52, 102, 152) begrenzt ist.

3. Vorrichtung nach Anspruch 1 oder 2, **dadurch gekennzeichnet, dass**
sich eine Innenwand (11, 61, 111, 161) von einem umlaufenden Rand der formgebenden Oberfläche (6, 56, 106, 156) des Gießformoberteils (5, 55, 105, 155) umlaufend von der Kavität (2, 52, 102, 152) des Gießformunterteils (1, 51, 101, 151) weg erstreckt, wobei vorzugsweise die Innenwand (11, 61, 111, 161) den mindestens einen Behälter (7, 57, 107, 157) zumindest bereichsweise begrenzt und/oder einander gegenüberliegende Teile der Innenwand (11, 61, 111, 161) parallel zueinander ausgerichtet sind oder die Innenwand (11, 61, 111, 161) die Form eines geraden oder schiefen allgemeinen Zylinders aufweist, dessen Grundfläche von dem umlaufenden Rand der formgebenden Oberfläche (6, 56, 106, 156) des Gießformoberteils (5, 55, 105, 155) begrenzt ist.

4. Vorrichtung nach Anspruch 3, **dadurch gekennzeichnet, dass**
die Innenwand (11, 61, 111, 161) und die Gießformwand (3, 53, 103, 153) flächenbündig aneinander anliegen, wenn das Gießformoberteil (5, 55, 105, 155) in die Gießformwand (3, 53, 103, 153) eingeschoben ist und/oder
die Innenwand (11, 61, 111, 161) gegen die Gießformwand (3, 53, 103, 153) für den Knochenzementteig (36) abdichtet, wenn das Gießformoberteil (5, 55, 105, 155) in die Gießformwand (3, 53, 103, 153) eingeschoben ist.

5. Vorrichtung nach einem der vorangehenden Ansprüche, **dadurch gekennzeichnet, dass**
der oder die Deckel (9, 59, 109, 159) zumindest eine Entlüftungsöffnung aufweist oder aufweisen und/oder der oder die Deckel (9, 59, 109, 159) gasdurchlässig mit dem Gießformoberteil (5, 55, 105, 155) und/oder dem Gießformunterteil (1, 51, 101, 151) verschlossen oder verschließbar sind.

6. Vorrichtung nach einem der vorangehenden Ansprüche, **dadurch gekennzeichnet, dass**
das Volumen der Kavität (2, 52, 102, 152) des Gießformunterteils (1, 51, 101, 151) und das von der Gießformwand (3, 53, 103, 153) begrenzte Volumen zusammen größer sind als das Volumen des zu erzeugenden Spacers (42, 92, 142, 192), und/oder
die Gießformwand (3, 53, 103, 153) und das Gießformunterteil (1, 51, 101, 151) einteilig ausgeführt sind, wobei vorzugsweise die Gießformwand (3, 53, 103, 153) ein Teil des Gießformunterteils (1, 51, 101, 151) ist.

7. Vorrichtung nach einem der vorangehenden Ansprüche, **dadurch gekennzeichnet, dass**
die Vorrichtung ein Mischsystem zum Mischen von Knochenzementteig (36), ein Zementpulver und eine Monomerflüssigkeit aufweist, wobei das Zementpulver und die Monomerflüssigkeit getrennt voneinander gelagert sind, wobei der Knochenzementteig (36) mit Hilfe des Mischsystems aus dem Zementpulver und der Monomerflüssigkeit mischbar ist, wobei bevorzugt
das Mischsystem einen Mischbecher (26) aufweist, der besonders bevorzugt eine Tülle (28) zum Einfüllen des Knochenzementteigs (36) aus dem Mischbecher (26) in die Kavität (2, 52, 102, 152) und den von der Gießformwand (3, 53, 103, 153) begrenzten Innenraum (4, 54, 104, 154) aufweist, oder
das Mischsystem eine Knochenzementkartusche zum Lagern und Mischen des Zementpulvers und der Monomerflüssigkeit und zum Austragen von gemischtem Knochenzementteig (36) aus der Knochenzementkartusche heraus ist, wobei besonders bevorzugt die Knochenzementkartusche die Knochenzement-Ausgangskomponenten zur Herstellung des Knochenzements in flüssigkeitsdicht voneinander getrennten Bereichen beinhaltet.

8. Vorrichtung nach einem der vorangehenden Ansprüche, **dadurch gekennzeichnet, dass**
das Gießformunterteil (1, 51, 101, 151) und das Gießformoberteil (5, 55, 105, 155), und bevorzugt auch die Gießformwand (3, 53, 103, 153) und gegebenenfalls der oder die Deckel (9, 59, 109, 159), aus einer Kunststofffolie bestehen oder im Wesentlichen aus einer Kunststofffolie bestehen oder das Gießformunterteil (1, 51, 101, 151) und das Gießformoberteil (5, 55, 105, 155), und bevorzugt auch die Gießformwand (3, 53, 103, 153) und gegebenenfalls der oder die Deckel (9, 59, 109, 159), jeweils aus zwei oder mehreren Kunststofffolien aufgebaut sind, die miteinander verbunden sind, besonders bevorzugt miteinander verschweißt oder verklebt sind, und/oder
das Gießformunterteil (1, 51, 101, 151), das Gießformoberteil (5, 55, 105, 155) und die Gießformwand (3, 53, 103, 153) und gegebenenfalls der oder die Deckel (9, 59, 109, 159) im Wesentlichen oder vollständig aus einem Kunststoff bestehen, bevorzugt aus einem Polyolefin, Polyethylen (PE) oder Polypropylen (PP), besonders bevorzugt aus einer PETG-Folie und/oder einer Polyamid-Folie und/oder einer PE-Folie gefertigt sind.

9. Vorrichtung nach einem der vorangehenden Ansprüche, **dadurch gekennzeichnet, dass**
die mindestens eine Durchbrechung (8, 58, 108, 158) eine minimale Querschnittslänge von maximal 2,5 mm hat, bevorzugt eine minimal Querschnittslänge von maximal 2 mm hat, besonders bevorzugt eine minimal Querschnittslänge von maximal 1,5 mm hat, ganz besonders bevorzugt eine minimal Querschnittslänge von maximal 1 mm hat, und/oder
die mindestens eine Durchbrechung (8, 58, 108, 158) eine minimale Querschnittslänge von mindestens 0,2 mm hat, bevorzugt von mindestens 0,5 mm hat, besonders bevorzugt von mindestens 1 mm hat.

10. Vorrichtung nach einem der vorangehenden Ansprüche, **dadurch gekennzeichnet, dass**
an dem der Kavität (2, 52, 102, 152) des Gießformunterteils (1, 51, 101, 151) gegenüberliegenden Ende der Gießformwand (3, 53, 103, 153) ein Anschlag (20, 70, 120, 170) zur Begrenzung der Bewegung des Gießformoberteils (5, 55, 105, 155) innerhalb der Gießformwand (3, 53, 103, 153) in Richtung der Kavität (2, 52, 102, 152) angeordnet ist, wobei bevorzugt eine Anlagefläche als Anschlag (20, 70, 120, 170) an dem der Kavität (2, 52, 102, 152) des Gießformunterteils (1, 51, 101, 151) gegenüberliegenden Ende der Gießformwand (3, 53, 103, 153) angeordnet ist, die besonders bevorzugt im rechten Winkel von der Gießformwand (3, 53, 103, 153) absteht, und/oder
die Vorrichtung einen Metallkern (10, 60) aufweist, der in der Kavität (2, 52) anzuordnen oder angeordnet ist, wobei bevorzugt die Vorrichtung mehrere Abstandhalter (12, 62) aufweist, die den Metallkern (10, 60) in der Kavität (2, 52) von der Innenseite der Kavität (2, 52) und von der Innenseite der Gießformwand (3, 53) beabstandet halten, wobei besonders bevorzugt die Abstandhalter (12, 62) aus ausgehärtetem Knochenzement, insbesondere aus Polymethylmethacrylat bestehen.

11. Vorrichtung nach einem der vorangehenden Ansprüche, **dadurch gekennzeichnet, dass**
das Gießformunterteil (1, 51, 101, 151) oder das Gießformunterteil (1, 51, 101, 151) und die Gießformwand (3, 53, 103, 153) oder das Gießformunterteil (1, 51, 101, 151), die Gießformwand (3, 53, 103, 153) und die Gießformoberteil (5, 55, 105, 155) transparent oder transluzent ist oder sind, und/oder
die mindestens eine Durchbrechung (8, 58, 108, 158) an den Bereichen angeordnet ist, die bei normaler Aufstellung der Gießform die höchsten Punkte des Hohlraums sind.

12. Vorrichtung nach einem der vorangehenden Ansprüche, **dadurch gekennzeichnet, dass**
die formgebende Oberfläche (6, 56, 106, 156) des Gießformoberteils (5, 55, 105, 155) mit einem äußeren Rand innen an der Gießformwand (3, 53, 103, 153) anliegt und der Rand beim Einschieben des Gießformoberteils (5, 55, 105, 155) innen an der Gießformwand (3, 53, 103, 153) gleitet, wobei vorzugsweise der Rand eine Abstreifkante oder eine Abstreiflippe umfasst, und/oder
die Vorrichtung einen Standfuß (16, 66, 116, 166) aufweist, wobei das Gießformunterteil (1, 51, 101, 151) in den Standfuß (16, 66, 116, 166) einsetzbar ist und der Standfuß (16, 66, 116, 166) dazu geeignet ist, mit dem Gießformunterteil (1, 51, 101, 151) darin auf eine ebene Unterlage gestellt zu werden, wobei bevorzugt der Standfuß (16, 66, 116, 166) im Wesentlichen oder vollständig aus einer Kunststofffolie oder aus zwei oder mehreren miteinander verbundenen Kunststofffolien besteht, besonders bevorzugt miteinander verschweißt oder verklebt sind.

13. Verfahren zur Herstellung eines Spacers (42, 92, 142, 192) zum temporären Ersetzen eines Gelenks oder eines Teils eines Gelenks, insbesondere eines Hüftgelenks, eines Kniegelenks oder eines Schultergelenks, umfassend eine artikulierende Oberfläche des Gelenks, wobei das Verfahren mit einer Vorrichtung nach einem der Ansprüche 1 bis 12 durchgeführt wird, das Verfahren aufweisend die folgenden chronologischen Schritte:
A) Einfüllen von fließfähigem Knochenzementteig (36) in die Kavität (2, 52, 102, 152) und den von der Gießformwand (3, 53, 103, 153) begrenzten Innenraum (4, 54, 104, 154), wobei der fließfähige Knochenzementteig (36) mit einem größeren Volumen eingefüllt wird, als es der Spacer (42, 92, 142, 192) erfordert;
B) Einsetzen des Gießformoberteils (5, 55, 105, 155) in die Gießformwand (3, 53, 103, 153), wobei die formgebende Oberfläche (6, 56, 106, 156) in Richtung der Kavität (2, 52, 102, 152) weist;
C) Eindrücken des Gießformoberteils (5, 55, 105, 155) in Richtung der Kavität (2, 52, 102, 152) innerhalb der Gießformwand (3, 53, 103, 153);
D) Austreten von überschüssigem Knochenzementteig (38) durch die mindestens eine Durchbrechung (8, 58, 108, 158) unter fortwährendem Eindrücken des Gießformoberteils (5, 55, 105, 155), wobei der überschüssige Knochenzementteig (38) in einen durch einen Deckel (9, 59, 109, 159) verschlossenen unteren Behälter (7, 57, 107, 157) zur Aufnahme des überschüssigen Knochenzementteigs (38) fließt und dort eingeschlossen wird und/oder in einen durch einen Deckel (9, 59, 109, 159) verschlossenen oberen Behälter (7, 57, 107, 157) zur Aufnahme des überschüssigen Knochenzementteigs (38) an dem Gießformoberteil (5, 55, 105, 155) fließt und dort eingeschlossen wird;
E) Beenden des Eindrückens bei Erreichen eines Anschlags (20, 70, 120, 170) oder bei Erreichen einer gewünschten Höhe des Spacers (42, 92, 142, 192);
F) Aushärten des Knochenzementteigs (36) in dem durch die Kavität (2, 52, 102, 152), die formgebende Oberfläche (6, 56, 106, 156) des Gießformoberteils (5, 55, 105, 155) und die Gießformwand (3, 53, 103, 153) gebildeten Hohlraum; und
G) Entnehmen des so geformten und ausgehärteten Spacers (42, 92, 142, 192) aus dem Hohlraum, wobei in der mindestens einen Durchbrechung (8, 58, 108, 158) gebildete Stege (40, 90, 140, 190) des ausgehärteten Knochenzementteigs (36) abgetrennt werden.

14. Verfahren nach Anspruch 13, **dadurch gekennzeichnet, dass**
beim Entformen des ausgehärteten Spacers (42, 92, 142, 192) nach Schritt F) oder in Schritt G) der ausgehärtete überschüssige Knochenzementteig (38) in dem unteren Behälter und/oder dem oberen Behälter (7, 57, 107, 157) verbleibt, und/oder
vor Schritt A) der Knochenzementteig (36) aus einer Monomerflüssigkeit und aus einem Zementpulver gemischt wird, insbesondere bis ein homogener Knochenzementteig (36) entstanden ist, und bevorzugt vor Schritt A) ein Metallkern (10, 60) in der Kavität (2, 52) angeordnet wird, wobei besonders bevorzugt der Metallkern (10, 60) mit Hilfe von stiftförmigen Abstandhaltern (12, 62) von der Innenwand der Kavität (2, 52) und der Gießformwand (3, 53) beabstandet wird.

15. Verfahren nach einem der Ansprüche 13 oder 14, **dadurch gekennzeichnet, dass**
während Schritt C) die Luft aus dem von der Kavität (2, 52, 102, 152) des Gießformunterteils (1, 51, 101, 151), der formgebenden Oberfläche (6, 56, 106, 156) des Gießformoberteils (5, 55, 105, 155) und der Gießformwand (3, 53, 103, 153) begrenzten Hohlraum durch die mindestens eine Durchbrechung (8, 58, 108, 158) herausgedrückt wird, und/oder
nach Schritt F) und vor Schritt G) das Gießformoberteil (5, 55, 105, 155) aus dem offenen Ende der Gießformwand (3, 53, 103, 153) herausgezogen wird.

## Claims

1. A device for producing a spacer (42, 92, 142, 192) by curing bone cement paste (36) in a casting mold, wherein the spacer (42, 92, 142, 192) is provided to temporarily replace a joint or a part of a joint comprising an articulating surface of the joint in the medical field, in particular to temporarily replace a hip joint, a knee joint or a shoulder joint, the device having
a casting mold lower part (1, 51, 101, 151), wherein the casting mold lower part (1, 51, 101, 151) has a cavity (2, 52, 102, 152) for receiving a bone cement paste (36) and for molding a first surface region of the spacer (42, 92, 142, 192) from the bone cement paste (36);
a casting mold wall (3, 53, 103, 153) which extends peripherally from a peripheral edge of the cavity (2, 52, 102, 152) of the casting mold lower part (1, 51, 101, 151) away from the cavity (2, 52, 102, 152) and which is open on the side opposite from the cavity (2, 52, 102, 152), such that the cavity (2, 52, 102, 152) is accessible through an interior (4, 54, 104, 154) delimited by the casting mold wall (3, 53, 103, 153);
a casting mold upper part (5, 55, 105, 155), wherein the casting mold upper part (5, 55, 105, 155) has a shaping surface (6, 56, 106, 156) for molding a second surface region of the spacer (42, 92, 142, 192) from the bone cement paste (36), wherein the casting mold upper part (5, 55, 105, 155) is insertable through the open side of the casting mold wall (3, 53, 103, 153) opposite from the cavity (2, 52, 102, 152) into the interior (4, 54, 104, 154) and is displaceable in the direction of the cavity (2, 52, 102, 152), such that a hollow space is formed which is delimited by the cavity (2, 52, 102, 152) of the casting mold lower part (1, 51, 101, 151), the shaping surface (6, 56, 106, 156) of the casting mold upper part (5, 55, 105, 155) and the casting mold wall (3, 53, 103, 153) and in which the spacer (42, 92, 142, 192) is moldable;
at least one container (7, 57, 107, 157) for receiving excess bone cement paste (38); and
at least one opening (8, 58, 108, 158) in the shaping surface (6, 56, 106, 156) of the casting mold upper part (5, 55, 105, 155) and/or in the cavity (2, 52, 102, 152) of the casting mold lower part (1, 51, 101, 151), wherein the at least one opening (8, 58, 108, 158) opens into the at least one container (7, 57, 107, 157) for receiving excess bone cement paste (38), wherein
the device has one or two lid(s) (9, 59, 109, 159), with which the casting mold upper part (5, 55, 105, 155) is closed or closable to the outside on the side opposite from the shaping surface (6, 56, 106, 156) of the casting mold upper part (5, 55, 105, 155), such that an upper container closed to the outside is formed as one of the at least one containers (7, 57, 107, 157) for receiving bone cement paste (36) between the casting mold upper part (5, 55, 105, 155) and the lid (9, 59, 109, 159), and/or
with which the casting mold lower part (1, 51, 101, 151) is closed or closable to the outside on the side opposite from the cavity (2, 52, 102, 152) of the casting mold lower part (1, 51, 101, 151), such that a lower container closed to the outside is formed as one of the at least one containers (7, 57, 107, 157) for receiving bone cement paste (36) between the casting mold lower part (1, 51, 101, 151) and the lid (9, 59, 109, 159), wherein the or one of the lid(s) (9, 59, 109, 159) is placed or placeable into or onto the casting mold upper part (5, 55, 105, 155) or into or onto an internal wall (11, 61, 111, 161) of the casting mold upper part (5, 55, 105, 155) in order to close the side opposite from the shaping surface (6, 56, 106, 156) of the casting mold upper part (5, 55, 105, 155) to the outside and there form a closed container (7, 57, 107, 157) for receiving excess bone cement paste (38), and/or
the or one of the lid(s) (9, 59, 109, 159) is placed or placeable into or onto the casting mold lower part (1, 51, 101, 151) in order to close the side opposite from the cavity (2, 52, 102, 152) of the casting mold lower part (1, 51, 101, 151) to the outside and there forms a closed container (7, 57, 107, 157) for receiving excess bone cement paste (38).

2. The device according to Claim 1, **characterized in that**
mutually opposing parts of the casting mold wall (3, 53, 103, 153) are oriented parallel to one another or the casting mold wall (3, 53, 103, 153) tapers slightly conically in the direction of the cavity (2, 52, 102, 152) or the casting mold wall (3, 53, 103, 153) has the shape of a right or skewed general cylinder, the base area of which is delimited by the peripheral edge of the cavity (2, 52, 102, 152).

3. The device according to Claim 1 or 2, **characterized in that**
an internal wall (11, 61, 111, 161) extends peripherally from a peripheral edge of the shaping surface (6, 56, 106, 156) of the casting mold upper part (5, 55, 105, 155) away from the cavity (2, 52, 102, 152) of the casting mold lower part (1, 51, 101, 151), wherein the internal wall (11, 61, 111, 161) preferably at least in places delimits the at least one container (7, 57, 107, 157) and/or mutually opposing parts of the internal wall (11, 61, 111, 161) are oriented parallel to one another or the internal wall (11, 61, 111, 161) has the shape of a right or skewed general cylinder, the base area of which is delimited by the peripheral edge of the shaping surface (6, 56, 106, 156) of the casting mold upper part (5, 55, 105, 155).

4. The device according to Claim 3, **characterized in that** the internal wall (11, 61, 111, 161) and the casting mold wall (3, 53, 103, 153) rest flush against one another when the casting mold upper part (5, 55, 105, 155) is pushed into the casting mold wall (3, 53, 103, 153) and/or
the internal wall (11, 61, 111, 161) forms a seal against the casting mold wall (3, 53, 103, 153) for the bone cement paste (36) when the casting mold upper part (5, 55, 105, 155) is pushed into the casting mold wall (3, 53, 103, 153).

5. The device according to any one of the preceding claims, **characterized in that**
the lid or lids (9, 59, 109, 159) has at least one vent opening and/or the lid or lids (9, 59, 109, 159) are gas-permeably closed or closable with the casting mold upper part (5, 55, 105, 155) and/or with the casting mold lower part (1, 51, 101, 151).

6. The device according to any one of the preceding claims, **characterized in that**
the volume of the cavity (2, 52, 102, 152) of the casting mold lower part (1, 51, 101, 151) and the volume delimited by the casting mold wall (3, 53, 103, 153) together are larger than the volume of the spacer (42, 92, 142, 192) to be produced, and/or
the casting mold wall (3, 53, 103, 153) and the casting mold lower part (1, 51, 101, 151) are formed as one part, wherein the casting mold wall (3, 53, 103, 153) is preferably part of the casting mold lower part (1, 51, 101, 151).

7. The device according to any one of the preceding claims, **characterized in that**
the device has a mixing system for mixing bone cement paste (36), a cement powder and a monomer liquid, wherein the cement powder and the monomer liquid are stored separately from one another, wherein the bone cement paste (36) is mixable from the cement powder and the monomer liquid with the assistance of the mixing system, wherein preferably
the mixing system includes a mixing cup (26) which particularly preferably has a spout (28) for introducing the bone cement paste (36) from the mixing cup (26) into the cavity (2, 52, 102, 152) and the interior (4, 54, 104, 154) delimited by the casting mold wall (3, 53, 103, 153), or
the mixing system is a bone cement cartridge for storing and mixing the cement powder and the monomer liquid and for delivering mixed bone cement paste (36) from the bone cement cartridge, wherein the bone cement cartridge particularly preferably contains the bone cement starting components for producing the bone cement in regions separated from one another in liquid-tight manner.

8. The device according to any one of the preceding claims, **characterized in that** the casting mold lower part (1, 51, 101, 151) and the casting mold upper part (5, 55, 105, 155), and preferably also the casting mold wall (3, 53, 103, 153) and optionally the lid or lids (9, 59, 109, 159) consist of a plastics film or substantially consist of a plastics film or the casting mold lower part (1, 51, 101, 151) and the casting mold upper part (5, 55, 105, 155), and preferably also the casting mold wall (3, 53, 103, 153) and optionally the lid or lids (9, 59, 109, 159) are in each case composed of two or more plastics films which are connected together and particularly preferably welded or adhesively bonded together, and/or
the casting mold lower part (1, 51, 101, 151), the casting mold upper part (5, 55, 105, 155) and the casting mold wall (3, 53, 103, 153) and optionally the lid or lids (9, 59, 109, 159) substantially or entirely consist of a plastics material and are preferably fabricated from a polyolefin, polyethylene (PE) or polypropylene (PP) and particularly preferably from a PETG film and/or a polyamide film and/or a PE film.

9. The device according to any one of the preceding claims, **characterized in that**
the at least one opening (8, 58, 108, 158) has a minimum cross-sectional length of a maximum of 2.5 mm, preferably a minimum cross-sectional length of a maximum of 2 mm, particularly preferably a minimum cross-sectional length of a maximum of 1.5 mm and very particularly preferably a minimum cross-sectional length of a maximum of 1 mm, and/or
the at least one opening (8, 58, 108, 158) has a minimum cross-sectional length of at least 0.2 mm, preferably of at least 0.5 mm and particularly preferably of at least 1 mm.

10. The device according to any one of the preceding claims, **characterized in that**,
at the end of the casting mold wall (3, 53, 103, 153) opposite from the cavity (2, 52, 102, 152) of the casting mold lower part (1, 51, 101, 151), a limit stop (20, 70, 120, 170) is arranged for limiting the movement of the casting mold upper part (5, 55, 105, 155) within the casting mold wall (3, 53, 103, 153) in the direction of the cavity (2, 52, 102, 152), wherein a contact surface is preferably arranged as a limit stop (20, 70, 120, 170) at the end of the casting mold wall (3, 53, 103, 153) opposite from the cavity (2, 52, 102, 152) of the casting mold lower part (1, 51, 101, 151), which contact surface particularly preferably projects out at right angles from the casting mold wall (3, 53, 103, 153), and/or
the device has a metal core (10, 60) which is to be or is arranged in the cavity (2, 52), wherein the device preferably has a plurality of spacing pieces (12, 62) which keep the metal core (10, 60) in the cavity (2, 52) spaced from the internal side of the cavity (2, 52) and from the internal side of the casting mold wall (3, 53), wherein the spacing pieces (12, 62) particularly preferably consist of cured bone cement and in particular of polymethyl methacrylate.

11. The device according to any one of the preceding claims, **characterized in that**
the casting mold lower part (1, 51, 101, 151) or the casting mold lower part (1, 51, 101, 151) and the casting mold wall (3, 53, 103, 153) or the casting mold lower part (1, 51, 101, 151), the casting mold wall (3, 53, 103, 153) and the casting mold upper part (5, 55, 105, 155) is or are transparent or translucent, and/or
the at least one opening (8, 58, 108, 158) is arranged in the regions which, in the normal disposition of the casting mold, are the highest points of the hollow space.

12. The device according to any one of the preceding claims, **characterized in that**
the shaping surface (6, 56, 106, 156) of the casting mold upper part (5, 55, 105, 155) rests with an outer edge internally against the casting mold wall (3, 53, 103, 153) and, when the casting mold upper part (5, 55, 105, 155) is pushed in, the edge slides internally on the casting mold wall (3, 53, 103, 153), wherein the edge preferably has a wiper rim or wiper lip, and/or
the device has a stand (16, 66, 116, 166), wherein the casting mold lower part (1, 51, 101, 151) is insertable into the stand (16, 66, 116, 166) and the stand (16, 66, 116, 166) is suitable, with the casting mold lower part (1, 51, 101, 151) therein, to be placed on a flat support, wherein the stand (16, 66, 116, 166) preferably substantially or entirely consists of a plastics film or of two or more plastics films which are joined together and are particularly preferably welded or adhesively bonded together.

13. A method for producing a spacer (42, 92, 142, 192) for temporarily replacing a joint or part of a joint, in particular a hip joint, a knee joint or a shoulder joint, comprising an articulating surface of the joint, wherein the method is carried out with the device according to any one of Claims 1 to 18, the method having the following chronological steps:
A) introduction of flowable bone cement paste (36) into the cavity (2, 52, 102, 152) and the interior (4, 54, 104, 154) delimited by the casting mold wall (3, 53, 103, 153), wherein a larger volume of the flowable bone cement paste (36) is introduced than is required by the spacer (42, 92, 142, 192);
B) insertion of the casting mold upper part (5, 55, 105, 155) into the casting mold wall (3, 53, 103, 153), wherein the shaping surface (6, 56, 106, 156) points in the direction of the cavity (2, 52, 102, 152);
C) pushing in of the casting mold upper part (5, 55, 105, 155) in the direction of the cavity (2, 52, 102, 152) within the casting mold wall (3, 53, 103, 153);
D) emergence of excess bone cement paste (38) through the at least one opening (8, 58, 108, 158) as the casting mold upper part (5, 55, 105, 155) continues to be pushed in, wherein the excess bone cement paste (38) flows into and is enclosed in a lower container (7, 57, 107, 157) for receiving the excess bone cement paste (38), the lower container (7, 57, 107, 157) being closed by a lid (9, 59, 109, 159), and/or the excess bone cement paste (38) flows into and is enclosed in an upper container (7, 57, 107, 157) for receiving the excess bone cement paste (38), the upper container (7, 57, 107, 157) being closed by a lid (9, 59, 109, 159);
E) completion of pushing in when a limit stop (20, 70, 120, 170) is reached or when a desired height of the spacer (42, 92, 142, 192) is reached;
F) curing of the bone cement paste (36) in the hollow space formed by the cavity (2, 52, 102, 152), the shaping surface (6, 56, 106, 156) of the casting mold upper part (5, 55, 105, 155) and the casting mold wall (3, 53, 103, 153) and
G) removal of the spacer (42, 92, 142, 192) molded and cured in this manner from the hollow space, wherein sprues (40, 90, 140, 190) of the cured bone cement paste (36) formed in the at least one opening (8, 58, 108, 158) are detached.

14. The method according to Claim 13, **characterized in that**,
on demolding the cured spacer (42, 92, 142, 192) after step F) or in step G), the cured excess bone cement paste (38) remains in the lower container and/or the upper container (7, 57, 107, 157) and/or
before step A), the bone cement paste (36) is mixed from a monomer liquid and a cement powder, in particular until a homogeneous bone cement paste (36) is obtained, and, preferably before step A), a metal core (10, 60) is arranged in the cavity (2, 52), wherein the metal core (10, 60) is particularly preferably spaced from the internal wall of the cavity (2, 52) and the casting mold wall (3, 53) with the assistance of pin-shaped spacing pieces (12, 62).

15. The method according to any one of Claims 13 or 14, **characterized in that**,
during step C), the air is expelled through the at least one opening (8, 58, 108, 158) from the hollow space delimited by the cavity (2, 52, 102, 152) of the casting mold lower part (1, 51, 101, 151), the shaping surface (6, 56, 106, 156) of the casting mold upper part (5, 55, 105, 155) and the casting mold wall (3, 53, 103, 153), and/or
after step F) and before step G), the casting mold upper part (5, 55, 105, 155) is withdrawn from the open end of the casting mold wall (3, 53, 103, 153).

## Revendications

1. Dispositif de fabrication d'un espaceur (42, 92, 142, 192) par durcissement d'une pâte de ciment osseux (36) dans un moule de coulée, l'espaceur (42, 92, 142, 192) étant conçu pour remplacer temporairement, dans le domaine médical, une articulation ou une partie d'une articulation comprenant une surface articulée de l'articulation, en particulier pour remplacer temporairement une articulation de la hanche, une articulation du genou ou une articulation de l'épaule, le dispositif présentant
une partie inférieure de moule de coulée (1, 51, 101, 151), la partie inférieure de moule de coulée (1, 51, 101, 151) présentant une cavité (2, 52, 102, 152) permettant de recevoir une pâte de ciment osseux (36) et permettant de mouler une première région de surface de l'espaceur (42, 92, 142, 192) à partir de la pâte de ciment osseux (36) ;
une paroi de moule de coulée (3, 53, 103, 153) qui s'étend de manière périphérique à partir d'un bord périphérique de la cavité (2, 52, 102, 152) de manière à s'éloigner de la cavité (2, 52, 102, 152) de la partie inférieure de moule de coulée (1, 51, 101, 151) et qui est ouverte sur le côté opposé à la cavité (2, 52, 102, 152), de sorte que la cavité (2, 52, 102, 152) est accessible à travers un espace intérieur (4, 54, 104, 154) délimité par la paroi de moule de coulée (3, 53, 103, 153) ;
une partie supérieure de moule de coulée (5, 55, 105, 155), la partie supérieure de moule de coulée (5, 55, 105, 155) présentant une surface de façonnage (6, 56, 106, 156) permettant de mouler une seconde région de surface de l'espaceur (42, 92, 142, 192) à partir de la pâte de ciment osseux (36), la partie supérieure de moule de coulée (5, 55, 105, 155) pouvant être enfichée dans l'espace intérieur (4, 54, 104, 154) à travers le côté ouvert de la paroi de moule de coulée (3, 53, 103, 153) opposé à la cavité (2, 52, 102, 152) et pouvant être déplacée en direction de la cavité (2, 52, 102, 152), de sorte qu'un espace creux délimité par la cavité (2, 52, 102, 152) de la partie inférieure de moule de coulée (1, 51, 101, 151), la surface de façonnage (6, 56, 106, 156) de la partie supérieure de moule de coulée (5, 55, 105, 155) et la paroi de moule de coulée (3, 53, 103, 153) est formé, dans lequel espace creux l'espaceur (42, 92, 142, 192) peut être moulé ;
au moins un récipient (7, 57, 107, 157) permettant de recevoir de la pâte de ciment osseux excédentaire (38) ; et
au moins une ouverture (8, 58, 108, 158) dans la surface de façonnage (6, 56, 106, 156) de la partie supérieure de moule de coulée (5, 55, 105, 155) et/ou dans la cavité (2, 52, 102, 152) de la partie inférieure de moule de coulée (1, 51, 101, 151), l'au moins une ouverture (8, 58, 108, 158) débouchant dans l'au moins un récipient (7, 57, 107, 157) permettant de recevoir de la pâte de ciment osseux excédentaire (38), le dispositif présentant un ou deux couvercles (9, 59, 109, 159),
à l'aide duquel ou à l'aide de l'un desquels la partie supérieure de moule de coulée (5, 55, 105, 155) est fermée ou peut être fermée vers l'extérieur sur le côté opposé à la surface de façonnage (6, 56, 106, 156) de la partie supérieure de moule de coulée (5, 55, 105, 155), de sorte qu'un récipient supérieur fermé vers l'extérieur est formé entre la partie supérieure de moule de coulée (5, 55, 105, 155) et le couvercle (9, 59, 109, 159) en tant que récipient parmi l'au moins un récipient (7, 57, 107, 157) permettant de recevoir de la pâte de ciment osseux (36), et/ou à l'aide duquel ou à l'aide de l'un desquels la partie inférieure de moule de coulée (1, 51, 101, 151) est fermée ou peut être fermée vers l'extérieur sur le côté opposé à la cavité (2, 52, 102, 152) de la partie inférieure de moule de coulée (1, 51, 101, 151), de sorte qu'un récipient inférieur fermé vers l'extérieur est formé entre la partie inférieure de moule de coulée (1, 51, 101, 151) et le couvercle (9, 59, 109, 159) en tant que récipient parmi l'au moins un récipient (7, 57, 107, 157) permettant de recevoir de la pâte de ciment osseux (36),
le couvercle ou l'un des couvercles (9, 59, 109, 159) étant placé ou pouvant être placé dans ou sur la partie supérieure de moule de coulée (5, 55, 105, 155) ou dans ou sur une paroi intérieure (11, 61, 111, 161) de la partie supérieure de moule de coulée (5, 55, 105, 155) afin de fermer vers l'extérieur le côté opposé à la surface de façonnage (6, 56, 106, 156) de la partie supérieure de moule de coulée (5, 55, 105, 155) et d'y former un récipient (7, 57, 107, 157) fermé permettant de recevoir de la pâte de ciment osseux excédentaire (38), et/ou
le couvercle ou l'un des couvercles (9, 59, 109, 159) étant placé ou pouvant être placé dans ou sur la partie inférieure de moule de coulée (1, 51, 101, 151) afin de fermer vers l'extérieur le côté opposé à la cavité (2, 52, 102, 152) de la partie inférieure de moule de coulée (1, 51, 101, 151) et d'y former un récipient (7, 57, 107, 157) fermé permettant de recevoir de la pâte de ciment osseux excédentaire (38).

2. Dispositif selon la revendication 1, **caractérisé en ce que**
des parties mutuellement opposées de la paroi de moule de coulée (3, 53, 103, 153) sont orientées parallèlement l'une à l'autre ou la paroi de moule de coulée (3, 53, 103, 153) converge de manière légèrement conique en direction de la cavité (2, 52, 102, 152) ou la paroi de moule de coulée (3, 53, 103, 153) présente la forme d'un cylindre général droit ou oblique, dont la surface de base est délimitée par le bord périphérique de la cavité (2, 52, 102, 152).

3. Dispositif selon la revendication 1 ou 2, **caractérisé en ce que**
une paroi intérieure (11, 61, 111, 161) s'étend de manière périphérique à partir d'un bord périphérique de la surface de façonnage (6, 56, 106, 156) de la partie supérieure de moule de coulée (5, 55, 105, 155) de manière à s'éloigner de la cavité (2, 52, 102, 152) de la partie inférieure de moule de coulée (1, 51, 101, 151), de préférence la paroi intérieure (11, 61, 111, 161) délimitant l'au moins un récipient (7, 57, 107, 157) au moins dans certaines régions et/ou des parties mutuellement opposées de la paroi intérieure (11, 61, 111, 161) étant orientées parallèlement l'une à l'autre ou la paroi intérieure (11, 61, 111, 161) présentant la forme d'un cylindre général droit ou oblique, dont la surface de base est délimitée par le bord périphérique de la surface de façonnage (6, 56, 106, 156) de la partie supérieure de moule de coulée (5, 55, 105, 155).

4. Dispositif selon la revendication 3, **caractérisé en ce que**
la paroi intérieure (11, 61, 111, 161) et la paroi de moule de coulée (3, 53, 103, 153) s'appuient l'une contre l'autre en affleurement de surface lorsque la partie supérieure de moule de coulée (5, 55, 105, 155) est insérée dans la paroi de moule de coulée (3, 53, 103, 153) et/ou
la paroi intérieure (11, 61, 111, 161) réalise l'étanchéité par rapport à la paroi de moule de coulée (3, 53, 103, 153) pour la pâte de ciment osseux (36) lorsque la partie supérieure de moule de coulée (5, 55, 105, 155) est insérée dans la paroi de moule de coulée (3, 53, 103, 153).

5. Dispositif selon l'une des revendications précédentes, **caractérisé en ce que**
le ou les couvercles (9, 59, 109, 159) présente ou présentent au moins un orifice d'aération et/ou le ou les couvercles (9, 59, 109, 159) sont fermés ou peuvent être fermés de manière étanche aux gaz à l'aide de la partie supérieure de moule de coulée (5, 55, 105, 155) et/ou de la partie inférieure de moule de coulée (1, 51, 101, 151).

6. Dispositif selon l'une des revendications précédentes, **caractérisé en ce que**
le volume de la cavité (2, 52, 102, 152) de la partie inférieure de moule de coulée (1, 51, 101, 151) et le volume délimité par la paroi de moule de coulée (3, 53, 103, 153) sont conjointement supérieurs au volume de l'espaceur (42, 92, 142, 192) devant être produit, et/ou la paroi de moule de coulée (3, 53, 103, 153) et la partie inférieure de moule de coulée (1, 51, 101, 151) sont réalisées d'un seul tenant, de préférence la paroi de moule de coulée (3, 53, 103, 153) faisant partie de la partie inférieure de moule de coulée (1, 51, 101, 151).

7. Dispositif selon l'une des revendications précédentes, **caractérisé en ce que**
le dispositif présente un système de mélange permettant de mélanger de la pâte de ciment osseux (36), une poudre de ciment et un liquide monomère, la poudre de ciment et le liquide monomère étant stockés indépendamment l'un de l'autre, la pâte de ciment osseux (36) pouvant être mélangée à partir de la poudre de ciment et du liquide monomère à l'aide du système de mélange, de préférence
le système de mélange présentant un gobelet de mélange (26) qui présente de manière particulièrement préférée un bec verseur (28) permettant d'introduire la pâte de ciment osseux (36) à partir du gobelet de mélange (26) dans la cavité (2, 52, 102, 152) et l'espace intérieur (4, 54, 104, 154) délimité par la paroi de moule de coulée (3, 53, 103, 153), ou
le système de mélange étant une cartouche de ciment osseux permettant de stocker et de mélanger la poudre de ciment et le liquide monomère et permettant d'évacuer de la pâte de ciment osseux (36) mélangée hors de la cartouche de ciment osseux, de manière particulièrement préférée, la cartouche de ciment osseux contenant, dans des régions séparées les unes des autres de manière étanche aux liquides, les composants de départ de ciment osseux permettant de fabriquer le ciment osseux.

8. Dispositif selon l'une des revendications précédentes, **caractérisé en ce que**
la partie inférieure de moule de coulée (1, 51, 101, 151) et la partie supérieure de moule de coulée (5, 55, 105, 155), et de préférence également la paroi de moule de coulée (3, 53, 103, 153) et éventuellement le ou les couvercles (9, 59, 109, 159), sont constitués d'une feuille de matière plastique ou sont constitués sensiblement d'une feuille de matière plastique ou la partie inférieure de moule de coulée (1, 51, 101, 151) et la partie supérieure de moule de coulée (5, 55, 105, 155), et de préférence également la paroi de moule de coulée (3, 53, 103, 153) et éventuellement le ou les couvercles (9, 59, 109, 159), sont construits respectivement à partir de deux feuilles de matière plastique ou plus, lesquelles sont reliées les unes aux autres, de manière particulièrement préférée sont soudées ou collées les unes aux autres, et/ou
la partie inférieure de moule de coulée (1, 51, 101, 151), la partie supérieure de moule de coulée (5, 55, 105, 155) et la paroi de moule de coulée (3, 53, 103, 153) et éventuellement le ou les couvercles (9, 59, 109, 159) sont constitués sensiblement ou complètement d'une matière plastique, de préférence sont fabriqués à partir d'une polyoléfine, d'un polyéthylène (PE) ou d'un polypropylène (PP), de manière particulièrement préférée à partir d'une feuille de PETG et/ou d'une feuille de polyamide et/ou d'une feuille de PE.

9. Dispositif selon l'une des revendications précédentes, **caractérisé en ce que**
l'au moins une ouverture (8, 58, 108, 158) possède une longueur en section transversale minimale d'au maximum 2,5 mm, de préférence une longueur en section transversale minimale d'au maximum 2 mm, de manière particulièrement préférée une longueur en section transversale minimale d'au maximum 1,5 mm, de manière tout particulièrement préférée une longueur en section transversale minimale d'au maximum 1 mm, et/ou l'au moins une ouverture (8, 58, 108, 158) possède une longueur en section transversale minimale d'au moins 0,2 mm, de préférence d'au moins 0,5 mm, de manière particulièrement préférée d'au moins 1 mm.

10. Dispositif selon l'une des revendications précédentes, **caractérisé en ce que**
au niveau de l'extrémité de la paroi de moule de coulée (3, 53, 103, 153) opposée à la cavité (2, 52, 102, 152) de la partie inférieure de moule de coulée (1, 51, 101, 151) est disposée une butée (20, 70, 120, 170) permettant de limiter le déplacement de la partie supérieure de moule de coulée (5, 55, 105, 155) à l'intérieur de la paroi de moule de coulée (3, 53, 103, 153) en direction de la cavité (2, 52, 102, 152), de préférence une surface d'appui étant disposée en tant que butée (20, 70, 120, 170) au niveau de l'extrémité de la paroi de moule de coulée (3, 53, 103, 153) opposée à la cavité (2, 52, 102, 152) de la partie inférieure de moule de coulée (1, 51, 101, 151), laquelle surface d'appui, de manière particulièrement préférée, fait saillie à angle droit à partir de la paroi de moule de coulée (3, 53, 103, 153), et/ou
le dispositif présentant un noyau métallique (10, 60) qui doit être disposé ou est disposé dans la cavité (2, 52), le dispositif présentant de préférence plusieurs pièces d'écartement (12, 62) qui maintiennent le noyau métallique (10, 60) dans la cavité (2, 52) à distance du côté intérieur de la cavité (2, 52) et du côté intérieur de la paroi de moule de coulée (3, 53), de manière particulièrement préférée, les pièces d'écartement (12, 62) étant constituées de ciment osseux durci, en particulier de polyméthacrylate de méthyle.

11. Dispositif selon l'une des revendications précédentes, **caractérisé en ce que**
la partie inférieure de moule de coulée (1, 51, 101, 151) ou la partie inférieure de moule de coulée (1, 51, 101, 151) et la paroi de moule de coulée (3, 53, 103, 153) ou la partie inférieure de moule de coulée (1, 51, 101, 151), la paroi de moule de coulée (3, 53, 103, 153) et la partie supérieure de moule de coulée (5, 55, 105, 155) sont transparentes ou translucides, et/ou
l'au moins une ouverture (8, 58, 108, 158) est disposée au niveau des régions qui, en cas d'agencement normal du moule de coulée, sont les points les plus élevés de l'espace creux.

12. Dispositif selon l'une des revendications précédentes, **caractérisé en ce que**
la surface de façonnage (6, 56, 106, 156) de la partie supérieure de moule de coulée (5, 55, 105, 155) s'appuie, par un bord extérieur, intérieurement contre la paroi de moule de coulée (3, 53, 103, 153) et, lorsque la partie supérieure de moule de coulée (5, 55, 105, 155) est insérée, le bord glisse intérieurement sur la paroi de moule de coulée (3, 53, 103, 153), le bord comprenant de préférence une arête de raclage ou une lèvre de raclage, et/ou
le dispositif présentant un pied (16, 66, 116, 166), la partie inférieure de moule de coulée (1, 51, 101, 151) pouvant être insérée dans le pied (16, 66, 116, 166) et le pied (16, 66, 116, 166) étant apte, lorsque la partie inférieure de moule de coulée (1, 51, 101, 151) est dans celui-ci, à être placé sur un support plat, de préférence le pied (16, 66, 116, 166) étant constitué sensiblement ou complètement d'une feuille de matière plastique ou de deux feuilles de matière plastique ou plus reliées les unes aux autres, de manière particulièrement préférée soudées ou collées les unes aux autres.

13. Procédé de fabrication d'un espaceur (42, 92, 142, 192) permettant de remplacer temporairement une articulation ou une partie d'une articulation, en particulier une articulation de la hanche, une articulation du genou ou une articulation de l'épaule, comprenant une surface articulée de l'articulation, le procédé étant mis en oeuvre à l'aide d'un dispositif selon l'une des revendications 1 à 12, le procédé présentant les étapes chronologiques suivantes :
A) introduction de pâte de ciment osseux (36) coulante dans la cavité (2, 52, 102, 152) et l'espace intérieur (4, 54, 104, 154) délimité par la paroi de moule de coulée (3, 53, 103, 153), un plus grand volume de pâte de ciment osseux (36) coulante que ce qui est requis pour l'espaceur (42, 92, 142, 192) étant introduit ;
B) insertion de la partie supérieure de moule de coulée (5, 55, 105, 155) dans la paroi de moule de coulée (3, 53, 103, 153), la surface de façonnage (6, 56, 106, 156) étant tournée en direction de la cavité (2, 52, 102, 152) ;
C) enfoncement de la partie supérieure de moule de coulée (5, 55, 105, 155) en direction de la cavité (2, 52, 102, 152) à l'intérieur de la paroi de moule de coulée (3, 53, 103, 153) ;
D) sortie de la pâte de ciment osseux excédentaire (38) à travers l'au moins une ouverture (8, 58, 108, 158) lorsque la partie supérieure de moule de coulée (5, 55, 105, 155) continue à être enfoncée, la pâte de ciment osseux excédentaire (38) s'écoulant dans un récipient (7, 57, 107, 157) inférieur fermé par un couvercle (9, 59, 109, 159) et permettant de recevoir la pâte de ciment osseux excédentaire (38) et y étant renfermée et/ou s'écoulant dans un récipient (7, 57, 107, 157) supérieur fermé par un couvercle (9, 59, 109, 159) et permettant de recevoir la pâte de ciment osseux excédentaire (38) au niveau de la partie supérieure de moule de coulée (5, 55, 105, 155) et y étant renfermée ;
E) achèvement de l'enfoncement lorsqu'une butée (20, 70, 120, 170) est atteinte ou lorsqu'une hauteur souhaitée de l'espaceur (42, 92, 142, 192) est atteinte ;
F) durcissement de la pâte de ciment osseux (36) dans l'espace creux formé par la cavité (2, 52, 102, 152), la surface de façonnage (6, 56, 106, 156) de la partie supérieure de moule de coulée (5, 55, 105, 155) et la paroi de moule de coulée (3, 53, 103, 153) ; et
G) retrait de l'espaceur (42, 92, 142, 192) ainsi moulé et durci hors de l'espace creux, des nervures (40, 90, 140, 190) de la pâte de ciment osseux (36) durcie formées dans l'au moins une ouverture (8, 58, 108, 158) étant détachées.

14. Procédé selon la revendication 13, **caractérisé en ce que**
lors du démoulage de l'espaceur (42, 92, 142, 192) durci après l'étape F) ou à l'étape G), la pâte de ciment osseux excédentaire (38) durcie demeure dans le récipient inférieur et/ou le récipient supérieur (7, 57, 107, 157), et/ou
avant l'étape A), la pâte de ciment osseux (36) est mélangée à partir d'un liquide monomère et à partir d'une poudre de ciment, en particulier jusqu'à ce qu'une pâte de ciment osseux (36) homogène soit obtenue, et de préférence, avant l'étape A), un noyau métallique (10, 60) est disposé dans la cavité (2, 52), de manière particulièrement préférée, le noyau métallique (10, 60) étant espacé de la paroi intérieure de la cavité (2, 52) et de la paroi de moule de coulée (3, 53) à l'aide de pièces d'écartement (12, 62) en forme de goupilles.

15. Procédé selon l'une des revendications 13 ou 14, **caractérisé en ce que**, pendant l'étape C), l'air est expulsé à travers l'au moins une ouverture (8, 58, 108, 158) hors de l'espace creux délimité par la cavité (2, 52, 102, 152) de la partie inférieure de moule de coulée (1, 51, 101, 151), la surface de façonnage (6, 56, 106, 156) de la partie supérieure de moule de coulée (5, 55, 105, 155) et la paroi de moule de coulée (3, 53, 103, 153), et/ou
après l'étape F) et avant l'étape G), la partie supérieure de moule de coulée (5, 55, 105, 155) est extraite de l'extrémité ouverte de la paroi de moule de coulée (3, 53, 103, 153).
